# EUROPEAN PATENT APPLICATION

(11) **EP 4 840 460 A1**
(43) Date of publication of application: **18.11.2026**
(21) Application number: 25738566.6
(22) Date of filing: 10.01.2025
(51) Int. Cl.: A61K 38/26, A61K 38/00, A61P 3/04, A61P 3/10

(54) **PHARMACEUTICAL COMPOSITION OF LONG-ACTING GLP-1/GIP DUAL AGONIST**

(30) Priority: 12.01.2024 CN 202410052070
(71) Applicant: Hangzhou Zhongmeihuadong Pharmaceutical Co., Ltd., GongShu District HangZhou, Zhejiang 310011 (CN)
(72) Inventor: ZHANG, Nan, Hangzhou, Zhejiang 310011 (CN); LI, Honghao, Hangzhou, Zhejiang 310011 (CN); YANG, Yuanyuan, Hangzhou, Zhejiang 310011 (CN); TENG, Li, Hangzhou, Zhejiang 310011 (CN); JIANG, Chunhua, Hangzhou, Zhejiang 310011 (CN); LIU, Dongzhou, Hangzhou, Zhejiang 310011 (CN)
(74) Representative: Nederlandsch Octrooibureau
(86) International application number: PCT/CN2025/071771
(87) International publication number: WO 2025/149039

(57) **Abstract**

A pharmaceutical composition comprising a long-acting GLP-1/GIP dual agonist, a kit comprising the pharmaceutical composition, and use of the pharmaceutical composition in the preparation of a drug for treating diabetes or obesity.

## Description

### TECHNICAL FIELD

The present invention relates to the field of polypeptide pharmaceutical formulations. In particular, the present invention provides a pharmaceutical composition comprising a long-acting GLP-1/GIP dual agonist, a kit comprising the pharmaceutical composition, and the use of the pharmaceutical composition in the manufacture of a medicament for treating diabetes mellitus or obesity.

### BACKGROUND

Glucagon-like peptide-1 (GLP-1) is a polypeptide hormone secreted by gut in response to food stimulation. GLP-1 stimulates insulin secretion and reduces glucagon secretion in a glucose-dependent manner. Glucose-dependent insulinotropic polypeptide (GIP) is currently believed to be primarily secreted by enteroendocrine K cells in the duodenum and proximal jejunum. Similar to GLP-1, GIP can stimulate insulin secretion. However, the half-lives of both natural GLP-1 and GIP in humans and animals as short as several minutes.

Pharmaceutical researchers have developed a series of GLP-1R/GIPR dual agonists to mimic the biological effects of native GLP-1 and GIP, and to extend their duration of action. Polypeptide GLP-1 receptor agonists and GIP receptor agonists are generally administered via injection or non-gastrointestinal routes. However, due to their amino acid sequences, net charges, secondary structures, and chemical modifications, as well as the medium environment in which they are situated, such as excipients, pH, and temperature, polypeptides are prone to undesirable product attributes such as chemical degradation, polymerization, and physical aggregation. Unfavorable medium conditions such as buffers, osmotic pressure regulators, and pH values, as well as environmental conditions like temperature and light, can affect and even destroy the stability of the polypeptide compounds, and lead to peptide isomerization, deamidation, amide bond cleavage, and undesirable chemical reactions of polymers, as well as physical aggregation between polypeptide compound molecules. Furthermore, as the concentration of the peptide compound increases, the tendency to physical aggregation also increases.

Therefore, it is still necessary to develop alternative pharmaceutical compositions comprising GLP-1 receptor agonists or GIP receptor agonists. It is desirable that the pharmaceutical composition exerts a good and lasting effect in lowering blood glucose or reducing weight, maintains stability during production, storage, and use, and can withstand certain abnormal temperatures without deterioration, thereby increasing the safety and effectiveness in use for users in different scenarios.

### DISCLOSURE OF THE INVENTION

In one aspect of the present invention, provided is a compound of Formula I, or a pharmaceutically acceptable salt thereof, or a physiologically acceptable salt thereof, or a precursor compound thereof: 
Y-Aib-E-G-T-αMePhe-T-S-D-Y-Aib-I-Aib-L-D-K-Q-A-Q-A-E-F-V-K24-W-L-L-K28-G-G-P-S-S-
G-A-P-P-P-S-K     Formula I (SEO ID NO:12);
 wherein
1 K, which is selected from the group consisting of K at position 16, K at position 24, K at position 28 and K at position 40, is chemically modified through conjugation to the ε-amino of the K side-chain with ([2-(2-amino-ethoxy)-ethoxy]-acetyl)ₐ-(γ-Glu)_{b}-CO-(CH₂)_{c}-Z, wherein each a is independently selected from an integer of 1, 2 and 3, each b is independently selected from an integer of 1, 2 and 3, and each c is independently selected from an integer of 16, 18 and 20, wherein Z is independently selected from the group consisting of -CH₃, carboxylic acids or carboxylic acid bioisosteres, phosphonates or sulfonates, preferably -COOH; and the C-terminal amino acid is amidated as a C-terminal primary amide.

In one aspect of the present invention, provided is a pharmaceutical composition, comprising a compound of Formula I, or a pharmaceutically acceptable salt thereof, or a physiologically acceptable salt thereof, or a precursor compound thereof.

In one aspect of the present invention, provided is a use of the pharmaceutical composition according to the present invention in the manufacuture of a medicament for treating a disease, such as a disease associated to diabetes mellitus or obesity and the like.

In one aspect of the present invention, provided is the pharmaceutical composition according to the present invention for use in treating a disease, such as diabetes mellitus or obesity.

In one aspect of the present invention, provided is a method for preventing or treating a disease, comprising administering an effective amount of the pharmaceutical composition according to the present invention to a human or animal in need thereof, wherein, e.g., the disease is diabetes mellitus or obesity.

The compound of Formula I of the present invention has a dual agonist effect on GLP-1 receptors and GIP receptors, thus can be used as a GLP-1/GIP dual agonist, has a good therapeutic effect of lowering the blood glucose and/or reducing weight, and has a long half-life. The pharmaceutical composition comprising the compound of the present invention maintains excellent stability, especially stability in terms of clarity, color, purity, polymer content, and pH value, etc., both during storage at a high-temperature, room-temperature, and refrigerated condition, and during use, and can withstand certain abnormal temperatures without deterioration.

### DETAILED DESCRIPTION

Unless additionalwise specified, all scientific and technical terms in the context of present invention should have the same meanings as known to those skilled in the art. If there is any inconsistency, the definitions provided in the present invention shall prevail.

It should be understood that all detailed discussions on materials, methods, and examples are merely for the purpose of illustration and cannot to be construed as limiting the present invention unless otherwise expressly specified.

The terms "containing" and "comprising" as used herein have the same meaning, indicating that additional ingredients or additional steps that do not affect the final effect may be included. Such terms include the terms "consisting of" and "substantially consisting of". The product and method according to the present invention may comprise, consist of, and substantially consist of the essential technical features and/or limitations of the present invention described herein as well as any additional and/or optional ingredients, components, steps or limitations described herein.

Unless additionalwise specified, in the context of the present invention, each numerical range is meant to include both endpoints and any values and sub-ranges falling within the numerical range.

The term "about" or "approximately" used in the present invention means that the numerial value is within an acceptable error range for the specific value determined by those ordinary skilled in the art in the art, which depend to some extent on how to determine or measure the value (i.e., the limitation of the measurement system). For example,"about" or "substantially comprising" may mean a fluctuation range of up to ±20%. For example,a pH ranging from "about" 5.5 implies that the pH is 5.5±1.1. In addition, especially for biological systems or processes, this term may imply up to one order of magnitude or up to five times of the value. Unless otherwise specified, when a specific value appears in the present application and claims, the meaning of "about" or "substantially comprising" should be presumed to be within an acceptable error range of the specific value.

Unless additionalwise specified, all materials and reagents used in the present invention are commercially available.

### [Pharmaceutical Composition and Method for Preparing the Same]

The present invention discloses a pharmaceutical composition comprising a compound of Formula I, or a pharmaceutically acceptable salt thereof, or a physiologically acceptable salt thereof, or a precursor compound thereof: 
Y-Aib-E-G-T-αMePhe-T-S-D-Y-Aib-I-Aib-L-D-K-Q-A-Q-A-E-F-V-K^24-W-L-L-K^28-G-G-P-S-S-
G-A-P-P-P-S-K      Formula I (SEQ ID NO:12);
 wherein
1 K, which is selected from the group consisting of K at position 16, K at position 24, K at position 28 and K at position 40 of Formula I, is chemically modified through conjugation to the ε-amino of the K side-chain with ([2-(2-amino-ethoxy)-ethoxy]-acetyl)ₐ-(γ-Glu)_{b}-CO-(CH₂)_{c}-Z, wherein each a is independently selected from an integer of 1, 2 and 3, each b is independently selected from an integer of 1, 2 and 3, and each c is independently selected from an integer of 16, 18 and 20, wherein Z is independently selected from the group consisting of -CH₃, carboxylic acids or carboxylic acid bioisosteres, phosphonates or sulfonates, preferably -COOH; and the C-terminal amino acid is amidated as a C-terminal primary amide.

The compounds of formula I according to the present invention have the same peptide backbone: Y-Aib-E-G-T-αMePhe-T-S-D-Y-Aib-I-Aib-L-D-K-Q-A-Q-A-E-F-V-K²⁴-W-L-L-K²⁸-G-G-P-S-S-G-A-P-P-P-S-K, therefore, they possess the physical and chemical stability characteristics associated with the common polypeptide backbone. The physical and chemical stability characteristics include the isoelectric points and solubility properties of the peptide backbones, as well as the chemical reactivity of specific functional groups of amino acid residues and their amido bonds within the medium environment. In the compounds of formula I according to the present invention, the peptide backbones are modified with ([2-(2-amino-ethoxy)-ethoxy]-acetyl)ₐ-(γ-Glu)_{b}-CO-(CH₂)_{c}-Z at the side chains, ant the modification imparts charges, hydrophilic, and hydrophobic properties to the polypeptide backbones, while preserving the inherent common characteristics of the polypeptide chain.

As used herein, the term "pharmaceutical composition" refers to a mixture containing one or more polypeptide compounds of Formula I according to the present invention, or pharmaceutically acceptable salts thereof, or physiologically acceptable salts thereof, or precursor compounds thereof, and optionally additional components. The additional components include physiological/pharmaceutically acceptable carriers and excipients. The purpose of the pharmaceutical composition is to promote drug delivery to organisms, so as to facilitate the absorption of the polypeptide compound of Formula I according to the present invention as an active pharmaceutical ingredient, and then exert its biological activity.

In the context of the present invention, unless otherwise specified or clearly contradictory, the terms "pharmaceutical composition", "composition" and "formulation" can be used interchangeably.

The compound of Formula I of the present invention has a dual agonist effect on GLP-1 receptors and GIP receptors, thus can be used as a GLP-1/GIP dual agonist, has a good therapeutic effect of lowering the blood glucose and/or reducing weight, and has a long half-life. The pharmaceutical composition comprising the compound of the present invention maintains excellent stability, especially stability in terms of clarity, color, purity, polymer content, and pH value, etc., both during storage at a high-temperature, room temperature, and refrigerated condition, and during use, and can withstand certain abnormal temperatures without deterioration.

In some embodiments, the pharmaceutical composition is in the form of an aqueous solution.

In some embodiments, the pharmaceutical composition is injectable.

In some embodiments, the pharmaceutical composition is a purified liquid formulation.

In some embodiments, the purified liquid formulation includes a subcutaneous injection formulation, an intravenous injection formulation, an intraperitoneal administration formulation, an intramuscular injection formulation, an intravenous/subcutaneous injection formulation, and an intravitreal injection formulation.

In some embodiments, the purified liquid formulation includes an aqueous injection formulation and a pre-filled injection formulaton, preferably an aqueous injection formulaton.

In some embodiments, the purified liquid formulation includes an aqueous injection formulation and a pre-filled injection formulaton, and the container for the pre-filled injection formulaton may be vials, cartridges, or pre-filled syringe.

In some embodiments, the pharmaceutical composition is an aqueous subcutaneous injection formulation.

In some embodiments, the pharmaceutical composition comprises a polypeptide compound of Formula I, a buffer, an osmotic pressure regulator, water, and optionally an pH adjusting agent, and optionally an additional excipient and/or carrier. The additional excipients and/or carriers include one or more of preservatives, stabilizers, and antioxidants.

In some embodiments, the water is purified water, deionized water, or injection water.

In some embodiments, the preservative includes phenol, m-cresol, cresol, phenoxyethanol, chlorobutanol, benzyl alcohol, benzoate derivatives, and any combination thereof; preferably, the preservative is phenol, m-cresol, benzyl alcohol, benzoate derivatives, and any combination thereof.

In some embodiments, cresol includes m-cresol, o-cresol, p-cresol, or any combination thereof.

In the context of the present invention, there is no particular limitation on the benzoate derivatives, and the benzoate derivatives commonly used in the art can be used in the present invention. In some embodiments, the benzoate derivatives may be a parahydroxybenzoate ester (paraben), such as a C1~C10 ester of parahydroxybenzoic acid, preferably a C1~C6 ester of parahydroxybenzoic acid, and more preferably a C1~C4 ester of parahydroxybenzoic acid, such as methyl p-hydroxybenzoate (methyl paraben), ethyl p-hydroxybenzoate (ethyl paraben), propyl p-hydroxybenzoate (propyl paraben), tert-butyl p-hydroxybenzoate (tert-butyl paraben), etc.

In some embodiments, the method for preparing the pharmaceutical composition comprises:
providing a buffer, an osmotic pressure regulator, and optionally an additional excipient and/or carrier to obtain a mixture,
mixing the mixture with water to obtain an aqueous mixture,
adding the polypeptide compound of Formula I or an aqueous solution of the polypeptide compound of Formula I to the aqueous mixture to obtain a mixture comprising the polypeptide compound of Formula I,
optionally adding a pH adjusting agent to adjust the pH value of the mixture to a target pH value based on the difference between the pH value of the mixture and the target pH value, and
adding additional water to make up the volume.

"Optionally adding a pH adjusting agent" means that: upon measuring the pH value of the mixture comprising the polypeptide compound of Formula I, if the measured pH value deviates from the target pH value, then a pH adjusting agent is adding to adjust the pH value of the aqeous mixture comprising the polypeptide to the target pH value; otherwise, if the measured pH value is exactly the target pH value, then no pH adjusting agent is added.

Those skilled in the art would appreciate that the water finally added is used to make up the volume, therefore it is not necessary to define the amount of water.

In some embodiments, the pharmaceutical composition comprises the polypeptide compound of Formula I, a buffer, an osmotic pressure regulator, water, and optionally a pH adjusting agent.

In some embodiments, the pharmaceutical composition comprises:
the polypeptide compound of Formula I,
a pH adjusting agent selected from the group consisting of an aqueous sodium hydroxide solution or hydrochloric acid,
a buffer selected from the group consisting of disodium hydrogen phosphate, tromethamine, trisodium citrate, trisodium citrate dihydrate, citric acid-trisodium citrate, citric acid monohydrate-trisodium citrate dihydrate, and a combination thereof,
an osmotic pressure regulator selected from the group consisting of mannitol, sodium chloride, propylene glycol, glycerol, and a combination thereof,
a preservative selected from the group consisting of phenol, m-cresol, cresol, phenoxyethanol, chlorobutanol, benzyl alcohol, benzoate derivatives, and any combination thereof, and
water.

In some embodiments, the pharmaceutical composition is an aqueous solution, and based on the total volume of the aqueous solution, the pharmaceutical composition comprises:
about 0.1 to about 50 mg/mL of the compound of Formula I, for example, about 1 to about 40 mg/mL of Compound P016, P014 or P020,
citric acid and trisodium citrate at a total molar concentration of from 0.5 to about 50 mM, preferably about 1.0 to about 10 mM of citric acid-trisodium citrate, wherein the molar ratio of citric acid to trisodium citrate is about 1:4-50, preferably about 1:4-20,
0 to about 50 mg/mL, preferably about 0.1 to about 50 mg/mL, more preferably about 1 to about 10 mg/mL, further more preferably about 2 to about 6 mg/mL of the preservative,
about 1 to about 100 mg/mL of the osmotic pressure regulator, preferably about 1 to about 100 mg/mL of mannitol, 1,2-propylene glycol, 1,3-propylene glycol, glycerol, sodium chloride, or any combination thereof,
optionally an aqueous sodium hydroxide solution or hydrochloric acid, and
water,
wherein the aqueous solution has a pH ranging about 5.0 to about 9.0, for example, about 5.8 to about 9.0, preferably about 6.0 to about 8.8, and more preferably about 6.5 to about 7.4.

In some embodiments, the pharmaceutical composition is an aqueous solution, and based on the total volume of the aqueous solution, the pharmaceutical composition comprises:
about 0.1 to about 50 mg/mL, for example, about 0.1 to about 40 mg/mL of the polypeptide compound of Formula I,
citric acid and trisodium citrate at a total molar concentration of about 0.5 to about 50 mM, preferably about 1.0 to about 10 mM, wherein the molar ratio of citric acid to trisodium citrate is about 1:4-50, preferably about 1:4-20,
about 0.1 to about 50 mg/mL, preferably about 1 to about 10 mg/mL, more preferably about 2 to about 6 mg/mL of the preservative,
about 1 to about 100 mg/mL of mannitol,
optionally an aqueous sodium hydroxide solution or hydrochloric acid, and
water,
wherein the aqueous solution has a pH ranging from about 5.0 to about 9.0, for example, from about 5.8 to about 9.0, preferably from about 6.0 to about 8.8, and more preferably from about 6.5 to about 7.4.

In some embodiments, the pharmaceutical composition comprises:
the polypeptide compound of Formula I,
a pH adjusting agent selected from the group consisting of an aqueous sodium hydroxide solution or hydrochloric acid,
a buffer selected from the group consisting of disodium hydrogen phosphate, tromethamine, trisodium citrate, trisodium citrate dihydrate, citric acid-trisodium citrate, citric acid monohydrate-trisodium citrate dihydrate, and a combination thereof,
an osmotic pressure regulator selected from the group consisting of mannitol, sodium chloride, propylene glycol, glycerol, and a combination thereof, and
water.

In some embodiments, the osmotic pressure regulator is mannitol, and the pharmaceutical composition does not comprise a preservative.

In some embodiments, the pharmaceutical composition is an aqueous solution, and based on the total volume of the aqueous solution, the pharmaceutical composition comprises:
about 0.1 to about 50 mg/mL, for example, about 0.1 to about 40 mg/mL of of the compound of Formula I,
citric acid and trisodium citrate at a total molar concentration of about 0.5 to about 50 mM, preferably about 1.0 to about 10 mM of citric acid-trisodium citrate, wherein the molar ratio of citric acid to trisodium citrate is about 1:4-50, preferably about 1:4-20,
about 1 to about 100 mg/mL of mannitol,
optionally an aqueous sodium hydroxide solution or hydrochloric acid, and
water,
wherein the aqueous solution has a pH ranging from about 5.0 to about 9.0, for example, from about 5.8 to about 9.0, preferably from about 6.0 to about 8.8, and more preferably from about 6.5 to about 7.4.

In some embodiments, the pharmaceutical composition is an aqueous solution, and based on the total volume of the aqueous solution, the pharmaceutical composition comprises:
about 0.1 to about 50 mg/mL of the compound of Formula I, for example, about 1 to about 40 mg/mL of Compound P016, P014 or P020,
citric acid and trisodium citrate at a total molar concentration of from 0.5 to about 50 mM, preferably about 1.0 to about 10 mM of citric acid-trisodium citrate, wherein the molar ratio of citric acid to trisodium citrate is about 1:4-50, preferably about 1:4-20,
about 1 to about 100 mg/mL of mannitol,
optionally an aqueous sodium hydroxide solution or hydrochloric acid, and
water,
wherein the aqueous solution has a pH ranging from about 5.0 to about 9.0, for example, from about 5.8 to about 9.0, preferably from about 6.0 to about 8.8, and more preferably from about 6.5 to about 7.4.

In some embodiments, the content or concentration of the polypeptide compound of Formula I is from about 0.1 to about 50 mg/mL, for example, from about 0.1 to about 40 mg/mL, from about 0.2 mg/mL to about 40 mg/mL, from about 0.3 mg/mL to about 40 mg/mL, from about 0.4 mg/mL to about 40 mg/mL, from about 0.5 mg/mL to about 40 mg/mL, from about 0.6 mg/mL to about 40 mg/mL, from about 0.7mg/mL to about 40 mg/mL, from about 0.8 mg/mL to about 40 mg/mL, from about 0.9 mg/mL to about 40 mg/mL, from about 1 to about 40 mg/mL, from about 1.2 to about 40 mg/mL, from about 1.5 to about 40 mg/mL, from about 2.0 to about 40 mg/mL, from about 2.4 to about 40 mg/mL, from about 3.0 to about 40 mg/mL, from about 4.0 to about 40 mg/mL, from about 4.8 to about 40 mg/mL, from about 5.0 to about 40 mg/mL, from about 1 to about 30 mg/mL, from about 1.2 to about 30 mg/mL, from about 1.5 to about 30 mg/mL, from about 2.0 to about 30 mg/mL, from about 2.4 to about 30 mg/mL, from about 3.0 to about 30 mg/mL, from about 4.0 to about 30 mg/mL, from about 4.8 to about 30 mg/mL, from about 5.0 to about 30 mg/mL, from about 0.1 mg/mL to about 30 mg/mL, from about 0.1 mg/mL to about 24 mg/mL, from about 0.1 mg/mL to about 20 mg/mL, from about 0.1 to about 10 mg/mL, from about 1 to about 30 mg/mL, from about 1 to about 24 mg/mL, from about 1 to about 20 mg/mL, from about 1 to about 10 mg/mL, or about 20 to about 30 mg/mL.

In some embodiments, the pH value of the aqueous solution of the pharmaceutical composition is from about 5.0 to about 9.0, for example, from about 5.8 to about 9.0, preferably about 6.0 to about 9.0, for example, from about 6.0 to about 8.8, from about 6.1 to about 9.0, from about 6.1 to about 8.8, from about 6.2 to about 9.0, from about 6.2 to about 8.8, from about 6.3 to about 9.0, from about 6.4 to about 9.0, from about 6.5 to about 9.0, from about 6.5 to about 8.9, from about 6.5 to about 8.8, from about 6.5 to about 8.7, from about 6.5 to about 8.6, from about 6.5 to about 8.5, from about 6.5 to about 8.4, from about 6.5 to about 8.3, from about 6.5 to about 8.2, from about 6.5 to about 8.1, from about 6.5 to about 8.0, from about 6.5 to about 7.9, from about 6.5 to about 7.8, from about 6.5 to about 7.7, from about 6.5 to about 7.6, from about 6.5 to about 7.5, from about 6.5 to about 7.4, from about 6.6 to about 7.4, from about 6.7 to about 7.4, from about 6.8 to about 7.4, more preferably about 6.5 to about 8.5, from about 6.5 to about 8.0, from about 6.3 to about 7.5, from about 6.5 to about 7.5, from about 6.8 to about 7.5, from about 6.8 to about 7.4, from about 7.1 to about 7.5, from about 7.1 to about 7.7, and about 7.2 to about 7.5. In some embodiments, the pH value of the aqueous solution of the pharmaceutical composition is about 6.5, about 6.6, about 6.7, about 6.8, about 6.9, about 7.0, about 7.1, about 7.2, about 7.3, about 7.4, or about 7.5, for example, from about 7.5 or about 7.4.

After the pharmaceutical composition according to the present invention is stored at high temperature, room temperature and/or low temperature conditions for a certain period of time, the following indexes are stable: clarity, color, pH value, API purity, polymer content, and biological activity. When being stored at high temperature, room temperature, and /or low temperature for a certain period of time, no significant change in terms of Appearance, pH value, osmolarity, insoluble particles, active pharmaceutical ingredients, polymer impurities, physical aggregates, precipitations and/or denaturation and the like is found in the pharmaceutical composition according to the present invention upon detection of the color and/or clarity, or upon detection by using a pH meter, osmolarity meter, particle analyzer, HPLC-UV, and size exclusion chromatography (SEC).

Moreover, the polypeptide pharmaceutical composition according to the present invention has acceptable stability of various indexes for up to 1 month at 40°C or 3-6 months at 25°C, wherein the indexes include appearance, concentration, clarity, pH value, osmotic pressure, purity of active pharmaceutical ingredients (API), polymer impurity content, insoluble particles, and the like. These indexes of the present invention meet the current quality standard requirements for GLP-1/GIP dual agonist drugs, and show good stability even when being stored at high temperature.

A stable pharmaceutical formulation means a formulation in which no significant changes is observed when being stored at refrigeration temperature (2-8°C) for at least 3 months, at least 6 months, at least 1 year, at least 2 years, or up to 3 years. Stable liquid formulations include such liquid formulations that exhibit a desired quality profile when being stored at 25°C for 1 month, 3 months, and 6 months, or at 40°C for 1 month. Typical acceptable standards for stability are as follows: the polymer content is no more than about 3% as measured by SEC-HPLC, and the change in the formulation content or in the total amount of related substances (total impurities) is no more than about 10% as measure by HPLC-UV. The pharmaceutical formulation is a colorless to pale yellow, clear or almost clear liquid upon visually inspection. The visible foreign matters and insoluble particles are in compliance with the safety requirements in pharmacopoeia for injection administration, and the biological activity is within the acceptable range of 60-140%.

In some embodiments, the pharmaceutical composition is stably stored for at least 3 months, at least 6 months, at least 12 months, at least 18 months, at least 24 months, or at least 36 months at from about 2 to about 8°C. In some embodiments, the pharmaceutical composition is stably stored for at least 3 months, at least 6 months, at least 12 months, at least 18 months, or at least 24 months at about 25°C. In some embodiments, the pharmaceutical composition is stably stored for at least 7 days, at least 14 days, at least 28 days, at least 1 month, at least 3 months, at least 6 months, at least 12 months, at least 18 months, or at least 24 months at about 40°C.

### [Polypeptide Compound]

According to the present invention, the active pharmaceutical ingredient in the pharmaceutical composition is the compound of the following Formula I, or a pharmaceutically acceptable salt thereof, or a physiologically acceptable salt thereof, or a precursor compound thereof: 
Y-Aib-E-G-T-αMePhe-T-S-D-Y-Aib-I-Aib-L-D-K-Q-A-Q-A-E-F-V-K²⁴-W-L-L-K²⁸-G-G-P-S-S-
G-A-P-P-P-S-K Formula I (SEQ ID NO: 12);
 wherein
1 K, which is selected from the group consisting of K at position 16, K at position 24, K at position 28 and K at position 40, is chemically modified through conjugation to the ε-amino of the K side-chain with ([2-(2-amino-ethoxy)-ethoxy]-acetyl)ₐ-(γ-Glu)_{b}-CO-(CH₂)_{c}-Z, wherein each a is independently selected from an integer of 1, 2 and 3, each b is independently selected from an integer of 1, 2 and 3, and each c is independently selected from an integer of 16, 18 and 20, wherein Z is independently selected from the group consisting of -CH₃, carboxylic acids or carboxylic acid bioisosteres, phosphonates or sulfonates, preferably -COOH; and the C-terminal amino acid is amidated as a C-terminal primary amide.

In the context of the present invention, the term "active pharmaceutical ingredient" (API) is the polypeptide compound of Formula I, which is a GLP-1/GIP dual agonist.

In some specific embodiments, the API refers to polypeptide compounds P007, P008, P014, P019, P013, P015, P016, P017, P018, and P020 according to the present invention, and a combination thereof.

In some specific embodiments, the API refers to polypeptide compound P016 according to the present invention.

In the context of the present invention, unless otherwise specified or clearly contradictory, the terms "polypeptide compound", "polypeptide", "pharmaceutical active ingredient" and "GLP-1/GIP dual agonist " can be used interchangeably.

In some embodiments, in the compound of Formula I,
K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H.

In some embodiments, in the compound of Formula, K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently an integer of 1, and each c is independently selected from an integer of 16, 18 and 20, wherein Z is independently -COOH.

In some embodiments, in the compound of Formula I, K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently selected from an integer of 1 and 3, and each c is independently selected from an integer of 16 and 20, wherein Z is independently -COOH.

In some embodiments, the compound of Formula I is one or more compounds selected from:
Compound P007 (SEQ ID NO:2): K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide. The structure is shown as below: 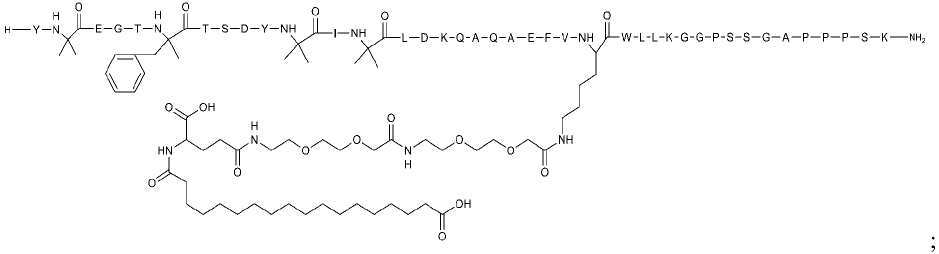
Compound P013 (SEQ ID NO:4): K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide. The structure is shown as below: 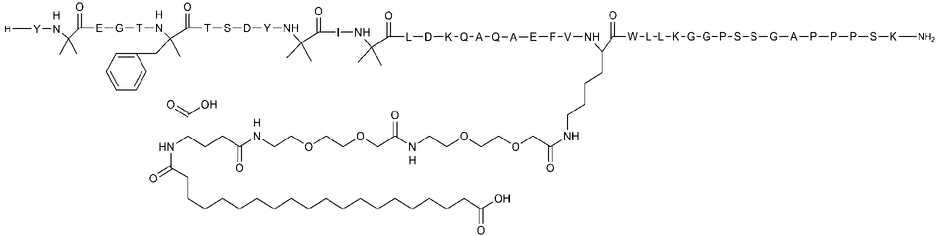 ,
Compound P014 (SEQ ID NO:5): K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide. The structure is shown as below: 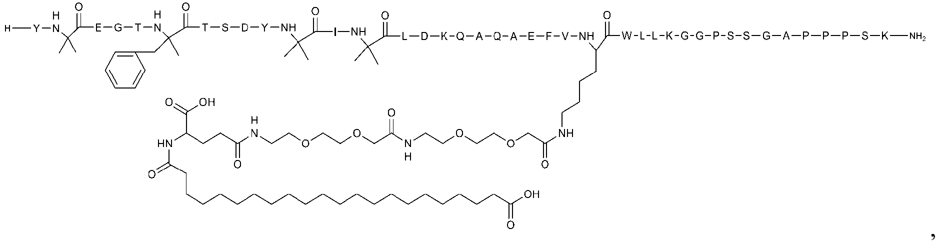
Compound P015 (SEQ ID NO:6): K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide. The structure is shown as below: 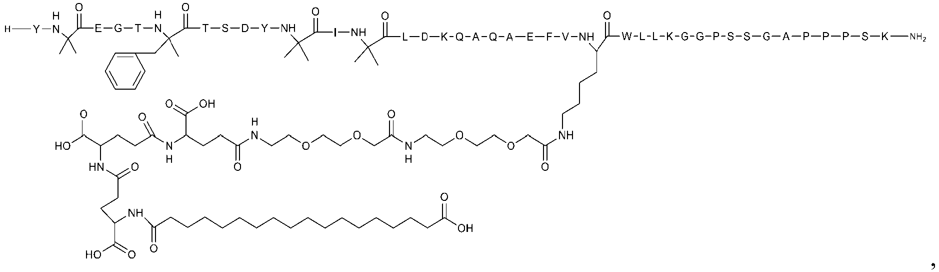
Compound P016 (SEQ ID NO:7): K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide. The structure is shown as below: 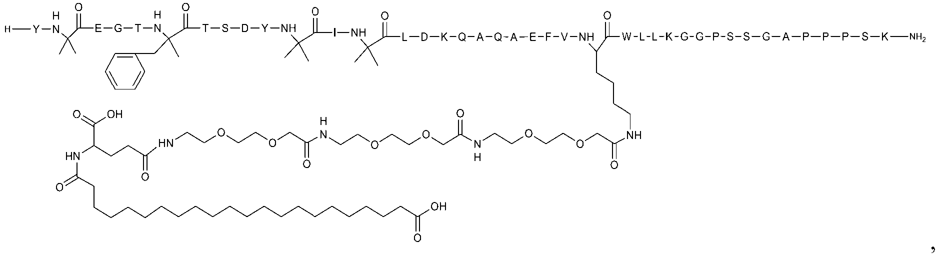
Compound P008 (SEQ ID NO:3): K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide. The structure is shown as below: 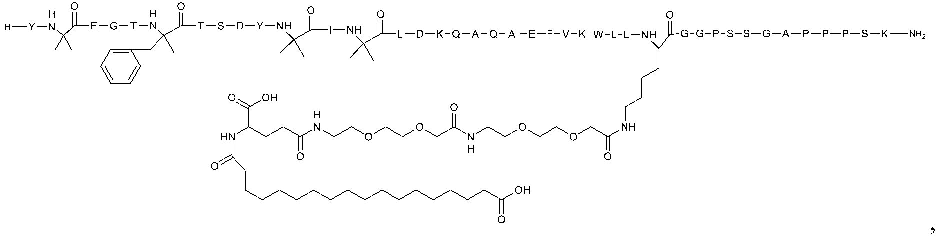
Compound P017 (SEQ ID NO:8): K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide. The structure is shown as below: 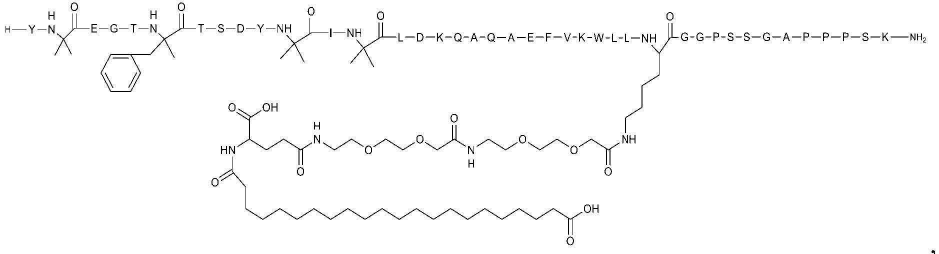
Compound P018 (SEQ ID NO:9): K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide. The structure is shown as below: 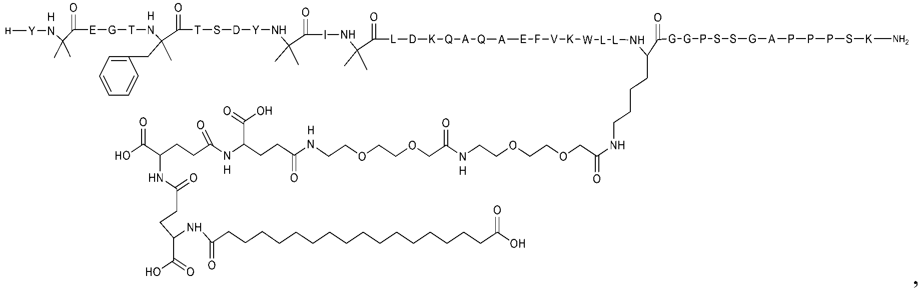
Compound P019 (SEQ ID NO:10): K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide. The structure is shown as below: 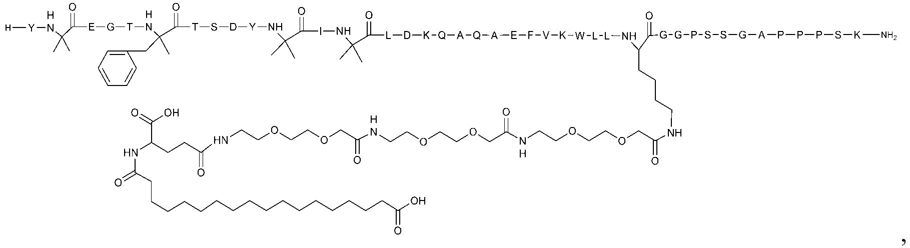 and
Compound P020 (SEQ ID NO:11): K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide. The structure is shown as below:

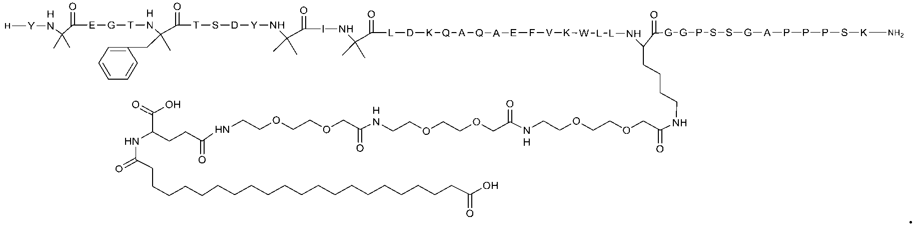

In some embodiments, the compound of Formula I is Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰ -Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-AEEA-γGlu-CO-(CH₂)₂₀-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:12), Molecule Formula: C₂₄₃H₃₈₂N₅₂O₇₃. The structure is shown as below:

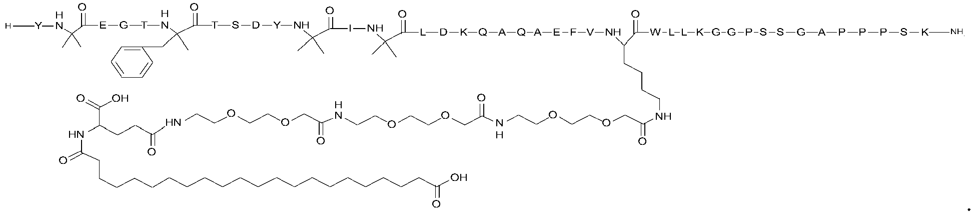

In some embodiments, in the pharmaceutical composition, the concentration of the polypeptide compound of Formula I is from about 0.1 to about 50 mg/mL, for example, from about 0.1 to about 40 mg/mL, from about 0.2 mg/mL to about 40 mg/mL, from about 0.3 mg/mL to about 40 mg/mL, from about 0.4 mg/mL to about 40 mg/mL, from about 0.5 mg/mL to about 40 mg/mL, from about 0.6 mg/mL to about 40 mg/mL, from about 0.7mg/mL to about 40 mg/mL, from about 0.8 mg/mL to about 40 mg/mL, from about 0.9 mg/mL to about 40 mg/mL, from about 1 to about 40 mg/mL, from about 1.2 to about 40 mg/mL, from about 1.5 to about 40 mg/mL, from about 2.0 to about 40 mg/mL, from about 2.4 to about 40 mg/mL, from about 3.0 to about 40 mg/mL, from about 4.0 to about 40 mg/mL, from about 4.8 to about 40 mg/mL, from about 5.0 to about 40 mg/mL, from about 1 to about 30 mg/mL, from about 1.2 to about 30 mg/mL, from about 1.5 to about 30 mg/mL, from about 2.0 to about 30 mg/mL, from about 2.4 to about 30 mg/mL, from about 3.0 to about 30 mg/mL, from about 4.0 to about 30 mg/mL, from about 4.8 to about 30 mg/mL, from about 5.0 to about 30 mg/mL, from about 0.1 mg/mL to about 30 mg/mL, from about 0.1 mg/mL to about 24 mg/mL, from about 0.1 mg/mL to about 20 mg/mL, from about 0.1 to about 10 mg/mL, from about 1 to about 30 mg/mL, from about 1 to about 24mg/mL, from about 1 to about 20 mg/mL, from about 1 to about 10 mg/mL, or about 20 to about 30 mg/mL.

In some embodiments, in the pharmaceutical composition, the concentration of the polypeptide compound of Formula I is at least about 0.1 mg/mL, at least about 1 mg/mL, at least about 10 mg/mL, more preferably at least about 20 mg/mL, even more preferably at least about 30 mg/mL, and still even more preferably at least about 50 mg/mL.

In some embodiments, the concentration of the polypeptide compound of Formula I is from about 0.1 to about 50 mg/mL, for example, about 0.1mg/mL, about 0.2 mg/mL, about 0.3 mg/mL, about 0.4 mg/mL, about 0.5 mg/mL, about 0.6 mg/mL, about 0.7mg/mL, about 0.8 mg/mL, about 0.9 mg/mL, about 1 mg/mL, about 1.2 mg/mL, about 2 mg/mL, about 2.4mg/mL, about 3 mg/mL, about 4 mg/mL, about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, about 20 mg/mL, about 21 mg/mL, about 22 mg/mL, about 23 mg/mL, about 24 mg/mL, about 25 mg/mL, about 26 mg/mL, about 27 mg/mL, about 28 mg/mL, about 29 mg/mL or about 30 mg/mL.

### [pH adjusting agent and Buffer]

In some embodiments, the pharmaceutical composition comprises a pH adjusting agent.

In some embodiments, the pharmaceutical composition does not comprise a pH adjusting agent.

In some embodiments, the pharmaceutical composition comprises a buffer.

In some embodiments, during the preparation of the pharmaceutical composition, after the active ingredient and the excipients other than the pH adjusting agent are mixed, the pH value of the mixture is detected. If the detected pH value is exactly the target pH or close to the target pH value, then no additional pH adjusting agent is needed. If the detected pH value deviates from the target pH value, then a small amount of pH adjusting agent is slowly added until the pH value reaches the target value. Even in the case that a pH adjusting agent is used, those skilled in the art would understand that it is not necessary to specify the amount of the pH adjusting agent, since the amount of the pH adjusting agent added is very small and can be adjusted according to the target pH valule.

In the context of the present invention, both the pH adjusting agent and the buffer can be used to adjust the pH value of the pharmaceutical composition. For the sake of distinction, in the context of the present invention, the "buffer" specifically refers to a substance that has the ability to keep the pH relatively stable through acid-base conjugation, and the "pH adjusting agent" specifically refers to a substance that is different from the "buffer" and has an effect of adjusting the pH.

Buffers and pH adjusting agents commonly used in drugs can be used in the present invention, as long as they do not affect the therapeutic effect and stability of the pharmaceutical composition according to the present invention.

In some embodiments, the buffer comprises an inorganic acid, an organic acid, an inorganic base, an organic base, a combination of an inorganic acid and a salt thereof, a combination of an organic acid and a salt thereof, a combination of an inorganic base and a salt thereof, and a combination of an organic base and a salt thereof.

In the context of the present invention, all the acids, bases and salts may exist in their anhydrous form or as hydrates.

In some embodiments, the salt of an inorganic acid is composed of an inorganic acid radical ion and an alkali metal ion or an ammonium ion. The alkali metal is preferably sodium or potassium.

In some embodiments, the salt of an organic acid is composed of an organic acid radical ion and an alkali metal ion or an ammonium ion. The alkali metal is preferably sodium or potassium.

In some embodiments, the buffer comprises acetic acid, succinic acid, citric acid, phosphoric acid, gluconic acid, oxalic acid, lactic acid, tartaric acid, fumaric acid, 4-hydroxyethylpiperazineethanesulfonic acid (HEPES), tromethamine (Tris), glycine , histidine , glycylglycine, glutamic acid, acetate, succinate, citrate, gluconate, histidine, oxalate, lactate, tartrate, fumarate, phosphate, citric acid-citrate, hydrates thereof, and any combination thereof.

In the context of the present invention, the same substance can be classified as different kinds of components, since it has different functions at the same time. For example, glycine can be used as a buffer, an osmotic pressure regulator, and a stabilizer.

In some embodiments, the buffer is selected from one or more of glycylglycine, histidine, glutamic acid, tromethamine (Tris), 4-hydroxyethylpiperazineethanesulfonic acid (HEPES), citric acid, citrate, phosphate, hydrates thereof, and any combination thereof.

In some embodiments, the buffer is selected from one or more of disodium hydrogen phosphate, tromethamine, trisodium citrate, trisodium citrate dihydrate, citric acid-trisodium citrate, citric acid monohydrate-trisodium citrate dihydrate, and a combination thereof.

In some embodiments, the molar concentration of the buffer in the pharmaceutical composition is from about 0.5 mM to about 40.0 mM, from about 0.5 mM to about 30.0 mM, from about 0.5 mM to about 20.0 mM, from about 0.5 mM to about 10.0 mM, from about 1.0 mM to about 40.0 mM, from about 1.0 mM to about 35.0 mM, from about 1.0 mM to about 30.0 mM, from about 1.0 mM to about 25.0 mM, from about 1.0 mM to about 20.0 mM, from about 1.0 mM to about 15.0 mM, from about 1.0 mM to about 10.0 mM, from about 2.0 mM to 40.0 mM, from about 2.0 mM to about 35.0 mM, from about 2.0 mM to about 30.0 mM, from about 2.0 mM to about 25.0 mM, from about 2.0 mM to about 20.0 mM, from about 2.0 mM to about 15.0 mM, from about 2.0 mM to about 10.0 mM, from about 5.0 mM to about 20.0 mM, from about 5.0 mM to about 15.0 mM, from about 5.0 mM to about 10.0 mM, from about 6.0 to about 9.0 mM, from about 6.0 to about 8.5 mM, from about 6.0 to about 8.0 mM, or about 6.0 to about 7.5 mM.

In some embodiments, the molar concentration of the buffer in the pharmaceutical composition is about 1.0 mM, about 1.5 mM, about 2.0 mM, about 2.5 mM, about 3.0 mM, about 3.5 mM, about 4.0 mM, about 4.5 mM, about 5.0 mM, about 5.5 mM, about 6.0 mM, about 6.5 mM, about 7.0 mM, about 7.5 mM, about 8.0 mM, about 8.5 mM, about 9.0 mM, about 9.5 mM, about 10.0 mM, about 10.5 mM, about 11 mM, about 12.0 mM, about 15.0 mM, about 18.0 mM, or about 20.0 mM.

In some embodiments, the buffer is phosphate, comprising one or more of disodium hydrogen phosphate, sodium dihydrogen phosphate, sodium phosphate, dipotassium hydrogen phosphate, potassium dihydrogen phosphate, potassium phosphate, diammonium hydrogen phosphate, ammonium dihydrogen phosphate, ammonium phosphate, disodium hydrogen phosphate-potassium dihydrogen phosphate, and disodium hydrogen phosphate-citric acid.

In some embodiments, the phosphate is selected from the group consisting of disodium hydrogen phosphate or its hydrate, sodium dihydrogen phosphate or its hydrate, dipotassium hydrogen phosphate or its hydrate, and potassium dihydrogen phosphate or its hydrate.

In some embodiments, the phosphate is a combination of disodium hydrogen phosphate and sodium dihydrogen phosphate, a combination of dipotassium hydrogen phosphate and potassium dihydrogen phosphate, or a phosphate buffer formed by other combination of phosphoric acids or pharmaceutically acceptable salts thereof or hydrates thereof.

In some embodiments, the phosphate is disodium hydrogen phosphate and/or disodium hydrogen phosphate hydrate. The molar concentration of the disodium hydrogen phosphate and/or disodium hydrogen phosphate hydrate in the pharmaceutical composition is from about 0.5 to about 50 mM, for example, from about 0.5 mM to about 40.0 mM, from about 0.5 mM to about about 30.0 mM, from about 0.5 mM to about 20.0 mM, from about 0.5 mM to about 10.0 mM, from about 1.0 mM to about 40.0 mM, from about 1.0 mM to about 35.0 mM, from about 1.0 mM to about 30.0 mM, from about 1.0 mM to about 25.0 mM, from about 1.0 mM to about 20.0 mM, from about 1.0 mM to about 15.0 mM, from about 1.0 mM to about 10.0 mM, from about 2.0 mM to 40.0 mM, from about 2.0 mM to about 35.0 mM, from about 2.0 mM to about 30.0 mM, from about 2.0 mM to about 25.0 mM, from about 2.0 mM to about 20.0 mM, from about 2.0 mM to about 15.0 mM, from about 2.0 mM to about 10.0 mM, from about 5.0 mM to about 20.0 mM, from about 5.0 mM to about 15.0 mM, from about 5.0 mM to about 10.0 mM, from about 6.0 to about 9.0 mM, from about 6.0 to about 8.5 mM, from about 6.0 to about 8.0 mM, or from about 6.0 to about 7.5 mM.

In some embodiments, the molar concentration of the disodium hydrogen phosphate and/or disodium hydrogen phosphate hydrate in the pharmaceutical composition is about 1.0 mM, about 1.5 mM, about 2.0 mM, about 2.5 mM, about 3.0 mM, about 3.5 mM, about 4.0 mM, about 4.5 mM, about 5.0 mM, about 5.5 mM, about 6.0 mM, about 6.5 mM, about 7.0 mM, about 7.5 mM, about 8.0 mM, about 8.5 mM, about 9.0 mM, about 9.5 mM, about 10.0 mM, about 10.5 mM, about 11 mM, about 12.0 mM, about 15.0 mM, about 18.0 mM, or about 20.0 mM.

In some embodiments, the molar concentration of tromethamine in the pharmaceutical composition is from about 0.5 to about 50 mM, for example, from about 0.5 mM to about 40.0 mM, from about 0.5 mM to about about 30.0 mM, from about 0.5 mM to about 20.0 mM, from about 0.5 mM to about 10.0 mM, from about 1.0 mM to about 40.0 mM, from about 1.0 mM to about 35.0 mM, from about 1.0 mM to about 30.0 mM, from about 1.0 mM to about 25.0 mM, from about 1.0 mM to about 20.0 mM, from about 1.0 mM to about 15.0 mM, from about 1.0 mM to about 10.0 mM, from about 2.0 mM to 40.0 mM, from about 2.0 mM to about 35.0 mM, from about 2.0 mM to about 30.0 mM, from about 2.0 mM to about 25.0 mM, from about 2.0 mM to about 20.0 mM, from about 2.0 mM to about 15.0 mM, from about 2.0 mM to about 10.0 mM, from about 5.0 mM to about 20.0 mM, from about 5.0 mM to about 15.0 mM, from about 5.0 mM to about 10.0 mM, from about 6.0 to about 9.0 mM, from about 6.0 to about 8.5 mM, from about 6.0 to about 8.0 mM, or from about 6.0 to about 7.5 mM.

In some embodiments, the molar concentration of tromethamine in the pharmaceutical composition is about 1.0 mM, about 1.5 mM, about 2.0 mM, about 2.5 mM, about 3.0 mM, about 3.5 mM, about 4.0 mM, about 4.5 mM, about 5.0 mM, about 5.5 mM, about 6.0 mM, about 6.5 mM, about 7.0 mM, about 7.5 mM, about 8.0 mM, about 8.5 mM, about 9.0 mM, about 9.5 mM, about 10.0 mM, about 10.5 mM, about 11 mM, about 12.0 mM, about 15.0 mM, about 18.0 mM, or about 20.0 mM.

In some embodiments, the buffer is a citrate, including trisodium citrate, tripotassium citrate, disodium citrate, calcium citrate, magnesium citrate, hydrates thereof, or any combination thereof.

In some embodiments, the buffer is a citric acid-citrate salt, including citric acid-trisodium citrate, citric acid-potassium citrate, citric acid-calcium citrate, citric acid-magnesium citrate, and the like.

In the context of the present invention, "citric acid" and "2-hydroxytricarballylic acid" have the same meaning and can be used interchangeably. "Sodium citrate", "trisodium citrate", "sodium 2-hydroxytricarballylate" and "trisodium 2-hydroxytricarballylate" have the same meaning and can be used interchangeably.

In some embodiments, the citric acid-citrate buffer is citric acid-trisodium citrate or a hydrate thereof. The molar concentration of citric acid-trisodium citrate in the pharmaceutical composition is from about 0.5 to about 50 mM, for example, from about 0.5 mM to about 40.0 mM, from about 0.5 mM to about 30.0 mM, from about 0.5 mM to about 20.0 mM, from about 0.5 mM to about 10.0 mM, from about 1.0 mM to about 40.0 mM, from about 1.0 mM to about 35.0 mM, from about 1.0 mM to about 30.0 mM, from about 1.0 mM to about 25.0 mM, from about 1.0 mM to about 20.0 mM, from about 1.0 mM to about 15.0 mM, from about 1.0 mM to about 10.0 mM, from about 2.0 mM to 40.0 mM, from about 2.0 mM to about 35.0 mM, from about 2.0 mM to about 30.0 mM, from about 2.0 mM to about 25.0 mM, from about 2.0 mM to about 20.0 mM, from about 2.0 mM to about 15.0 mM, from about 2.0 mM to about 10.0 mM, from about 5.0 mM to about 20.0 mM, from about 5.0 mM to about 15.0 mM, from about 5.0 mM to about 10.0 mM, from about 6.0 to about 9.0 mM, from about 6.0 to about 8.5 mM, from about 6.0 to about 8.0 mM, or from about 6.0 to about 7.5 mM, based on the total moles of citric acid and trisodium citrate.

In some embodiments, the molar concentration of trisodium citrate in the pharmaceutical composition is about 1.0 mM, about 1.5 mM, about 2.0 mM, about 2.5 mM, about 3.0 mM, about 3.5 mM, about 4.0 mM, about 4.5 mM, about 5.0 mM, about 5.5 mM, about 6.0 mM, about 6.5 mM, about 7.0 mM, about 7.5 mM, about 8.0 mM, about 8.5 mM, about 9.0 mM, about 9.5 mM, about 10.0 mM, about 10.5 mM, about 11 mM, about 12.0 mM, about 15.0 mM, about 18.0 mM, or about 20.0 mM.

In some embodiments, the molar concentration of citric acid-trisodium citrate or a hydrate thereof in the pharmaceutical composition is about 1.0 mM, about 1.5 mM, about 2.0 mM, about 2.5 mM, about 3.0 mM, about 3.5 mM, about 4.0 mM, about 4.5 mM, about 5.0 mM, about 5.5 mM, about 6.0 mM, about 6.5 mM, about 7.0 mM, about 7.5 mM, about 8.0 mM, about 8.5 mM, about 9.0 mM, about 9.5 mM, about 10.0 mM, about 10.5 mM, about 11 mM, about 12.0 mM, about 15.0 mM, about 18.0 mM, or about 20.0 mM, based on the total moles of citric acid and trisodium citrate.

In some embodiments, the molar ratio of citric acid to trisodium citrate in the citric acid-trisodium citrate buffer is about 1:4-50, for example, about 1:5-50, preferably about 1:6-50, for example, about 1:1-20. In some embodiments, the molar ratio of citric acid to trisodium citrate is about 1:18. In some embodiments, the molar ratio of citric acid to trisodium citrate is about 1:16. In some embodiments, the molar ratio of citric acid to trisodium citrate is about 1:14. In some embodiments, the molar ratio of citric acid to trisodium citrate is about 1:12. In some embodiments, the molar ratio of citric acid to trisodium citrate is about 1:10. In some embodiments, the molar ratio of citric acid to trisodium citrate is about 1:9. In some embodiments, the molar ratio of citric acid to trisodium citrate is about 1:8. In some embodiments, the molar ratio of citric acid to trisodium citrate is about 1:7. In some embodiments, the molar ratio of citric acid to trisodium citrate is about 1:6.15.

In the same solution system, the molar ratio of citric acid to trisodium citrate is equal to the molar concentration ratio of citric acid to trisodium citrate.

In some embodiments, the total molar concentration of citric acid and trisodium citrate is about 7.5 mM, and the molar ratio of citric acid to trisodium citrate is about 1:6.15.

In some embodiments, the total molar concentration of citric acid and trisodium citrate is about 7.5 mM, and the molar ratio of citric acid to trisodium citrate is about 1:9.

In some embodiments, the total molar concentration of citric acid-trisodium citrate is about 5 mM, and the molar ratio of citric acid to trisodium citrate is about 1:9.

The pH adjusting agent comprises one or more of citric acid, sodium hydroxide, and hydrochloric acid (i.e., aqueous HCl solution); preferably sodium hydroxide and/or hydrochloric acid.

In the context of the present invention, "aqueous HCl solution" and "hydrochloric acid" have the same meaning and can be used interchangeably.

In some embodiments, the pH adjusting agent is from about 0.1 to about 0.5 M of aqueous sodium hydroxide solution or aqueous HCl solution.

In some embodiments, the pH adjusting agent is about 0.1 M, about 0.2 M, about 0.3 M, about 0.4 M, or about 0.5 M of aqueous sodium hydroxide solution.

In some embodiments, the pH adjusting agent is about 0.1 M, about 0.2 M, about 0.3 M, about 0.4 M, or about 0.5 M of aqueous HCl solution.

In some embodiments, during the preparation of the pharmaceutical composition, in order to adjust the pH, the aqueous HCl solution and the aqueous sodium hydroxide solution can be alternately used. In other words, the pH adjusting agent can be a combination of an aqueous HCl solution and an aqueous NaOH solution. In some embodiments, the pH adjusting agent can be a combination of about 0.1 to about 0.5 M of an aqueous HCl solution and about 0.1 to about 0.5 M of an aqueous NaOH solution.

### [Osmotic Pressure Regulator]

In some embodiments, the pharmaceutical composition, preferably the aqueous solution of the pharmaceutical composition, comprises an osmotic pressure regulator.

In the context of the present invention, the "osmotic pressure regulator" and the "isotonic regulator" can be used interchangeably. Osmotic pressure regulators commonly used in drugs can be used in the present invention, as long as they do not affect the therapeutic effect and stability of the pharmaceutical composition according to the present invention.

In some embodiments, the osmotic pressure regulator includes, but is not limited to, salts (e.g., sodium chloride, phosphates, citrates, borates, and tartrates), sugars or sugar alcohols (lactose, trehalose, sucrose, glucose, mannitol, sorbitol, xylitol), amino acids (e.g., L-glycine, L-histidine, arginine, lysine, isoleucine, aspartic acid, tryptophan, threonine), polyols (e.g., glycerol, 1,2-propylene glycol (also known as propylene glycol), 1,3-propylene glycol, 1,3-butylene glycol), polyethylene glycols (e.g., PEG 400), or any combination thereof. Phosphates comprise one or more of disodium hydrogen phosphate, sodium dihydrogen phosphate, sodium phosphate, dipotassium hydrogen phosphate, potassium dihydrogen phosphate, potassium phosphate, diammonium hydrogen phosphate, ammonium dihydrogen phosphate, ammonium phosphate, and disodium hydrogen phosphate-potassium dihydrogen phosphate.

In some embodiments, the osmotic regulator is selected from one or more of propylene glycol, mannitol, sorbitol, xylitol, glycerol, lactose, trehalose, sucrose, glucose, sodium chloride, phosphates, trisodium citrate, boric acid and sodium tartrate.

In some embodiments, the osmotic pressure regulator comprises one or more of mannitol, sodium chloride, propylene glycol, and glycerol.

In some embodiments, the pharmaceutical composition has an osmotic pressure of from about 200 to about 500 mOsm/kg, preferably from about 250 to about 350 mOsm/kg, and more preferably from about 260 to about 320 mOsm/kg. The osmotic pressure can be measured by an osmolarity meter.

In some embodiments, the osmotic regulator is sodium chloride.

In some embodiments, the concentration of sodium chloride is from about 1 to 20 mg/mL, preferably from about 5 to 20 mg/mL, more preferably from about 6 to 15 mg/mL, and more preferably from about 6 to 10 mg/mL.

In some embodiments, the concentration of sodium chloride in the pharmaceutical composition is from about 1 mg/mL to about 20 mg/mL, for example, from about 1 mg/mL to about 19 mg/mL, from about 1 mg/mL to about 18 mg/mL, from about 1 mg/mL to about 17 mg/mL, from about 1 mg/mL to about 16 mg/mL, from about 1 mg/mL to about 15 mg/mL, from about 2 mg/mL to about 18 mg/mL, from about 2 mg/mL to about 17 mg/mL, from about 2 mg/mL to about 16 mg/mL, from about 2 mg/mL to about 15 mg/mL, from about 3 mg/mL to about 18 mg/mL, from about 3 mg/mL to about 17 mg/mL, from about 3 mg/mL to about 16 mg/mL, from about 3 mg/mL to about 15 mg/mL, from about 4 mg/mL to about 14 mg/mL, from about 5 mg/mL to about 13 mg/mL, from about 6 mg/mL to about 12 mg/mL, from about 7 mg/mL to about 11 mg/mL, from about 7 mg/mL to about 10.5 mg/mL, from about 7 mg/mL to about 10 mg/mL, from about 7 mg/mL to about 9 mg/mL, from about 7 mg/mL to about 9.5 mg/mL, from about 7 mg/mL to about 9 mg/mL, from about 7 mg/mL to about 8.5 mg/mL, from about 7.5 mg/mL to about 11 mg/mL, from about 7.5 mg/mL to about 10.5 mg/mL, from about 7.5 mg/mL to about 10 mg/mL, from about 7.5 mg/mL to about 9.5 mg/mL, from about 7.5 mg/mL to about 9.0 mg/mL, from about 7.5 mg/mL to about 8.5 mg/mL, from about 8 mg/mL to about 11 mg/mL, from about 8 mg/mL to about 10.5 mg/mL, from about 8 mg/mL to about 10 mg/mL, from about 8 mg/mL to about 9.5 mg/mL, from about 8 mg/mL to about 9 mg/mL. In some embodiments, the concentration of sodium chloride in the pharmaceutical composition is from about 2 mg/mL to about 18 mg/mL. In some embodiments, the concentration of sodium chloride in the pharmaceutical composition is from about 3 mg/mL to about 15 mg/mL. In some embodiments, the concentration of sodium chloride in the pharmaceutical composition is from about 7 mg/mL to about 10 mg/mL, for example, about 7.5 mg/mL, about 7.6 mg/mL, about 7.7 mg/mL, about 7.8 mg/mL, about 7.9 mg/mL, about 8 mg/mL, about 8.1 mg/mL, about 8.2 mg/mL, about 8.3 mg/mL, about 8.4 mg/mL, about 8.5 mg/mL, about 8.6 mg/mL, about 8.7 mg/mL, about 8.8 mg/mL, about 8.9 mg/mL, about 9.0 mg/mL, about 9.1 mg/mL, about 9.28 mg/mL, about 9.3 mg/mL, about 9.4 mg/mL, about 9.5 mg/mL, about 9.6 mg/mL, about 9.7 mg/mL, about 9.8 mg/mL, about 9.9 mg/mL or about 10 mg/mL. In some embodiments, the concentration of sodium chloride in the pharmaceutical composition is from about 7.5 mg/mL to about 9.5 mg/mL.

In some embodiments, the osmotic pressure regulator is propylene glycol.

In some embodiments, the concentration of propylene glycol is from about 5 to 50 mg/mL, preferably from about 10 to 50 mg/mL, more preferably from about 15 to 40 mg/mL, more preferably from about 20 to 40 mg/mL, and more preferably from about 20 to 30 mg/mL.

In some embodiments, the concentration of propylene glycol in the pharmaceutical composition is from about 10 mg/mL to about 20 mg/mL, for example, from about 10 mg/mL to about 19 mg/mL, from about 10 mg/mL to about 18 mg/mL, from about 10 mg/mL to about 17 mg/mL, from about 10 mg/mL to about 16 mg/mL, from about 10 mg/mL to about 15 mg/mL, from about 11 mg/mL to about 20 mg/mL, from about 11 mg/mL to about 19 mg/mL, from about 11 mg/mL to about 18 mg/mL, from about 11 mg/mL to about 17 mg/mL, from about 11 mg/mL to about 16 mg/mL, from about 11 mg/mL to about 15 mg/mL, from about 12 mg/mL to about 20 mg/mL, from about 12 mg/mL to about 19 mg/mL, from about 12 mg/mL to about 18 mg/mL, from about 12 mg/mL to about 17 mg/mL, from about 12 mg/mL to about 16 mg/mL, from about 12 mg/mL to about 15 mg/mL, from about 13 mg/mL to about 20 mg/mL, from about 13 mg/mL to about 19 mg/mL, from about 13 mg/mL to about 18 mg/mL, from about 13 mg/mL to about 17 mg/mL, from about 13 mg/mL to about 16 mg/mL, or about 13 mg/mL to about 15 mg/mL. In some embodiments, the concentration of propylene glycol in the pharmaceutical composition is from about 12 mg/mL to about 16 mg/mL, for example, is about 14 mg/mL.

In some embodiments, the osmotic regulator is mannitol.

In some embodiments, the concentration of mannitol in the pharmaceutical composition is from about 10 mg/mL to about 60 mg/mL, for example, from about 15 mg/mL to about 48 mg/mL, from about 20 mg/mL to about 48 mg/mL, from about 20 mg/mL to about 48 mg/mL, from about 25 mg/mL to about 48 mg/mL, from about 30 mg/mL to about 48 mg/mL, from about 15 mg/mL to about 40 mg/mL, from about 15 mg/mL to about 35 mg/mL, from about 20 mg/mL to about 40 mg/mL, from about 20 mg/mL to about 35 mg/mL, from about 25 mg/mL to about 40 mg/mL, from about 25 mg/mL to about 35 mg/mL, from about 30 mg/mL to about 40 mg/mL, from about 35 mg/mL to about 40 mg/mL. In some embodiments, the concentration of mannitol in the pharmaceutical composition is about 25 mg/mL, about 25.5 mg/mL, about 26 mg/mL, 26.5 mg/mL, about 27 mg/mL, about 27.5 mg/mL, about 28 mg/mL, about 28.5 mg/mL, about 29 mg/mL, about 29.5 mg/mL, about 30 mg/mL, 30.5 mg/mL, about 31 mg/mL, about 31.5 mg/mL, about 32 mg/mL, about 32.5 mg/mL, about 33 mg/mL, 33.5 mg/mL, about 34 mg/mL, 34.5 mg/mL, about 35 mg/mL, 35.5 mg/mL, about 36 mg/mL, 36.5 mg/mL, about 37 mg/mL, 37.5 mg/mL, about 38 mg/mL, 38.5 mg/mL, about 39 mg/mL, 39.5 mg/mL, about 40 mg/mL, about 41 mg/mL, about 41.5 mg/mL, about 42 mg/mL, about 42.5 mg/mL, about 43 mg/mL, about 43.5 mg/mL, about 44 mg/mL, about 44.5 mg/mL, about 45 mg/mL, about 45.5 mg/mL, about 46 mg/mL, about 46.5 mg/mL, about 47 mg/mL, about 47.5 mg/mL, about 48 mg/mL, about 48.5 mg/mL, about 49 mg/mL, about 49.5 mg/mL, or about 50 mg/mL.

### [Preservative]

The pharmaceutical compositions of the present invention may optionally comprise a preservative.

In some embodiments, the pharmaceutical composition comprises a preservative.

In some embodiments, the pharmaceutical composition does not comprises a preservative.

In the context of the present invention, the "preservative" and the "antibacterial agent" can be used interchangeably. Preservatives commonly used in medicines can be used in the present invention as long as they do not affect the therapeutic effect and stability of the pharmaceutical composition according to the present invention.

In some embodiments, the preservative comprises phenol, m-cresol, cresol, phenoxyethanol, chlorobutanol, benzyl alcohol, benzoate deriatives, and any combination thereof.

In some embodiments, the preservative comprises phenol, m-cresol, cresol, chlorobutanol, benzyl alcohol, and any combination thereof. In some embodiments, the concentration of m-cresol in the pharmaceutical composition is from about 0.10 mg/mL to about 10.00 mg/mL, for example, from about 0.5 mg/mL to about 9.00 mg/mL, from about 1.0 mg/mL to about 8.00 mg/mL, from about 1.5 mg/mL to about 7.00 mg/mL, or from about 2.0 mg/mL to about 6.00 mg/mL. In some embodiments, the concentration of m-cresol in the pharmaceutical composition is about 0.50 mg/mL, about 1.00 mg/mL, about 2.00 mg/mL, about 2.10 mg/mL, about 2.20 mg/mL, about 2.30 mg/mL, about 2.40 mg/mL, about 2.50 mg/mL, about 2.60 mg/mL, about 2.70 mg/mL, about 2.80 mg/mL, about 2.90 mg/mL, about 3.00 mg/mL, about 3.50 mg/mL, about 4.00 mg/mL, about 4.50 mg/mL, about 5.00 mg/mL, about 5.50 mg/mL, about 6.0 mg/mL, about 6.5 mg/mL, about 7.0 mg/mL, about 7.5 mg/mL, about 8.0 mg/mL, about 8.5 mg/mL, about 9.0 mg/mL, about 9.5 mg/mL, or about 10.0 mg/mL.

In some embodiments, the concentration of phenol in the pharmaceutical composition is from about 0.10 mg/mL to about 10.00 mg/mL, for example, from about 1.0 mg/mL to about 9.00 mg/mL, from about 2.0 mg/mL to about 8.00 mg/mL, from about 3.0 mg/mL to about 7.00 mg/mL, or from about 4.0 mg/mL to about 6.00 mg/mL. In some embodiments, the concentration of phenol in the pharmaceutical composition is about 0.50 mg/mL, about 0.60 mg/mL, about 1.00 mg/mL, about 2.00 mg/mL, about 3.00 mg/mL, about 4.00 mg/mL, about 4.10 mg/mL, about 4.20 mg/mL, about 4.30 mg/mL, about 4.40 mg/mL, about 4.50 mg/mL, about 4.60 mg/mL, about 5.0 mg/mL, about 5.5 mg/mL, about 6.0 mg/mL, about 6.5 mg/mL, about 7.0 mg/mL, about 7.5 mg/mL, about 8.0 mg/mL, about 8.5 mg/mL, about 9.0 mg/mL, about 9.5 mg/mL, or about 10.0 mg/mL.

In some embodiments, the concentration of benzyl alcohol in the pharmaceutical composition is from about 0.10 mg/mL to about 10.00 mg/mL, for example, from about 0.5 mg/mL to about 9.00 mg/mL, from about 1.0 mg/mL to about 8.00 mg/mL, from about 1.5 mg/mL to about 7.00 mg/mL, or from about 2.0 mg/mL to about 6.00 mg/mL. In some embodiments, the concentration of benzyl alcohol in the pharmaceutical composition is about 0.50 mg/mL, about 1.00 mg/mL, about 2.00 mg/mL, about 2.10 mg/mL, about 2.20 mg/mL, about 2.30 mg/mL, about 2.40 mg/mL, about 2.50 mg/mL, about 2.60 mg/mL, about 2.70 mg/mL, about 2.80 mg/mL, about 2.90 mg/mL, about 3.00 mg/mL, about 3.50 mg/mL, about 4.00 mg/mL, about 4.50 mg/mL, about 5.00 mg/mL, about 5.50 mg/mL, about 6.0 mg/mL, about 6.5 mg/mL, about 7.0 mg/mL, about 7.5 mg/mL, about 8.0 mg/mL, about 8.5 mg/mL, about 9.0 mg/mL, about 9.5 mg/mL, or about 10.0 mg/mL.

### [Antioxidant]

The pharmaceutical composition of the present invention may optionally comprise an antioxidant.

In some embodiments, the pharmaceutical composition comprises an antioxidant.

In some embodiments, the pharmaceutical composition does not comprise an antioxidants.

Antioxidants commonly used in medicines can be used in the present invention as long as they do not affect the therapeutic effect and stability of the pharmaceutical composition according to the present invention.

In some embodiments, the antioxidant is selected from the group consisting of Vitamin E, ascorbic acid, ascorbyl palmitate, butylated hydroxyanisole, sodium bisulfite, sodium sulfite, sodium thiosulfate, or a combination thereof.

In some embodiments, the pharmaceutical composition does not comprise Vitamin E, ascorbic acid, ascorbyl palmitate, butylated hydroxyanisole, sodium bisulfite, sodium sulfite, and sodium thiosulfate.

### [Stabilizer]

The pharmaceutical composition according to the present invention may optionally comprise a stabilizer.

In some embodiments, the pharmaceutical composition comprises a stabilizer to inhibit chemical degradation or polymerization, physical aggregation or precipitation of the polypeptide.

In some embodiments, the pharmaceutical composition does not comprise a stabilizer.

Stabilizers or surfactants commonly used in drugs can be used in the present invention, as long as they do not affect the therapeutic effect and stability of the pharmaceutical composition according to the present invention.

The formulation of the present invention may comprise a stabilizer, which may be selected from the group consisting of conventional surfactants, amino acids, polysaccharides, and metal chelators in the art, for example, a nonionic surfactant and/or an ionic surfactants, specific examples of which may be lipid adjuvants (for example, oleoyl macrogol glyceride Labrafil, caprylic/capric macrogol glyceride Labrasol, propylene glycol dicaprylate/dicaprate Labrafac, Gelucire), Polysorbates (for example, Polysorbate 20 and Polysorbate 80), Poloxamer (for example, Poloxamer 188), Triton, sodium dodecyl sulfate (SDS), sodium lauryl sulfate, sodium octyl glucoside; lauryl-, myristyl-, linoleyl-, or stearoyl-sulfobetaine; lauryl-, myristyl-, linoleyl-, or stearoyl-sarcosine; linoleyl-, myristyl- or cetyl-betaine; lauramidopropyl-, cocoamidopropyl-, linoleamidopropyl-, myristamidopropyl-, palmitamidopropyl-, or isostearamidopropyl-betaine (e.g., lauramidopropyl betaine); myristamidopropyl-, palmitamidopropyl-, or isostearamidopropyl-dimethylamine; sodium methyl cocoyl taurate or disodium methyl oleyl taurate; as well as polyethylene glycol, polypropylene glycol, and copolymers of ethylene glycol and propylene glycol (e.g. Pluronics PF68, etc.). Among them, Polysorbate is preferred, and Polysorbate 80 is more preferred. Based on the total volume of the pharmaceutical composition, the content of the surfactant may be from about 0.01 mg/mL to about 50 mg/mL, and preferably from about 0.5 mg/mL to 50 mg/mL.

In some embodiments, the stabilizer is selected from Tween (e.g., Tween-40), Poloxamer, amino acids (e.g., glycine, histidine), or any combination thereof.

In some embodiments, the pharmaceutical composition does not comprise glycine and Tween.

In some embodiments, the pharmaceutical composition does not comprise Poloxamer and histidine.

### [Use of Pharmaceutical Composition for Treating Diseases, Use in the Manufacture of a Medicament, and Method for Treating Diseases]

In some embodiments, the present invention provides a use of the pharmaceutical composition in the manufacure of a medicament for preventing or treating a disease.

In some embodiments, the pharmaceutical compositions of the present invention are used to for preventing or treating a disease.

In some embodiments, the present invention provides a method for preventing or treating a disease, comprising administering an effective amount of the pharmaceutical composition according to the present invention to a human or an animal in need thereof.

In some embodiments, the disease comprises diabetes mellitus, obesity, a disease associated with diabetes mellitus, and/or a disease associated with obesity.

In some embodiments, the unit dose of the pharmaceutical composition is from about 0.1 mg to about 0.5 mg or from about 0.5 mg to about 50mg, for example, from about 1 to about 40 mg, from about 10 to about 35 mg, from about 20 to about 30 mg, about 0.5mg, about 1mg, about 1.5mg, about 2mg, about 2.5mg, about 3mg, about 3.5mg, about 4mg, about 5 mg, about 10 mg, about 15 mg, about 20 mg, about 25 mg, about 30 mg, about 35 mg, about 40 mg or about 45 mg. In the context of the present invention, the "unit dose" and the "unit administration dose" are synonymous and may be used interchangeably to refer to a quantity of pharmaceutical composition in a single dose administration package, or to a quantity of pharmaceutical composition in a formulation unit that can be independently administered in a multiple dose administration package.

In some embodiments, the disease comprises one or more of the following: non-insulin dependent diabetes mellitus/Type II diabetes mellitus, insulin dependent diabetes mellitus, obesity, non-alcoholic fatty liver, hepatic steatosis, diabetic retinopathy, diabetic neuropathy, diabetic nephropathy, insulin resistance, dyslipidemia associated with insulin resistance, and/or dyslipidemia associated with diabetes mellitus.

In some embodiments, the disease comprises one or more of the following: symptomatic obesity, obesity based on simple obesity, obesity-related conditions or diseases, eating disorders, diabetes mellitus (for example, Type I diabetes mellitus, Type II diabetes mellitus, gestational diabetes mellitus, obesity diabetes mellitus), hyperlipidemia (for example, hypertriglyceridemia, hypercholesterolemia, hyper-LDL-cholesterolemia, low HDL-cholesterolemia, postprandial hyperlipidemia), hypertension, heart failure, complications of diabetes mellitus (for example, neuropathy, nephropathy, retinopathy, diabetic cardiomyopathy, cataract, macroangiopathy, osteopenia, hyperosmolar diabetic coma), infectious disease (for example, respiratory tract infection, urinary tract infection, gastrointestinal infection, superficial soft tissue infection, lower extremity infection), diabetic gangrene, xerostomia, hypacusia, cerebrovascular disorder, peripheral blood circulation disorder, metabolic syndrome (with three or more conditions selected from the group consisting of hypertriglyceridemia (TG), low HDL-cholesterolemia (HDL-C) , hypertension, abdominal obesity, and impaired glucose tolerance), and/or sarcopenia, and the like.

In some embodiments, the disease comprises one or more of the following: endocrine obesity (e.g., Cushing's syndrome, hypothyroidism, insulinoma, obese Type II diabetes mellitus, pseudohypoparathyroidism, hypogonadism), central obesity (e.g. hypothalamic obesity, frontal lobe syndrome, Klein-Lewis syndrome), genetic obesity (e.g., Prader-Willi syndrome, Laurence-Moon-Biedl's syndrome), drug-induced obesity (e.g., steroids, phenothiazines, insulin, sulfonylurea (SU) drugs, β-blocker induced obesity), and the like.

In some embodiments, the conditions or diseases associated with obesity comprise: glucose tolerance disorder, diabetes mellitus (especially Type II diabetes mellitus, obesity diabetes mellitus), abnormal lipid metabolism (synonymous with hyperlipidemia above), hypertension, heart failure, hyperuricemia, fatty liver (including non-alcoholic hepatitis), coronary heart disease (myocardial infarction, angina pectoris), cerebral infarction (cerebral thrombosis, transient ischemic attack), bone/joint disease (knee osteoarthritis, hip osteoarthritis, deformed spondylitis, low back pain), sleep apnea syndrome/Pickwick syndrome, menstrual disorders (abnormal menstrual cycle, amenorrhea, abnormal menstrual symptoms), metabolic syndrome, and the like.

"Effective amount" or "therapeutically effective amount" as disclosed in the present invention refers to the amount or dose of the compound according to the present invention which, upon single or multiple dose administration to the subject, provides the desired effect in the animal under diagnosis or treatment.

"Treatment" as disclosed in the present invention includes attenuating, inhibiting, reversing, slowing, delaying or halting the progression or severity of an existing condition, disease, disorder or symptom. "Prevention" as disclosed in the present invention includes reducing the risk of acquiring a particular disease, disease condition or disorder.

Unless otherwise specified or clearly contradictory, the pharmaceutical composition according to the present invention can be administered to humans and animals (including pets). The animals comprise monkeys, mice, dogs, cats, rabbits, pigs, alpacas, horses, sheep, cattle and the like.

### [Container and Kit]

In some embodiments, the present invention provides an article or a kit, comprising: a container, and the pharmaceutical composition according to the present invention contained in the container.

In some embodiments, the container is a molded bottle or a tubular bottle made of borosilicate glass sealed by an elastic sealing component, for example, a vial, a cartridge, a pre-filled glass needle, or a pre-filled needle. "Pre-filled syringe " refers to a syringe filled with a pharmaceutical composition (i.e., the formulation comprising the GLP-1/GIP dual agonist of the present invention) before being distributed to an end user who will administer the drug to a patient. A pre-filled syringe or pre-filled needle generally comprises: a drug-containing container, which forms a part of the body of the syringe (i.e., the syringe barrel). As used herein, the term "pre-filled glass syringe" refers to a pre-filled syringe, wherein at least the syringe barrel is made of glass.

In the context of the present invention, "prefilled" and "pre-filled" have the same meaning and can be used exchangably.

In some embodiments, the container is an injection pen or device so as to facilitate an accurate, safe and convenient administration.

### [Specific Embodiment of the Pharmaceutical Composition Comprising Polypeptide Compound P016]

Embodiment 1. A pharmaceutical composition, wherein the pharmaceutical composition has a pH ranging from about 5.8 to about 9.0, and comprises from about 0.1 to about 50 mg/mL of the following polypeptide compound:
Compound P016 (SEQ ID NO:7):H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂

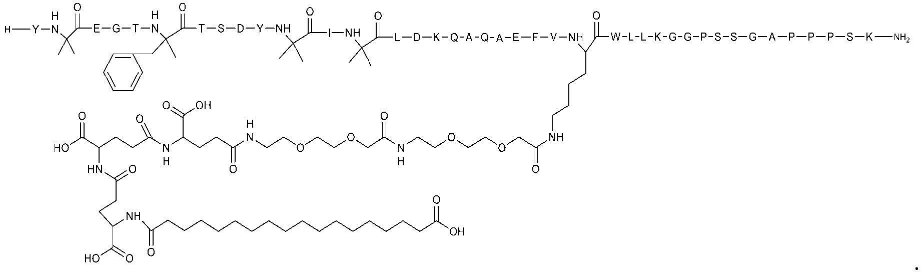

Embodiment 2. The pharmaceutical composition according to Embodiment 1, wherein the pharmaceutical composition is an aqueous solution, and the concentration of the polypeptide compound in the aqueous solution is at least 10 mg/mL, based on the total volume of the aqueous solution.

Embodiment 3. The pharmaceutical composition according to Embodiment 1 or 2, wherein the pharmaceutical composition further comprises an osmotic pressure regulator, a buffer and water, and optionally a pH adjusting agent.

Embodiment 4. The pharmaceutical composition according to Embodiment 3, wherein the buffer is selected from disodium hydrogen phosphate, tromethamine, trisodium citrate, trisodium citrate dihydrate, citric acid-trisodium citrate, citric acid monohydrate-trisodium citrate dihydrate, and a combination thereof.

Embodiment 5. The pharmaceutical composition according to Embodiment 3 or 4, wherein the molar concentration of the buffer is from about 0.5 to about 50 mM, based on the total volume of the aqueous solution.

Embodiment 6. The pharmaceutical composition according to any one of Embodiments 3 to 5, wherein the osmotic pressure regulator is selected from mannitol, sodium chloride, propylene glycol, and a combination thereof.

Embodiment 7. The pharmaceutical composition according to any one of Embodiments 3 to 6, wherein the content of the osmotic pressure regulator is from about 1 to about 100 mg/mL, based on the total volume of the aqueous solution.

Embodiment 8. The pharmaceutical composition according to any one of Embodiments 3 to 7, wherein the pH adjusting agent is an aqueous sodium hydroxide solution or hydrochloric acid.

Embodiment 9. The pharmaceutical composition according to any one of Embodiments 1 to 8, wherein the pharmaceutical composition is an aqueous solution, and based on the total volume of the aqueous solution, the pharmaceutical composition comprises:
from about 0.1 to about 50 mg/mL, for example, from about 1 to about 40 mg/mL of Compound P016,
citric acid and trisodium citrate at a total molar concentration of from 0.5 to about 50 mM, preferably from about 1.0 to about 10 mM of citric acid-trisodium citrate, wherein the molar ratio of citric acid to trisodium citrate is about 1:4-50, preferably about 1:4-20,
from about 1 to about 100 mg/mL of mannitol,
optionally an aqueous sodium hydroxide solution or hydrochloric acid, and
water,
wherein the aqueous solution has a pH ranging from about 5.8 to about 9.0, preferably about 6.0 to about 8.8, and more preferably about 6.5 to about 7.4.

Embodiment 10. A kit comprising a container and the pharmaceutical composition according to any one of Embodiments 1 to 9 contained in the container.

Embodiment 11. Use of the pharmaceutical composition according to any one of Embodiments 1 to 10 in the manufacture of a medicament for treating a disease, such as diabetes mellitus or obesity.

Embodiment 12. The pharmaceutical composition according to any one of Embodiments 1 to 10, for use in treating a disease, such as diabetes mellitus or obesity.

Embodiment 13. A method for preventing or treating a disease, such as diabetes mellitus or obesity, comprising administering an effective amount of the pharmaceutical composition according to any one of Embodiments 1 to 10 to a human or animal in need thereof.

The following examples are intended to illustrate the present invention in detail, and do not constitute a limitation on the scope of the present invention.

### EXAMPLES

### [Preparation of Polypeptide Compounds]

### [Compound Preparation Example 1 - Preparation of Polypeptide Compound P015]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (Polypeptide Compound P015, SEQ ID NO:6).

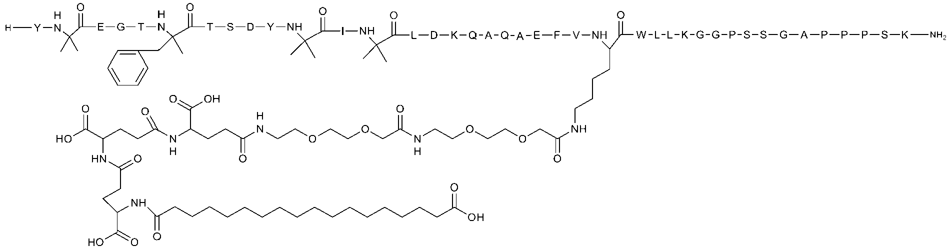

The above structure contains the standard single letter amino acid code with exception of residues Aib2, αMePhe6, Aib11, Aib13 and K24 where the structures of amino acid residues Aib2, αMePhe6, Aib11, Aib13 and K24 have been shown specifically.

Its preparation method applies a strategy of Fmoc solid-phase polypeptide synthesis, and said method comprises:
(1) Synthesis of Peptide-resin Intermediate 1

Fmoc-Rink Linker-Nle-MBHA resin (S=0.49 mmol/g) was charged, swelled with an appropriate amount of DCM, washed with DCM for 2 to 3 times, de-protected with 20% PIP/DMF solution for 30 minutes, filtered and washed to obtain a NH₂-Rink linker-Nle-MBHA resin with Fmoc removed, which was suctioned to remove the solvent for further use.

4 equivalents of Fmoc-Lys(Boc)-OH and HOBt were charged respectively, and dissolved in an appropriate amount of DMF/DCM. Separately, 4 equivalents of DIC was charged and double diluted with DCM. The resultant diluent was slowly added to the DMF/DCM solution, and reacted under stirring at -5 to 0°C for no less than 60 minutes so as to be activated for further use.

The activated Fmoc-Lys(Boc)-OH solution was added to the NH₂-Rink linker-Nle-MBHA resin. The reaction temperature was controlled at 10 to 30°C. The coupling reaction was performed for 240 to 480 minutes. After filtration and washing, a Fmoc-Lys(Boc)-Rink linker-Nle-MBHA resin was obtained. The resin was de-protected with 20% PIP/DMF solution for 30 minutes. After filtration and washing, a Lys(Boc)-Rink linker-Nle-MBHA resin with Fmoc removed was obtained.

According to the reaction conditions as stated above, each amino acid was successively coupled starting from the second amino acid at the C-terminus to the N-terminus. If the coupling was incomplete (a color development reaction occurs), a second condensation was carried out using HBTU/DIEA to ensure that each amino acid was completely condensed. The couplings were successively performed in the following order: Fmoc-Ser(tBu)-OH, Fmoc-Pro-OH, Fmoc-Pro-OH, Fmoc-Pro-OH, Fmoc-Ala-OH.H₂O, Fmoc-Gly-OH, Fmoc-Ser(tBu)-OH, Fmoc-Ser(tBu)-OH, Fmoc-Pro-OH, Fmoc-Gly-Gly-OH, Fmoc-Lys(Boc)-OH, Fmoc-Leu-OH, Fmoc-Leu-OH, Fmoc-Trp(Boc)-OH, Fmoc-Lys(Mtt)-OH, Fmoc-Val-OH, Fmoc-Phe-OH, Fmoc-Glu(OtBu)-OH.H₂O, Fmoc-Ala-OH.H₂O, Fmoc-Gln(Trt)-OH, Fmoc-Ala-OH.H₂O, Fmoc-Gln(Trt)-OH, Fmoc-Lys(Boc)-OH, Fmoc-Asp(OtBu)-OH, Fmoc-Leu-OH, Fmoc-Aib-OH, Fmoc-Ile-OH, Fmoc-Aib-OH, Fmoc-Tyr(tBu)-OH, Fmoc-Asp(OtBu)-OH, Fmoc-Ser(tBu)-OH, Fmoc-Thr(tBu)-OH, Fmoc-α-Me-Phe-OH, Fmoc-Thr(tBu)-OH, Fmoc-Glu(OtBu) - Gly-OH, Fmoc-Aib-OH and Boc-Tyr(tBu)-OH.

The following resin was obtained: Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(Mtt)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink Linker-Nle-MBHA resin.

When the above condensation was completed, the Mtt protecting groups were removed with 50% HFIP/DCM solution for 30 minutes. After washing and filtration, the following peptide-resin intermediate 1 was obtained:
Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val- Lys-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink Linker-Nle-MBHA Resin.

### (2) Modification Steps with Modifying chain

4 equivalents of Fmoc-AEEA-OH and HOBt were charged, and dissolved in an appropriate amount of DMF/DCM. Separately, 4 equivalents of DIC was charged and double diluted with DCM. The diluent was slowly added to the DMF/DCM solution, and reacted under stirring at -5 to 0°C for no less than 60 minutes so as to be activated for further use.

The activated Fmoc-AEEA-OH solution was added to the previously swollen and washed peptide-resin intermediate 1. The reaction temperature was controlled at 10 to 30°C. The coupling reaction was performed for 240 to 480 minutes. The resin was filtrated and washed. The Fmoc protecting groups were removed with 20% PIP/DMF solution for 30 minutes. The resin was filtrated and washed. According to the reaction conditions as stated above, the activated Fmoc-AEEA-OH, Fmoc-AEEA-OH, Fmoc-Glu(α-OtBu)-OH, Fmoc-Glu(α-OtBu)-OH, Fmoc-Glu(α-OtBu)-OH and mono-tert-butyl octadecanedioate were successively coupled on the resin, Fmoc deprotected, washed with DCM and dried to obtain the following P015 peptide-resin: Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(tBuO-Ste-γ-Glu(α-OtBu)-γ-Glu(α-OtBu)-γ-Glu(α-OtBu)-AEEA-AEEA)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink-Linker-Nle-MBHA Resin.

The P015 peptide-resin was charged. 12-15 mL/g cleavage reagent for the peptide-resin (TFA:EDT:TIS:H₂O, 94:2:2:2 v/v) was added and reacted with stirring at 25±5°C for 4 hours. The reaction mixture was filtered with a sand core funnel. The filtrate was collected. The resin was washed with a small amount of TFA for three times, and the filtrates were combined and concentrated under reduced pressure. Methyl tert-butyl ether (MBTE) was added, and the resulting precipitate was washed with MBTE for 3 to 4 times, and dried by evaporating MBTE. The crude product was dried under reduced pressure at room temperature to constant weight to obtain the crude P015 product, which was subsequently purified and dried to obtain the P015 sample (the molecular weight determined by MS was 5255.2).

### [Compound Preparation Example 2 - Preparation of Polypeptide Compound P019]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 28 are chemically modified through conjugation to the ε-aminos of the K side-chains with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (Polypeptide Compound P019, SEQ ID NO:10).

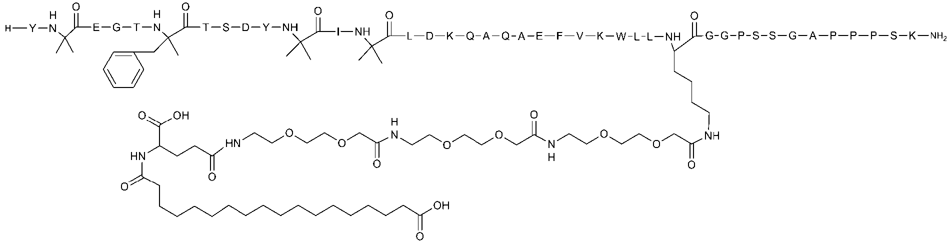

The above structure contains the standard single letter amino acid code with exception of residues Aib2, αMePhe6, Aib11, Aib13 and K28 where the structures of amino acid residues Aib2, αMePhe6, Aib11, Aib13 and K28 have been shown specifically.

Its preparation method applies a strategy of Fmoc solid-phase polypeptide synthesis, and said method comprises:
(1) Synthesis of Peptide-resin Intermediate 2

Fmoc-Rink Linker-Nle-MBHA resin (S=0.49 mmol/g) was charged, swelled with an appropriate amount of DCM, washed with DCM for 2 to 3 times, de-protected with 20% PIP/DMF solution for 30 mins, washed and filtered to obtain a NH₂-Rink linker-Nle-MBHA resin with Fmoc removed, which was suctioned to remove the solvent for further use.

4 equivalents of Fmoc-Lys(Boc)-OH and HOBt were charged respectively, and dissolve in an appropriate amount of DMF/DCM. Separately, 4 equivalents of DIC was charged and double diluted with DCM. The diluent was slowly added to the DMF/DCM solution, and reacted under stirring at -5 to 0°C for no less than 60 minutes so as to be activated for further use.

The activated Fmoc-Lys(Boc)-OH solution was added to the NH₂-Rink linker-Nle-MBHA resin. The reaction temperature was controlled at 10 to 30°C. The coupling reaction was performed for 240 to 480 minutes. After filtration and washing, a Fmoc-Lys(Boc)-Rink linker-Nle-MBHA resin was obtained. The resin was de-protected with 20% PIP/DMF solution for 30 min. After filtration and washing, a Lys(Boc)-Rink linker-Nle-MBHA resin with Fmoc removed was obtained.

According to the reaction conditions as stated above, each amino acid was successively coupled starting from the second amino acid at the C-terminus to the N-terminus. If the coupling was incomplete (a color development reaction occurs), a second condensation was carried out using HBTU/DIEA to ensure that each amino acid was completely condensed. The coupling were successively performed in the following order: Fmoc-Ser(tBu)-OH, Fmoc-Pro-OH, Fmoc-Pro-OH, Fmoc-Pro-OH, Fmoc-Ala-OH.H₂O, Fmoc-Gly-OH, Fmoc-Ser(tBu)-OH, Fmoc-Ser(tBu)-OH, Fmoc-Pro-OH, Fmoc-Gly-Gly-OH, Fmoc-Lys(Mtt)-OH, Fmoc-Leu-OH, Fmoc-Leu-OH, Fmoc-Trp(Boc)-OH, Fmoc-Lys(Boc)-OH, Fmoc-Val-OH, Fmoc-Phe-OH, Fmoc-Glu(OtBu)-OH.H₂O, Fmoc-Ala-OH.H₂O, Fmoc-Gln(Trt)-OH, Fmoc-Ala-OH.H₂O, Fmoc-Gln(Trt)-OH, Fmoc-Lys(Boc)-OH, Fmoc-Asp(OtBu)-OH, Fmoc-Leu-OH, Fmoc-Aib-OH, Fmoc-Ile-OH, Fmoc-Aib-OH, Fmoc-Tyr(tBu)-OH, Fmoc-Asp(OtBu)-OH, Fmoc-Ser(tBu)-OH, Fmoc-Thr(tBu)-OH, Fmoc-α-Me-Phe-OH, Fmoc-Thr(tBu)-OH, Fmoc-Glu(OtBu) - Gly-OH, Fmoc-Aib-OH and Boc-Tyr(tBu)-OH.

The following resin was obtained:
Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(Boc)-Trp(Boc)-Leu-Leu-Lys(Mtt)-Gly-Gly-Pro-Ser(tBu)-Ser(tBu)-Gly-Ala-Pro-Pro-Pro-Ser(tBu)-Lys(Boc)-Rink Linker-Nle-MBHA Resin.

When the above condensation was completed, the Mtt protecting groups were removed with 50% HFIP/DCM solution for 30 minutes. After washing and filtration, the following peptide-resin intermediate 2 was obtained:
Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(Boc)-Trp(Boc)-Leu-Leu-Lys-Gly-Gly-Pro-Ser(tBu)-Ser(tBu)-Gly-Ala-Pro-Pro-Pro-Ser(tBu)-Lys(Boc)-Rink Linker-Nle-MBHA Resin.

### (2) Modification Steps with Modifying chain

4 equivalents of Fmoc-AEEA-OH and HOBt were charged, and dissolve in an appropriate amount of DMF/DCM. Separately, 4 equivalents of DIC was charged and double diluted with DCM. The diluent was slowly added to the DMF/DCM solution, and reacted under stirring at -5 to 0°C for no less than 60 minutes so as to be activated for further use.

The activated Fmoc-AEEA-OH solution was added to the previously swollen and washed peptide-resin intermediate 2. The reaction temperature was controlled at 10 to 30°C. The coupling reaction was performed for 240 to 480 minutes. The resin was filtrated and washed. The Fmoc protecting groups were removed with 20% PIP/DMF solution for 30 minutes. The resin was filtrated and washed. According to the reaction conditions as stated above, the activated Fmoc-AEEA-OH, Fmoc-AEEA-OH, Fmoc-AEEA-OH, Fmoc-Glu(α-OtBu)-OH and mono-tert-butyl octadecanedioate were successively coupled on the resin, Fmoc deprotected, washed with DCM and dried to obtain the following P019 peptide-resin:
Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(tBuO-Ste-γ-Glu(α-OtBu)-AEEA-AEEA-AEEA)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink-Linker-Nle-MBHA Resin.

The P019 peptide-resin was charged. 12-15 mL/g cleavage reagent for the peptide-resin (TFA:EDT:TIS:H₂O, 94:2:2:2 v/v) was added and reacted with stirring at 25±5°C for 4 hours. The reaction mixture was filtered with a sand core funnel. The filtrate was collected. The resin was washed with a small amount of TFA three times, and the filtrates were combined and concentrated under reduced pressure. Methyl tert-butyl ether (MBTE) was added, and the resulting precipitate was washed with MBTE for 3 to 4 times, and dried by evaporating MBTE. The crude product was dried under reduced pressure at room temperature to constant weight to obtain the crude Polypeptide Compound P019 product, which was subsequently purified and dried to obtain the Polypeptide Compound P019 sample (the molecular weight determined by MS was 5142.0).

### [Compound Preparation Example 3 - Preparation of Polypeptide Compound P007]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (Polypeptide Compound P007, SEQ ID NO:2).

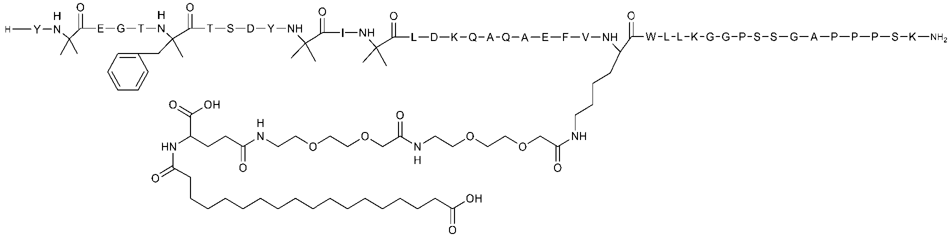

The above structure contains the standard single letter amino acid code with exception of residues Aib2, αMePhe6, Aib11, Aib13 and K24 where the structures of amino acid residues Aib2, αMePhe6, Aib11, Aib13 and K24 have been shown specifically.

Similar to the method described in Compound Preparation Example 1 above, the peptide-resin intermediate 1 was used to carry out the preparation steps to synthesize the peptide of Polypeptide Compound P007 of the present invention (the molecular weight determined by MS was 4997.2).

The difference was that the modification process with the modifying chain was sequentially coupling Fmoc-AEEA-OH, Fmoc-AEEA-OH, Fmoc-Glu(α-OtBu)-OH and mono-tert-butyl octadecanedioate on the resin. The resultant product was de-protected by removing Fmoc group, and washed with DCM and dried to obtain P007 peptide-resin. Other steps were similar to above.

### [Compound Preparation Example 4 - Preparation of Polypeptide Compound P013]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (Polypeptide Compound P013, SEQ ID NO:4).

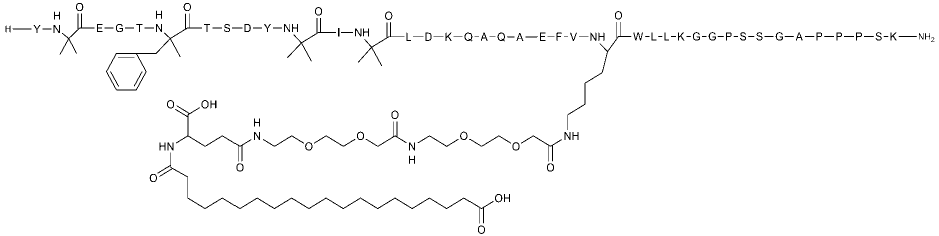

The above structure contains the standard single letter amino acid code with exception of residues Aib2, αMePhe6, Aib11, Aib13 and K24 where the structures of amino acid residues Aib2, αMePhe6, Aib11, Aib13 and K24 have been shown specifically.

Similar to the method described in Compound Preparation Example 1 above, the peptide-resin intermediate 1 was used to carry out the preparation steps to synthesize the peptide of Polypeptide Compound P013 of the present invention (the molecular weight determined by MS was 5025.2). The difference was that the modification process with the modifying chain was sequentially coupling Fmoc-AEEA-OH, Fmoc-AEEA-OH, Fmoc-Glu(α-OtBu)-OH and eicosanedioic acid mono-tert-butyl ester on the resin. The resultant product was de-protected by removing Fmoc group, and washed with DCM and dried to obtain P013 peptide-resin. Other steps were similar to above.

### [Compound Preparation Example 5 - Preparation of Polypeptide Compound P014]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (Polypeptide Compound P014, SEQ ID NO:5).

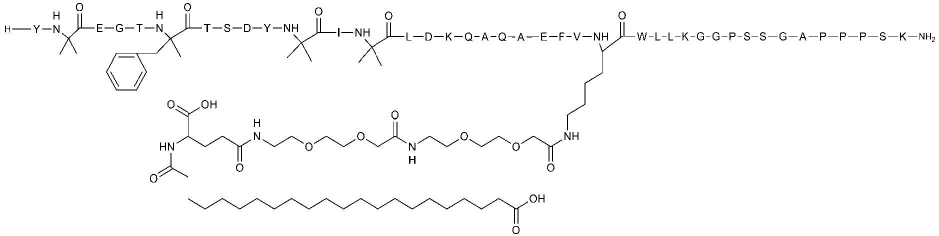

The above structure contains the standard single letter amino acid code with exception of residues Aib2, αMePhe6, Aib11, Aib13 and K24 where the structures of amino acid residues Aib2, αMePhe6, Aib11, Aib13 and K24 have been shown specifically.

Similar to the method described in Compound Preparation Example 1 above, the peptide-resin intermediate 1 was used to carry out the preparation steps to synthesize the peptide of Polypeptide Compound P014 of the present invention (the molecular weight determined by MS was 5052.4). The difference was that the modification process with the modifying chain was sequentially coupling Fmoc-AEEA-OH, Fmoc-AEEA-OH, Fmoc-Glu(α-OtBu)-OH and docosanedioic acid mono-tert-butyl ester on the resin. The resultant product was de-protected by removing Fmoc group, and washed with DCM and dried to obtain P014 peptide-resin. Other steps were similar to above.

### [Compound Preparation Example 6 - Preparation of Polypeptide Compound P016]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (Polypeptide Compound P016, SEQ ID NO:7).

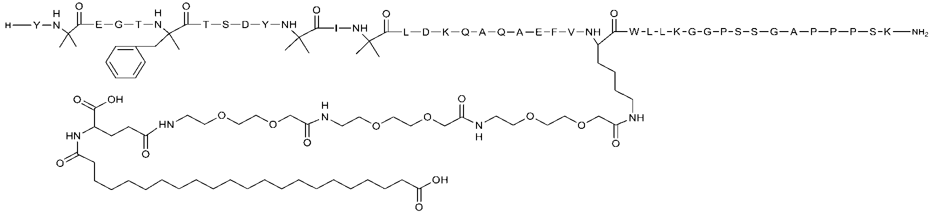

The above structure contains the standard single letter amino acid code with exception of residues Aib2, αMePhe6, Aib11, Aib13 and K24 where the structures of amino acid residues Aib2, αMePhe6, Aib11, Aib13 and K24 have been shown specifically.

Similar to the method described in Compound Preparation Example 1 above, the peptide-resin intermediate 1 was used to carry out the preparation steps to synthesize the peptide of Polypeptide Compound P016 of the present invention (the molecular weight determined by MS was 5198.0). The difference was that the modification process with the modifying chain was sequentially coupling Fmoc-AEEA-OH, Fmoc-AEEA-OH, Fmoc-AEEA-OH, Fmoc-Glu(α-OtBu)-OH and docosanedioic acid mono-tert-butyl ester on the resin. The resultant product was de-protected by removing Fmoc group, and washed with DCM and dried to obtain P016 peptide-resin. Other steps were similar to above.

### [Compound Preparation Example 7 - Preparation of Polypeptide Compound P008]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (Polypeptide Compound P008, SEQ ID NO:2).

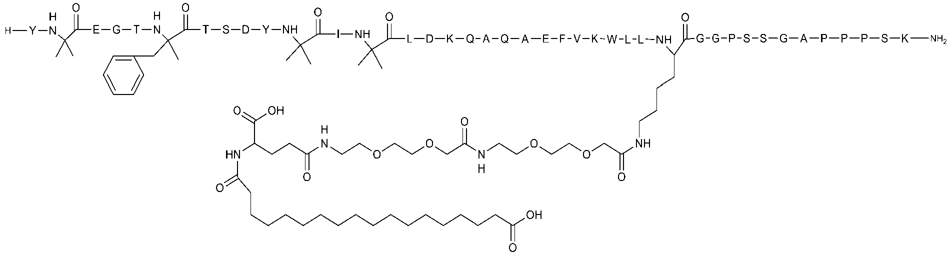

The above structure contains the standard single letter amino acid code with exception of residues Aib2, αMePhe6, Aib11, Aib13 and K28 where the structures of amino acid residues Aib2, αMePhe6, Aib11, Aib13 and K28 have been shown specifically.

Similar to the method described in Compound Preparation Example 2 above, the peptide-resin intermediate 2 was used to carry out the preparation steps to synthesize the peptide of Polypeptide Compound P008 of the present invention (the molecular weight determined by MS was 4996.8). The difference was that the modification process with the modifying chain was sequentially coupling Fmoc-AEEA-OH, Fmoc-AEEA-OH, Fmoc-Glu(α-OtBu)-OH and mono-tert-butyl octadecanedioate on the resin. The resultant product was de-protected by removing Fmoc group, and washed with DCM and dried to obtain P008 peptide-resin. Other steps were similar to above.

### [Compound Preparation Example 8 - Preparation of Polypeptide Compound P017]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (Polypeptide Compound P017, SEQ ID NO:8).

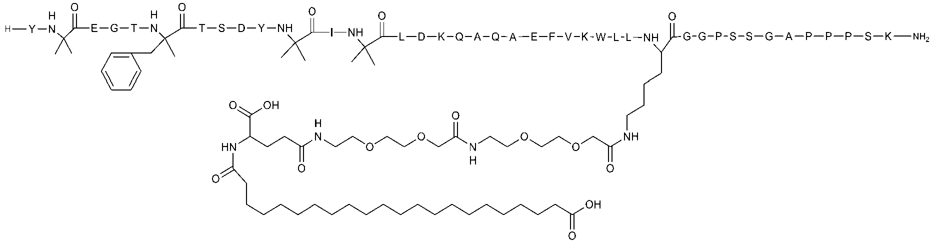

The above structure contains the standard single letter amino acid code with exception of residues Aib2, αMePhe6, Aib11, Aib13 and K28 where the structures of amino acid residues Aib2, αMePhe6, Aib11, Aib13 and K28 have been shown specifically.

Similar to the method described in Compound Preparation Example 2 above, the peptide-resin intermediate 2 was used to carry out the preparation steps to synthesize the peptide of Polypeptide Compound P017 of the present invention (the molecular weight determined by MS was 5052.8). The difference was that the modification process with the modifying chain was sequentially coupling Fmoc-AEEA-OH, Fmoc-AEEA-OH, Fmoc-Glu(α-OtBu)-OH and docosanedioic acid mono-tert-butyl ester on the resin. The resultant product was de-protected by removing Fmoc group, and washed with DCM and dried to obtain P017 peptide-resin. Other steps were similar to above.

### [Compound Preparation Example 9 - Preparation of Polypeptide Compound P018]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (Polypeptide Compound P018, SEQ ID NO:9).

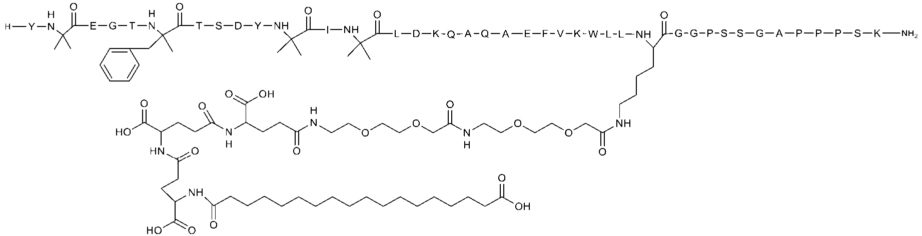

The above structure contains the standard single letter amino acid code with exception of residues Aib2, αMePhe6, Aib11, Aib13 and K28 where the structures of amino acid residues Aib2, αMePhe6, Aib11, Aib13 and K28 have been shown specifically.

Similar to the method described in Compound Preparation Example 2 above, the peptide-resin intermediate 2 was used to carry out the preparation steps to synthesize the peptide of Polypeptide Compound P018 of the present invention (the molecular weight determined by MS was 5255.2). The difference was that the modification process with the modifying chain was sequentially coupling Fmoc-AEEA-OH, Fmoc-AEEA-OH, Fmoc-Glu(α-OtBu)-OH, Fmoc-Glu(α-OtBu)-OH , Fmoc-Glu(α-OtBu)-OH and mono-tert-butyl octadecanedioate on the resin. The resultant product was de-protected by removing Fmoc group, and washed with DCM and dried to obtain P018 peptide-resin. Other steps were similar to above.

### [Compound Preparation Example 10 - Preparation of Polypeptide Compound P020]

Y-X₁-E-G-T-X₂-T-S-D-Y-X₃-I-X₄-L-D-K-Q-A-Q-A-E-F-V-K-W-L-L-K-G-G-P-S-S-G-A-P-P-P-S-K;
wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (Polypeptide Compound P020, SEQ ID NO:11).

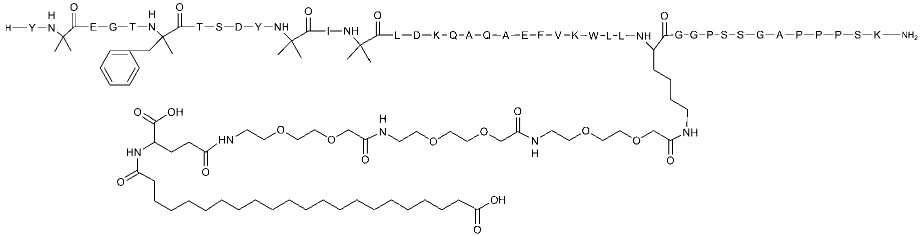

The above structure contains the standard single letter amino acid code with exception of residues Aib2, αMePhe6, Aib11, Aib13 and K28 where the structures of amino acid residues Aib2, αMePhe6, Aib11, Aib13 and K28 have been shown specifically.

Similar to the method described in Compound Preparation Example 2 above, the peptide-resin intermediate 2 was used to carry out the preparation steps to synthesize the peptide of Polypeptide Compound P020 of the present invention (the molecular weight determined by MS was 5198.4). The difference was that the modification process with the modifying chain was sequentially coupling Fmoc-AEEA-OH, Fmoc-AEEA-OH, Fmoc-AEEA-OH, Fmoc-Glu(α-OtBu)-OH and docosanedioic acid ester mono-tert-butyl on the resin. The resultant product was de-protected by removing Fmoc group, and washed with DCM and dried to obtain P020 peptide-resin. Other steps were similar to above.

In some embodiments of the present invention, as for the preparation of the peptide-resin intermediate 3 (a peptide-resin intermediate wherein the modifying chain was bonded to the Lys at position 16) that was Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys-Gln(Trt)-Ala-Gln(Trt)-Ala-Glu(OtBu)-Phe-Val-Lys(Boc)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink Linker-Nle-MBHA Resin, the preparation method for peptide-resin intermediate 1 in Example 1 was referred to.

As for the preparation of the peptide-resin intermediate 4 (a peptide-resin intermediate wherein the modifying chain was bonded to the Lys at position 40) that was Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(Boc)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys-Rink Linker-Nle-MBHA Resin, the preparation method for peptide-resin intermediate 1 in Compound Preparation Example 1 was referred to.

### [Pharmacodynamics Effects of the Polypeptide Compounds]

### [Pharmacodynamics Effect Example 1] Pharmacodynamics Effects of the Polypeptide Compounds in db/db Mice

In this study, db/db mice were subcutaneously single administered, and the changes in the blood glucose, the food intake, and the body weight of the mice were measured to illustrate the hypoglycemic effects of the compounds according to the present invention as well as the duration of the drug effect, and compared them with those of the positive controls of Tirzepatide and P001. In this study, 8-9 weeks old male db/db mice were used. The db/db mice were placed in an individual ventilated cage (IVC) facility with a controlled temperature (20-26°C) and humidity (40-70%) and a controlled 12h:12 h light-dark cycle. The mice can receive food and water ad libitum. Blood was sampled at the tail tip, and the base blood glucose was measured by a Roche blood glucose meter. The mice were randomly divided into groups according to the initial blood glucose and initial body weight (n=6/group), and each group had similar body weight and blood glucose.

The compound according to the present invention (10 nmol/kg), or the positive controls of Tirzepatide (10 nmol/kg) and P001 (10 nmol/kg), were dissolved in a vehicle (PBS containing 0.1% Tween 20, pH 7.2-7.4). After a single subcutaneous administration, the random blood glucoses were recorded at the set time point (0 to 120 hours, the recording of the random blood glucose was stopped until there was no difference between the random blood glucose of the compound and that of the vehicle group), and the daily body weight and food intake were also recorded. The data results were statistically analyzed using GraphPad Prism8, and the statistical differences among the groups were analyzed following a T-Test. Significant differences were identified at *p*<0.05.

Hypoglycemic Duration: the longest time during which the random blood glucose of the compound has a significant difference (*p*<0.05) vs. the vehicle group.

**Table 1-1. Hypoglycemic Duration of the Compounds**

| Group | Compound | Hypoglycemic Duration (h) |
|---|---|---|
| Group 1 | P001* | 32 |
| | Tirzepatide | 56 |
| | P007 | 56 |
| | P008 | 72 |
| | P014 | 104 |
| | P019 | 56 |
| Group 2 | Tirzepatide | 48 |
| | P013 | 72 |
| | P015 | 32 |
| | P016 | 104 |
| | P017 | 72 |
| | P018 | 48 |
| | P020 | 104 |

| | | |
|---|---|---|
| * Compound P001 (SEQ ID NO:1) has the same peptide chain as Formula I, except that it is not modified with the side chain ε-amino group at positions 24K and 28K. | | |

As compared with P001, the hypoglycemic duration of the compounds according to the present invention was more than 24 hours (P007, P008, P014, P019), even more than 40 hours (P008, P014), and more than 72 hours (P014) longer than that of P001.

As compared with Tirzepatide, the hypoglycemic duration of the compounds according to the present invention was equivalent or longer (P007, P008, P013, P014, P016, P017, P018, P019, and P020), even more than 16 hours (P008, P013, P014, P016, P017, P020), more than 24 hours (P013, P014, P016, P017, P020), more than 48 hours (P014, P016, P020), more than 56 hours (P016, P020) longer than that of Tirzepatide.

As compared with P001, based on the experiments according to the method described above, during the test period, the hypoglycemic effects of P008 (*p*<0.01), P014 (*p*<0.001) and P019 (*p*<0.05) were significantly better than that of P001 (blood glucose AUC, *p*<0.05). The inhibition of the blood glucose AUC was 2.3, 2.6 and 2.2 times of that of P001 respectively; the duration of hypoglycemic effect was 40 hours, 72 hours and 24 hours longer than that of P001 respectively (Table 13). The hypoglycemic effect of P007 was comparable to that of P001, but the duration of hypoglycemic effect was 24 hours longer than that of P001.

As compared with Tirzepatide: the hypoglycemic effects of P014 (*p*<0.05), P016 (*p*<0.05) and P020 (*p*<0.05) were stronger than that of Tirzepatide (blood glucose AUC, p< 0.05). The inhibitions of the blood glucose AUC were 1.7, 2.2 and 2.0 times of that of Tirzepatide respectively. Moreover, they also show significantly longer duration of drug effects (48 hours, 56 hours and 56 hours longer than that of Tirzepatide respectively, Table 9). The hypoglycemic effects of P013 and P017 were comparable to that of Tirzepatide, but the duration of drug effects was 24 hours longer than that of Tirzepatide.

The acylated GLP-1/GIP agonists according to the present invention significantly improve the hypoglycemic effect. The compounds according to the present invention with modifying chains in different forms can realize stronger and longer-lasting hypoglycemic effects than P001. Moreover, some compounds according to the present invention exhibit stronger and longer-lasting hypoglycemic effects than Tirzepatide.

### [Pharmacodynamics Effect Example 2] Weight Loss Effect in DIO Mice Obesity Models of Polypeptide Compound 016

In this study, DIO mice were subcutaneously administrated multiple times with the Polypeptide Compound P016 according to the present invention, and the changes in the body weight and blood glucose of the mice were determined to illustrate the weight reduction effect of the compound according to the present invention, and were compared with those of the positive control Tirzepatide. In this study, 26-week-old male DIO mice were used. DIO mice were housed in appropriate cages. DIO mice were housed in an environment-monitored and well-ventilated SPF-grade animal room, with the temperature maintained between 20 and 26°C and relative humidity between 40% and 70%. A 12-hour light/dark cycle was provided for illumination. The mice received food and water ad libitum. In the experiment, DIO mice with similar random blood glucoses (RBG) (D1) and body weights (D1) were selected and randomly grouped (n=10/group). The compound P016 of the present invention (0.3, 1, 3, 30 nmol/kg) or the positive control Tirzepatide (1, 30 nmol/kg) was dissolved in a vehicle (PBS containing 0.1% Tween20, pH 7.2-7.4). The mice were subcutaneously administrated once every 3 days for 4 consecutive weeks. Subcutaneous injection administration was conducted on Day 1, 4, 7, 10, 13, 16, 19, 22, 25, and 28.

The animals were weighed before administration on the day of first administration (D1), and at a fixed time once every 3 days after administration. Food intake was measured once daily after administration. RBG was measured before grouping and at 48 h after each administration. After fasted for 5 h on D30 (no water deprivation was required), FBG and glycated hemoglobin A1c (HbA1c) were detected, and blood was collected intravenously to separate the serum. A mouse insulin ELISA kit was used to detect the insulin content in the serum, and a biochemistry analyser was used to detect total cholesterol (TC), TG, low-density lipoprotein cholesterol (LDL), high-density lipoprotein cholesterol (HDL), free fatty acid (FFA), ALT, and aspartate aminotransferase (AST) content. The liver was collected, the perirenal and paratesticular fat tissues were separated and weighed, the organ-body ratio was calculated. The liver was stained with HE and Oil Red O for pathological scoring. The data results were statistically analyzed using one-way analysis of variance (ANOVA). A Levene's test was performed to test for variance homogeneity. If the result was homogeneous (P>0.05), a one-way analysis of variance (ANOVA) would be performed. If ANOVA showed significance (P≤0.05), a Tukey test of variancpair-wise comparisons would be performed. In the case of heterogeneity of variance (P ≤0.05 ), a Dunnett's T3 test would be performed. Results were expressed as mean and standard deviation (Mean ± SEM). The test level was 0.05. Both statistical significance and biological significance were considered when analyzing the results.

In the experiment conducted as described in the above method, the Polypeptide Compound P016 according to the present invention significantly reduced the cumulative food intake and body weight of obese mice in a dose-dependent manner. The minimum effective dose was 1 nmol/kg, and the weight loss effect at this dose was significantly better than the positive control Tirzepatide at the same dose. At a dose of 30 nmol/kg, the weight loss degree of the compound according to the present invention was greater than that of Tirzepatide (percentage of weight loss, 39.97% vs 34.47%). The compound according to the present invention significantly reduced the total abdominal fat (sum of perirenal fat and paratesticular fat) and serum TC in DIO mice, and had significant lipid-lowering effect. 1, 3, and 30 nmol/kg of the polypeptide compound P016 according to the present invention significantly reduced FBG and insulin level on D30, and improved insulin resistance. The compound according to the present invention significantly reduced liver weight and serum ALT level and improved liver function.

**Table 1-2. Cumulative food intake, body weight, fat and liver weights of DIO mice**

| | Cumulative food intake | Body weight (g) | Total abdominal fat (g) | Liver weight (g) |
|---|---|---|---|---|
| Vehicle | 78.83±0.73 | 48.0±0.5 | 3.071±0.115 | 1.758±0.072 |
| P016 0.3 nmol/kg | 75.75±2.81 | 45.4±1.3 | 2.631±0.069 | 1.552±0.106 |
| P016 1 nmol/kg | 61.40±1.58^{****####} | 38.3±1.1^{****##} | 1.849±0.181^{***} | 1.191±0.075^{***} |
| P016 3 nmol/kg | 55.47:1::2.72^{****} | 34.1±0.7^{****} | 0.972±0.076^{****} | 1.069±0.029^{****} |
| P016 30 nmol/kg | 48.38±2.31^{****} | 29±0.7^{****} | 0.461±0.034^{****} | 0.995±0.029^{****} |
| Tirzepatide 1 nmol/kg | 76.51±1.67 | 44.7±0.87 | 2.318±0.123^{**} | 1.305±0.073^{**} |
| Tirzepatide 30 nmol/kg | 47.95±2.14^{****} | 31.8±0.7^{****} | 0.781±0.093^{****} | 0.951±0.031^{****} |

| | | | | |
|---|---|---|---|---|
| Note: ***p*≤0.01, ****p*≤0.001, *****p*≤0.0001 were compared with the Vehicle group, ##*p*≤0.01, ####*p*≤0.0001 were compared with the same dose of positive control Tirzepatide group. Results were expressed as Mean±SEM for 10 mice. | | | | |

**Table 1-3. Fasting blood glucose, insulin and insulin resistance index in DIO mice**

| | Fasting blood glucose | Insulin | Insulin resistance index |
|---|---|---|---|
| Vehicle | 14.84±0.67 | 2.911±0.294 | 41.698±4.071 |
| P016 0.3 nmol/kg | 13.78±0.39 | 3.285±0.481 | 43.94±6.288 |
| P016 1 nmol/kg | 11.23±0.40^{****#} | 1.911±0.29 | 21.532±L3.705^{*} |
| P016 3 nmol/kg | 10.36±0.67^{****} | 1.196±0.185* | 11.991±1.907^{***} |
| P016 30 nmol/kg | 8.39±0.30^{****} | 0.624±0.130^{**} | 5.179±1.178^{****} |
| Tirzepatide 1 nmol/kg | 9.19±0.35^{****} | 2.317±0.511 | 20.983±4.926 |
| Tirzepatide 30 nmol/kg | 7.96±0.33^{****} | 1.418±0.535 | 11.023±4.147^{*} |

| | | | |
|---|---|---|---|
| Note: **p*≤0.05, ***p*≤0.01, ****p*≤0.001, *****p*≤0.0001 were compared with the Vehicle group, #P≤0.05 was compared with the same dose of positive control Tirzepatide group. Results were expressed as Mean±SEM for 10 mice. | | | |

**Table 1-4. Serum TC and ALT of DIO mice**

| | Serum TC | ALT |
|---|---|---|
| Vehicle | 4.48±0.24 | 120.90±16.22 |
| P016 0.3 nmol/kg | 4.07±0.26 | 95.60±14.09 |
| P016 1 nmol/kg | 3.26±0.20^{*} | 43.90±3.86^{*} |
| P016 3 nmol/kg | 2.61±0.13^{***} | 52.80±9.62^{*} |
| P016 30 nmol/kg | 2.50±0.18^{****} | 49.60±5.67^{*} |
| Tirzepatide 1 nmol/kg | 3.54±0.15 | 56.20±4.66^{*} |
| Tirzepatide 30 nmol/kg | 2.65±0.08^{***} | 41.50±5.31^{*} |

| | | |
|---|---|---|
| Note: **p*≤0.05, ****p*≤0.001, *****p*≤0.0001 were compared with the Vehicle group. Results were expressed as Mean±SEM for 10 mice. | | |

In summary, in this experimental system, the Polypeptide Compound P016 of the present invention could effectively reduce the weight of DIO mice after 4 weeks of subcutaneous injection. The minimum effective dose was 1 nmol/kg. The weight loss effect at this dose was significantly better than the positive control Tirzepatide at the same dose, and the maximum weight loss of the compound according to the present invention (30 nmol/kg) was greater than Tirzepatide at the same dose (percentage of weight loss, 39.97% vs 34.47%). In addition, the compound of the present invention exhibited effects on lowering blood glucose, improving insulin resistance, and improving liver function.

### [Method for Measuring the Indexes of the Composition]

The stability of various indexes for each composition sample under different storage conditions over time was measured respectively, wherein the appearance, clarity, color, osmotic pressure, pH value, purity, and polymer content of the solution are taken as the indexes.

### [Appearance]

An appropriate amount of a composition sample to be tested is taken and observed directly in a brightly lit place.

### [Visible Foreign matter]

An appropriate amount of a composition sample to be tested is taken and inspected according to the visible foreign matter inspection method (Pharmacopoeia of the People's Republic of China 2020 Edition, Volume Four, General Chapters 0904).

### [pH Value]

The pH value for each composition sample is measured by using a pH meter or a micro pH meter.

### [Clarity and Color] Visual Inspection Method

An appropriate amount of the composition sample to be tested and the same volume of standard turbidity solution are taken and placed into a colorless transparent test tube or vial respectively, and are both placed vertically under a Clarity Testing Umbrella Lamp with an illumination of 1000 lx in a dark room, and observed horizontally. The observations were compared. The turbidity of the aqueous solution sample is defined as "clear" if the turbidity does not exceed that of the 0.5 NTU (Nephelometric Turbidity Units) standard solution. The turbidity of the aqueous solution sample is defined as "almost clear" if the turbidity is between that of the 0.5 NTU standard solution and the 1 NTU standard solution. Other turbidities correspond to the tubidity values of the corresponding turbidity standard solution.

An appropriate amount of the composition sample to be tested and the same volume of standard colorimetric solution are taken and placed into a Nessler colorimetric tubes or colorless transparent vial. Both the sample and the comparative colorimetric solution are placed on a white background. The color of the sample and that of the comparative standard colorimetric solution are compared by horizontal observation, and the color of the sample is determined. The color of the aqueous solution sample is defined as "colorless" if the color is identical with that of water or the solvent used. The color of the aqueous solution sample is defined as "almost colorless" if the color is not darker than that of the corresponding 0.5 NTU standard solution. Other color numbers correspond to the hue value of the corresponding standard colorimetric solutions.

### [Insoluble particles]

An appropriate amount of a composition sample to be tested is taken and inspected according to the insoluble particle inspection method (Pharmacopoeia of the People's Republic of China 2020 Edition, Volume Four, General Chapters 0903).

### [Purity or Related Substances] High-Performance Liquid Chromatography (HPLC Method)

**Method I:** An appropriate amount of the composition sample to be tested is taken and dissolved in water, and diluted into about 1.0 mg/mL of an aqueous solution sample. Chromatographic conditions: YMC-Pack ODS-AQ 150×4.6 mm, 3.0 µm chromatographic column; mobile phase A is 25 mM aqueous potassium dihydrogen phosphate solution, and the pH is adjused to 2.1-2.3 with sulfuric acid or phosphoric acid, mobile phase B is acetonitrile; gradient elution is performed; the detection wavelength is 220 nm; the column temperature is 40-45°C; the flow rate is 1.0 mL/min; the injection volume is 20 µL.

Purity refers to the chromatographic purity (%) of the main peak of the active pharmaceutical ingredient (API) in the composition.

### Method II:

### Chromatographic conditions

Chromatographic column: octadecylsilane bonded silica gel as filler (YMC-Pack ODS-AQ 150×4.6mm 3µm chromatographic column);
Mobile phase A: 100mM potassium dihydrogen phosphate solution-acetonitrile (90:10);
Mobile phase B: 100mM potassium dihydrogen phosphate solution-acetonitrile (40:60);
Flow rate: 1.0 mL/min. Detection wavelength: 220 nm; column temperature: 45°C; injection volume: 10 µL.

The content of related substances (total impurities) refers to the chromatographic peak area percentage (%) of all impurities contained in the composition sample.

### [Polymer Content] Size Exclusion Chromatography (SEC Method)

An appropriate amount of the composition sample to be tested is taken and dissolved in water, and diluted into about 0.25 to about 0.3 mg/mL of an aqueous P016 solution sample.

System Suitability Solution: an appropriate amount of Polypeptide Compound P016 reference according to the present invention is taken and degraded at 100°C for 1 hour, natually cooled down to room temperature, dissolved in water and diluted into about 0.25 mg/mL of a system suitability solution.

Chromatographic Conditions: hydrophilic modified silica gel as filler, TSKgel G2000SWxl, 300 mm×7.8 mm, 5 µm column; mobile phase: trifluoroacetic acid-acetonitrile-water (0.1:30:70); isocratic elution for 35 minutes; column temperature: 35°C; detection wavelength: 210 nm; flow rate: 0.5 mL/min; injection volume: 10 or 20 µL.

System suitability requires that the resolution between the peaks of the polymer impurites and the main peak of Polypeptide Compound P016 (API) in the chromatography of the system suitability solution should not be less than 1.5.

Polymer content refers to the percentage (%) of the chromatographic peak area of polymer impurities contained in the active pharmaceutical ingredient (API) in the composition.

### [Molar Concentration Osmotic Pressure]

### Method I

Instruments and reagents: osmolarity tester, pipette gun, 200 mOsmol/kg standard sodium chloride solution, 300 mOsmol/kg standard sodium chloride solution, 400 mOsmol/kg standard sodium chloride solution.

### Operation Method:

Zero-point calibration: using a pipette to inject 60µL of purified water into a clean and dry test tube, making sure there are no visible bubbles in it, performing calibration, pushing the test tube into the support seat to the stop position, and making the temperature measurement probe completely enter the purified water in the test tube. The test results should comply with 0±2 mOsmol/kg H₂O.

Calibration using standard solutions: Taking 60uL of 200 mOsmol/kg, 300 mOsmol/kg and 400 mOsmol/kg standard sodium chloride solutions in sequence, placing them in a clean and dry test tube (making sure there are no visible bubbles in it), and pushing the test tube into the support seat to the stop position so that the temperature measurement probe completely enters the standard sample in the test tube and calibrating the instrument. The test results should all meet ±2 mOsmol/kg H₂O.

Sample measurement: Taking 60 µL of the composition sample to be tested, injecting it into the test tube (making sure there are no visible bubbles in it), pushing the test tube into the support seat to the stop position, so that the temperature measurement probe completely enters the test sample in the test tube, and measuring the molar concentration osmotic pressure of the sample solution.

### Method II

Instruments and reagents: osmolarity tester, pipette gun, 200 mOsmol/kg standard sodium chloride solution, 400 mOsmol/kg standard sodium chloride solution.

### Operation Method:

Zero-point calibration: using a pipette to inject 60µL of purified water into a clean and dry test tube, making sure there are no visible bubbles in it, performing calibration, pushing the test tube into the support seat to the stop position, and making the temperature measurement probe completely enter the purified water in the test tube. The test results should comply with 0±3 mOsmol/kg H₂O.

Calibration using standard solutions: Taking 60uL of 200 mOsmol/kg and 400 mOsmol/kg standard sodium chloride solutions in sequence, placing them in a clean and dry test tube (making sure there are no visible bubbles in it), and pushing the test tube into the support seat to the stop position so that the temperature measurement probe completely enters the standard sample in the test tube, and calibrating the instrument. The test results should all meet ±3 mOsmol/kg H₂O.

Sample measurement: Taking 60 µL of the composition sample to be tested, injecting it into the test tube (making sure there are no visible bubbles in it), pushing the test tube into the support seat to the stop position, so that the temperature measurement probe completely enters the test sample in the test tube, and measuring the molar concentration osmotic pressure of the sample solution.

### [Composition Example 1] pH Range Screening - Phosphate and Sodium Chloride System

According to Table 2-1 below, 80 mg of NaCl and 14.2 mg of Na₂HPO₄ were weighed into a beaker respectively, into which 8 mL of purified water was added with stirring for dissolution. 300 mg of Polypeptide Compound P016 (API) was added to the beaker with stirring until dissolved. The pH value of the resulting mixture was measured. Then a 0.5 M NaOH or 0.1 M HCl aqueous solution was added to adjust the pH value to the target pH values as shown in Table 2-1 below. Purified water was added to adjust the volume to 10 mL, and the mixture was mixed homogeneously and filtered through a 0.22 µm PES membrane, thereby obtaining Composition A1, Composition A2 and Composition A3 having the following composition: 30 mg/mL of API, 8 mg/mL of NaCl, and 10 mM Na₂HPO₄.

A composition sample to be tested was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

After resting for a period of time, the resulting compositions were measured for clarity, color, and purity over different time at different temperatures according to the measurement methods as described above, and the results were recorded in Tables 2-2 and 2-3 below.

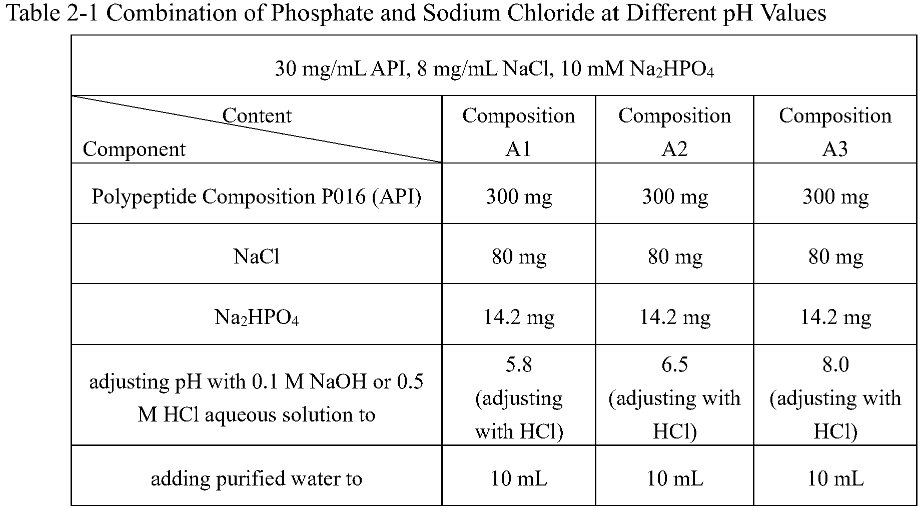

**Table 2-2 Clarity and Color over Time at Different pH Values**

| 30 mg/mL API, 8 mg/mL NaCl, 10 mM Na₂HPO₄ | | | |
|---|---|---|---|
| Storage Conditions | Composition A1 | Composition A2 | Composition A3 |
| | pH5.8 | pH6.5 | pH8.0 |
| 0 day | turbid colorless | clear colorless | clear colorless |
| 2-8°C, 10 days | / | clear colorless | clear colorless |
| 40°C, 10 days | / | clear Yellow 1 | clear Yellow 1 |
| 40°C, 20 days | / | almost clear Orange-yellow 1-2 | almost clear Orange-yellow 1-2 |
| 60°C, 10 days | / | clear Yellow 1-2 | clear Yellow 2 |
| 60°C, 20 days | / | Turbidity 2 Orange-yellow 2-3 | clear Orange-yellow 3-4 |

In Table 2-2 above and the Tables hereinafter, the number following the color indicated the hue value of the standard colorimetric solution corresponding to the color of the composition. The higher the value was, the deeper the color was. The number following the turbidity denoted the turbidity value of the turbidity standard solution corresponding to the turbidity of the composition. The higher the value was, the more turbid the solution was. The results in Table 2-2 above showed that, Composition A1 at pH 5.8 was a suspension with P016 precipitated, indicating that the compound of the present invention becomes turbid at a pH as low as 5.8, and a clear solution cannot be obtained; both Compositions A2 with pH 6.5 and Compositions A3 with pH 8.0 were clear solutions; Compositions A2 and A3 remained clear when being placed at low temperatures (2-8°C) and high temperatures (40°C and 60°C) for 10 days; Compositions A2 and A3 were almost clear when being placed at 40°C for 20 days; the turbidity of Composition A2 increased to Turbidity 2 when being placed at 60°C for 20 days, while Composition A3 remained clear when being placed at 60°C for 20 days. It was shown that the clarity of the polypeptide compound of the present invention was affected by high temperature, and somewhat related to pH. Turbidity was more likely to increase at pH 6.5 than at pH 8.0.

When each stored at 2-8°C, 40°C, and 60°C for 10 or 20 days, Composition A2 and A3 showed a deeper color as the temperature increased. The color of the solution of the polypeptide compounds of the present invention exhibited a temperature-dependent trend.

**Table 2-3 Purity over Time at Different pH Values**

| 30 mg/mL API, 8 mg/mL NaCl, 10 mM Na₂HPO₄ | | |
|---|---|---|
| Storage Conditions | Composition A2 Purity (%) | Composition A3 Purity (%) |
| | pH6.5 | pH8.0 |
| 2-8°C 10 days | 95.51 | 95.07 |
| 40°C 10 days | 94.04 | 92.10 |
| 40°C 20 days | 91.08 | 90.22 |
| 60°C 10 days | 80.84 | 69.77 |
| 60°C 20 days | 66.93 | 55.65 |

The results in Table 2-3 above showed that, the purity decrease of Composition A2 and Composition A3 at 60°C was significantly higher than that of these compositions at 40°C, indicating that the purity stability of the polypeptide compound solution of the present invention exhibited a temperature-dependent trend. After being placed at 60°C for 10 days and 20 days, Composition A2 with pH 8.0 exhibited a more significant increase in purity than Composition A2 with pH 6.5, indicating that the purity stability of the polypeptide compound solution of the present inventionin exhibited a pH-dependent trend at a high temperature. In summary, the purity stability of the polypeptide compound solution of the present invention had a significant pH-related and temperature-dependent trend.

### [Composition Example 2] Buffer Type Screening - Citrate and Phosphate

According to Table 2-4 below, 470 mg of mannitol, 29.4 mg of trisodium citrate dihydrate or 14.2 mg of Na₂HPO₄ were weighed into a beaker respectively, into which 8 mL of purified water was added with stirring for dissolution. 300 mg of Polypeptide Compound P016 (API) was added to the beaker with stirring until dissolved. Then a 0.1 to 0.5 M HCl aqueous solution was added to adjust the pH value to the target pH values as shown in Table 2-4 below. Purified water was added to adjust the volume to 10 mL, and the mixture was mixed homogeneously and filtered through a 0.22 µm PES membrane, thereby obtaining Composition B1 to Composition B3, and Composition B4 to Composition B6 having the following composition: 30 mg/mL of API, 47 mg/mL of mannitol, and 10 mM of Na₂HPO₄ or 10 mM trisodium citrate.

A composition sample to be tested was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

After resting for a period of time, the resulting compositions sample were measured for clarity, color and purity over different time at different temperatures according to the measurement methods as described above, and the results were recorded in Tables 2-5 and 2-6 below.

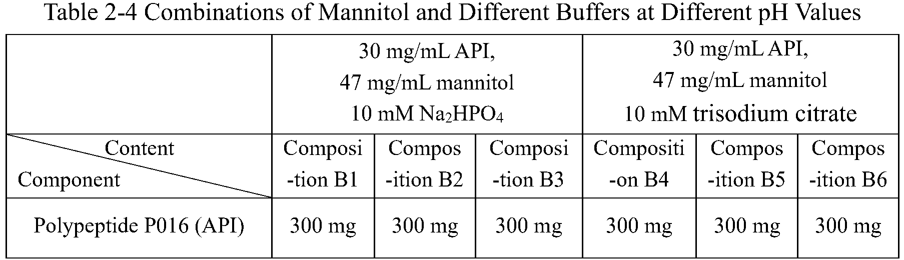

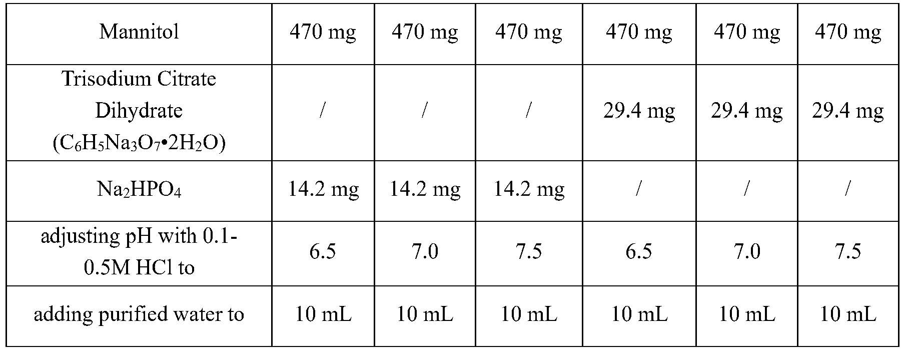

**Table 2-5 Effects of Different Buffers on Clarity and Color**

| | 30 mg/mL API, 47 mg/mL mannitol 10 mM Na₂HPO₄ | | | 30 mg/mL API, 47 mg/mL mannitol 10 mM trisodium citrate | | |
|---|---|---|---|---|---|---|
| Storage Conditions | Composition B1 | Composition B2 | Composition B3 | Composition B4 | Composition B5 | Composition B6 |
| | pH 6.5 | pH 7.0 | pH 7.5 | pH 6.5 | pH 7.0 | pH 7.5 |
| 2-8°C 5 days | clear colorless | clear colorless | clear colorless | clear colorless | clear colorless | clear colorless |
| 40°C 5 days | clear colorless | clear colorless | clear colorless | clear colorless | clear colorless | clear colorless |
| 40°C 20 days | clear Orange-yellow 2 | clear Orange-yellow 2 | clear Orange-yellow 2 | clear Orange-yellow 1 | N/A | clear Orange-yellow 1 |
| 60°C 5 days | clear Yellow 0.5-1 | clear Yellow 2-3 | clear Yellow 1-2 | clear Yellow 0.5-1 | clear Yellow 0.5-1 | clear Yellow 0.5-1 |
| 60°C 20 days | clear Orange-yellow 7 | clear Orange-yellow 7 | clear Orange-yellow 10 | clear Orange-yellow 2 | clear Orange-yellow 2 | clear Orange-yellow 2 |

The results in Tables 2-5 above showed that, after Compositions B1, B2, and B3 containing disodium hydrogen phosphate + mannitol and Compositions B4, B5, and B6 containing trisodium citrate + mannitol were prepared and placed at high temperatures of 40°C and 60°C for 5 days and 20 days respectively, Compositions B1 to B6 all maintained clear. In view of the obeservations in terms of the color of the samples at high temperagure, the citrate buffer represented by trisodium citrate can more effeciently inhibit the formation of colorful degradation purities in the solution of the compound of the present invention as compared with the phosphate buffer represented by Na₂HPO₄.

In addition, in combination with the results for Composition A2 in Table 2-2 and Composition B1 in Table 2-5, it was indicated that, at pH 6.5, as compared with sodium chloride as an osmotic regulator, mannitol as an osmotic regulator had the effect of improving the clarity stability of the composition.

**Table 2-6 Effects of Different Buffer on Purity (%)**

| | 30 mg/mL API, 47 mg/mL mannitol 10 mM Na₂HPO₄ | | | 30 mg/mL API, 47 mg/mL mannitol 10 mM trisodium citrate | | |
|---|---|---|---|---|---|---|
| Storage Conditions | Composition B1 (%) | Composition B2 (%) | Composition B3 (%) | Composition B4 (%) | Composition B5 (%) | Composition B6 (%) |
| | pH 6.5 | pH 7.0 | pH 7.5 | pH 6.5 | pH 7.0 | pH 7.5 |
| 2-8°C 5 days | 95.25 | 95.25 | 95.13 | 95.34 | 95.27 | 95.23 |
| 40°C 5 days | 94.91 | 94.47 | 94.81 | 94.70 | 94.76 | 94.92 |
| 40°C 20 days | 91.76 | 90.67 | 89.10 | 93.63 | 93.78 | 93.94 |
| 60°C 5 days | 85.93 | 81.23 | 85.23 | 85.41 | 84.31 | 86.67 |
| 60°C 20 days | 61.18 | 62.66 | 47.62 | 69.91 | 65.04 | 59.68 |

The results in Table 2-6 above showed that, after Compositions B1 to B6 were placed at 60°C for 5 days and 20 days respectively, the purites substantially decreased. After Compositions B1 to B6 were placed at 40°C for 5 days and 20 days respectively, these compositions all exhibited realatively excellent purity at pH 6.5, 7.0, and 7.5. The purity stability did not obviously depend on pH within the pH range of 6.5 to 7.5. It was indicated that the purity stability of the solution of Polypeptide Compound P016 of the present invention was highly temperature-dependent.

The results in Table 2-6 above showed that, as compared with Compositions B1 to B3 containing phosphate as a buffer, Compositions B4 to B6 containing citrate as a buffer showed a smaller trend of purity change at high temperature, and exhibit better stability.

In summary, the results in Tables 2-5 and 2-6 showed that, in the compositions containing Polypeptide Compound P016 of the present invention and mannitol, as compared with compositions containing phosphate as a buffer, the compositions containing citrate as a buffer exhibited less decrease in purity and color darkening when being stored at high temperature; when Composition B1 to B6 at pH 6.5 to pH 7.5 were stored at 40°C for 20 days, these compositions still had good purity and color. Compositions B4 to B6 containing citrate as a buffer exhibited better purity stability and color stability than Compositions B1 to B3 containing phosphate buffer at the same pH.

### [Composition Example 3] Screening of Osmotic Pressure Regulators - Propylene Glycol, Mannitol, and Sodium Chloride

According to Table 2-7 below, mannitol or NaCl or propylene glycol, and trisodium citrate dihydrate and/or Na₂HPO₄ were weighed into a beaker respectively, into which 28 mL of purified water was added with stirring for dissolution. 1050 mg of Polypeptide Compound P016 was slowly added to the beaker with stirring until dissolved. The pH value of the resulting mixture was measured. Then 0.5 M HCl aqueous solution or 0.5 M NaOH aqueous solution was added to adjust the pH value to 6.5. Purified water was added to adjust the volume to 35 mL, and the mixture was mixed homogeneously and filtered through a 0.22 µm PES membrane, thereby obtaining Composition C1 to Composition C7 containing 30 mg/mL of Peptide Compound P016 (API) of the present invention.

A composition sample to be tested was obtained by aliquoting Compositions C1 to C7 into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

After resting for a period of time, the resulting compositions sample were measured for clarity, color and purity over different time at different temperatures according to the measurement methods as described above, and the results were recorded in Tables 2-8 and 2-9 below.

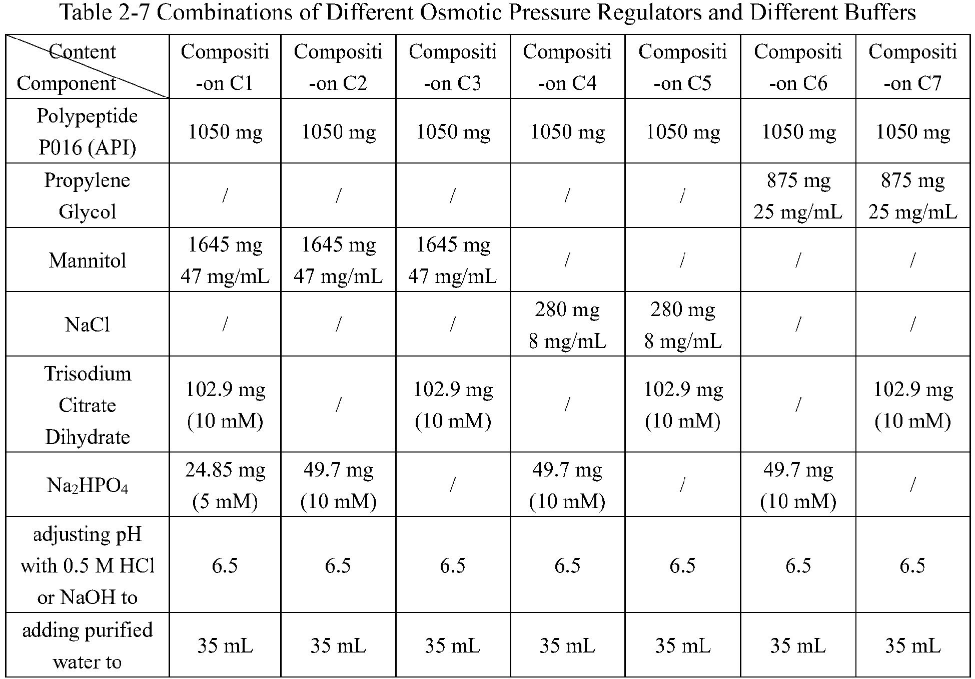

**Table 2-8 Effects of Different Osmotic Pressure Regulators and Different Buffers on Clarity and Color**

| Storage Conditions | Composition C1 | Composition C2 | Composition C3 | Composition C4 | Composition C5 | Composition C6 | Composition C7 |
|---|---|---|---|---|---|---|---|
| | mannitol, trisodium citrate, Na₂HPO₄ | mannitol, Na₂HPO₄ | mannitol, trisodium citrate | NaCl, Na₂HPO₄ | NaCl, trisodium citrate | propylene glycol, Na₂HPO₄ | propylene glycol, trisodium citrate |
| 0 day | clear colorless | clear colorless | clear colorless | clear colorless | clear colorless | clear colorless | clear colorless |
| 40°C 5 days | clear colorless | clear colorless | clear colorless | almost clear colorless | Turbidity 1 colorless | almost clear colorless | Turbidity 1-2 colorless |
| 40°C 10 days | almost clear colorless | almost clear colorless | clear colorless | Turbidity 1 colorless | Turbidity 1-2 colorless | almost clear colorless | Turbidity 1-2 colorless |
| 60°C 5 days | almost clear colorless | clear colorless | clear colorless | Turbidity 1 colorless | Turbidity 1-2 colorless | almost clear colorless | Turbidity 1-2 colorless |
| 60°C 10 days | Turbidity 1 colorless | almost clear colorless | almost clear colorless | Turbidity 1 colorless | Turbidity 2-3 colorless | Turbidity 1 colorless | Turbidity 1-2 colorless |
| 25°C 30 days | Turbidity 2 colorless | almost clear colorless | clear colorless | Turbidity 1-2 colorless | almost clear colorless | Turbidity > 4 colorless | almost clear colorless |

The results in Table 2-8 above showed that, when solution clarity and color were used as stability indexes, as for the compositions at pH 6.5 containing 30 mg/mL the polypeptide compound of the present invention, in the Compositions C2 to C7 which used trisodium citrate or Na₂HPO₄ alone as a buffer, Compositions C2 and C3 using mannitol as an osmotic pressure regulator showed a slower increase in turbidity at room temperature (25°C) and high temperatures (40°C, 60°C) as compared with those using sodium chloride or propylene glycol as an osmotic pressure regulator, indicating that mannitol was more suitable as an osmotic pressure regulator for the solution of the polypeptide compound of the present invention than sodium chloride or propylene glycol.

In summary, when trisodium citrate or Na₂HPO₄ was used as a buffer, mannitol was more suitable as an osmotic pressure regulator for maintaining the clarity of the solution of the pharmaceutical composition according to the present invention.

**Table 2-9: Effects of Different Osmotic Pressure Regulators and Buffers on Purity**

| Storage Conditions | Composition C1 | Composition C2 | Composition C3 | Composition C4 | Composition C5 | Composition C6 | Composition C7 |
|---|---|---|---|---|---|---|---|
| | Mannitol, trisodium | Mannitol, Na₂HPO₄ | Mannitol, trisodium | NaCl, Na₂HPO₄ | NaCl, trisodium | propylene glycol, | propylene glycol, |
| | citrate, Na₂HPO₄ | | citrate | | citrate | Na₂HPO₄ | trisodium citrate |
| 2-8°C 5 days | 99.50 | 99.47 | 99.52 | 99.44 | 99.50 | 98.41 | 98.41 |
| 40°C 5 days | 98.86 | 98.81 | 98.95 | 98.76 | 98.50 | 97.94 | 97.87 |
| 40°C 10 days | 98.28 | 98.05 | 98.32 | 98.18 | 97.89 | 97.13 | 97.16 |
| 60°C 5 days | 94.34 | 93.56 | 95.13 | 94.80 | 93.99 | 94.24 | 93.49 |
| 60°C 10 days | 91.38 | 86.89 | 92.06 | 90.23 | 89.43 | 87.37 | 86.95 |
| 25°C 30 days | 98.85 | 98.97 | 99.04 | 99.08 | 98.89 | N/A | 98.00 |

| | | | | | | | |
|---|---|---|---|---|---|---|---|
| Note: The purity of the active pharmaceutical ingredient P016 in Compositions C1-C5 was 99.48%, and the purity of the active pharmaceutical ingredient P016 in Compositions C6-C7 was 98.46%. The purity of P016 in Compositions C1-C7 after being placed at 2-8°C for 5 days were comparable to the purity of the active pharmaceutical ingredient used in the compositions. | | | | | | | |

The results in Table 2-9 showed that, when purity was used as a stability index, after being stored at 25°C for 30 days, Compositions C1 to C7 showed small differences in the change of purity. Among Compositions C3, C5 and C7 containing trisodium citrate as a buffer, Composition C3 using mannitol as an osmotic pressure regulator showed a comparable or smaller decrease in purity at high temperatures (40°C, 60°C) as compared with Compositions C5 and C7 using sodium chloride or propylene glycol as an osmotic pressure regulator, and demonstrated better overall stability, indicating that mannitol was more suitable as the osmotic pressure regulator for the solution of the present invention than sodium chloride or propylene glycol.

It can be seen that, the combination of citrate buffer represented by trisodium citrate and mannitol was more suitable as the medium environment for the solution of the present invention and thus maintained the stability of purity indexes at different temperatures, as compared with the combination of Na₂HPO₄ buffer and sodium chloride or propylene glycol.

In combination with the results in above Tables 2-8 and 2-9, it can be seen that in compositions at pH 6.5 containing 30 mg/mL API, the composition using mannitol as an osmotic pressure regulator and trisodium citrate as a buffer exhibited the best stability in clarity, color, and purity of the solution when being stored at room temperature (25°C) and high temperatures (40°C, 60°C).

### [Composition Example 4] Screening of Stabilizer- Tween 20

According to Table 2-10 below, 1645 mg of mannitol, 102.9 mg of trisodium citrate dihydrate and 14 mg of Tween 20 were weighed into a beaker respectively, into which 28 mL of purified water was added and stirred homogeneously. 1050 mg of Polypeptide Compound P016 was slowly added to the beaker with stirring until dissolved. Then 0.1 M HCl aqueous solution or 0.5 M NaOH aqueous solution was added to adjust the pH value to the target pH. Purified water was added to adjust the volume to 35 mL, and the mixture was mixed homogeneously and filtered through a 0.22 µm PES membrane, thereby obtaining Composition D1 to Composition D5 containing 30 mg/mL of the API.

A composition sample to be tested was obtained by aliquoting Compositions D1 to D5 into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

After resting for a period of time, the resulting compositions sample were measured for clarity, color and purity over different time at different temperatures according to the measurement methods as described above, and the results were recorded in Tables 2-10 and 2-11 below.

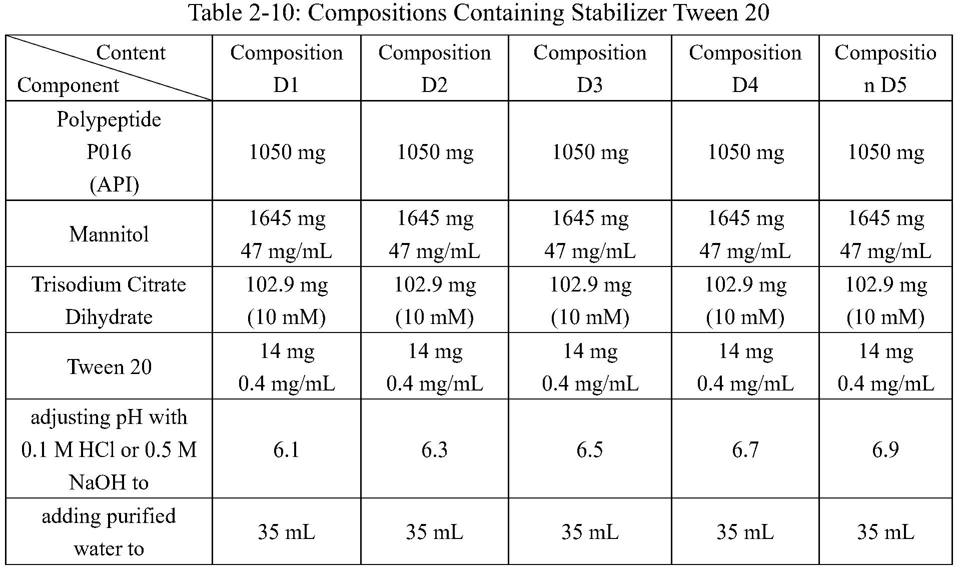

**Table 2-11: Clarity and Color of Compositions Containing Stabilizer Tween 20 at Different pH Values**

| Storage Conditions | Composition D1 | Composition D2 | Composition D3 | Composition D4 | Composition D5 |
|---|---|---|---|---|---|
| | pH=6.1 | pH=6.3 | pH=6.5 | pH=6.7 | pH=6.9 |
| 0 day | clear colorless | clear colorless | clear colorless | clear colorless | clear colorless |
| 40°C 5 days | Tubidity 4 colorless | Tubidity 4 colorless | Tubidity 4 colorless | Tubidity 4 colorless | Tubidity 4 colorless |
| 40°C 10 days | Tubidity >4 colorless | Tubidity >4 colorless | Tubidity >4 colorless | Tubidity >4 colorless | Tubidity >4 colorless |
| 60°C 5 days | Turbidity 1 colorless | Tubidity 3 colorless | Tubidity 3 colorless | Turbidity 1 colorless | Turbidity 2-3 colorless |
| 60°C 10 days | Tubidity 3 colorless | Tubidity 4 colorless | Tubidity 4 colorless | Turbidity 2 colorless | Turbidity 2 colorless |

The results in Table 2-11 showed that, although the compositions containing the polypeptide compound of the present invention, mannitol, and trisodium citrate can be formulated into clear and colorless solutions containing 30 mg/mL API at a pH within the range of 6.1-6.9, the incorporation of Tween 20 in Composition D3 in Table 2-11 actually exacerbated the increase in turbidity as compared with Composition C3 in Table 2-8 above, indicating that Tween 20 accelerates the aggregation of the polypeptide compounds of the present invention in the composition.

Tween 20 is not suitable as the stabilizer for the polypeptide compounds of the present invention.

### [Composition Example 5] Screening of Citric Acid-Trisodium Citrate Buffer Concentration, P016 (API) Concentration, and pH Value -- Citric Acid:Trisodium Citrate = 1:9 (Molar Ratio)

Citric acid-trisodium citrate buffer were prepared as follows:
40 mM aqueous citric acid solution: 8.4056 g of citric acid monohydrate (C₆H₈O₇•H₂O) was diluted and dissolved with water to 1000 mL to obtain the 40 mM aqueous citric acid solution.
40 mM aqueous trisodium citrate solution: 11.764 g of trisodium citrate dihydrate (C₆H₅Na₃O₇•2H₂O) was diluted and dissolved with water to 1000 mL to obtain the 40 mM aqueous trisodium citrate solution.
40 mM citric acid-trisodium citrate buffer: 10 mL of 40 mM aqueous citric acid solution was mixed with 90 mL of 40 mM aqueous trisodium citrate solution to obtain the 40 mM citric acid-trisodium citrate buffer, wherein the total molar concentration of citric acid and trisodium citrate was 40 mM, the molar ratio of citric acid to trisodium citrate was 1 to 9; the molar concentration of citric acid was 4 mM, and the molar concentration of trisodium citrate was 36 mM.
20 mM citric acid-trisodium citrate buffer: 50 mL of 40 mM citric acid-trisodium citrate buffer was mixed with 50 mL of purified water to obtain the 20 mM citric acid-trisodium citrate buffer, wherein the total molar concentration of citric acid and trisodium citrate was 20 mM, the molar ratio of citric acid to trisodium citrate was 1 to 9, the molar concentration of citric acid was 2 mM, and the molar concentration of trisodium citrate was 18 mM.
10 mM citric acid-trisodium citrate buffer: 50 mL of 20 mM citric acid-trisodium citrate buffer was mixed with 50 mL of purified water to obtain the 10 mM citric acid-trisodium citrate buffer, wherein the total molar concentration of citric acid and trisodium citrate was 10 mM, the molar ratio of citric acid to trisodium citrate was 1 to 9, the molar concentration of citric acid was 1 mM, and the molar concentration of trisodium citrate was 9 mM.

Unless otherwise specified, in Composition Example 5, the final concentration of citric acid-trisodium citrate in Compositions E1 to E14 refers to the total molar concentration of citric acid and trisodium citrate, wherein the molar concentration of citric acid was 1/10 of the total molar concentration, the molar concentration of trisodium citrate was 9/10 of the total concentration, and the molar ratio of citric acid to trisodium citrate was 1:9.

According to Table 2-12 below, solid adjuvants were weighed into a container, and an appropriate amount of puried water were added. The mixture was stirred or shaked until dissolved homogeneously. Optionally, an appropriate amount of citric acid-trisodium citrate buffer solution with suitable concentration was added to the container, and shaked homogenously. An appropriate amount of Polypeptide Compound P016 (API) was slowly added and shaked homogenously until dissolved. Then 0.1 to 0.5 M HCl or NaOH aqueous solution was added to adjust the pH value to the target pH in Table 2-12. Purified water was added to adjust the volume to 3 mL, and the mixture was mixed homogeneously and filtered through a 0.22 µm PES membrane, thereby obtaining Compositions E1 to E14 containing Polypeptide Compound P016 (API).

A composition sample to be tested was obtained by aliquoting Compositions E1 to E14 into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

After resting for a period of time, the resulting compositions were measured for clarity, color, purity and polymer content over different time at different temperatures according to the measurement methods as described above, and the results are recorded in Tables 2-13 to 2-15 below.

In Compositions E1 to E14, mannitol was used as an osmotic pressure regulator; citric acid-trisodium citrate, disodium hydrogen phosphate, or tromethamine (Tris) was used as a buffer, and glycine was used as a potential stabilizer.

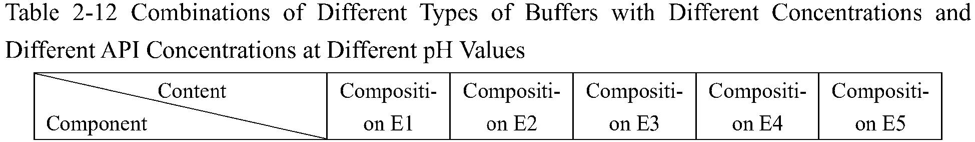

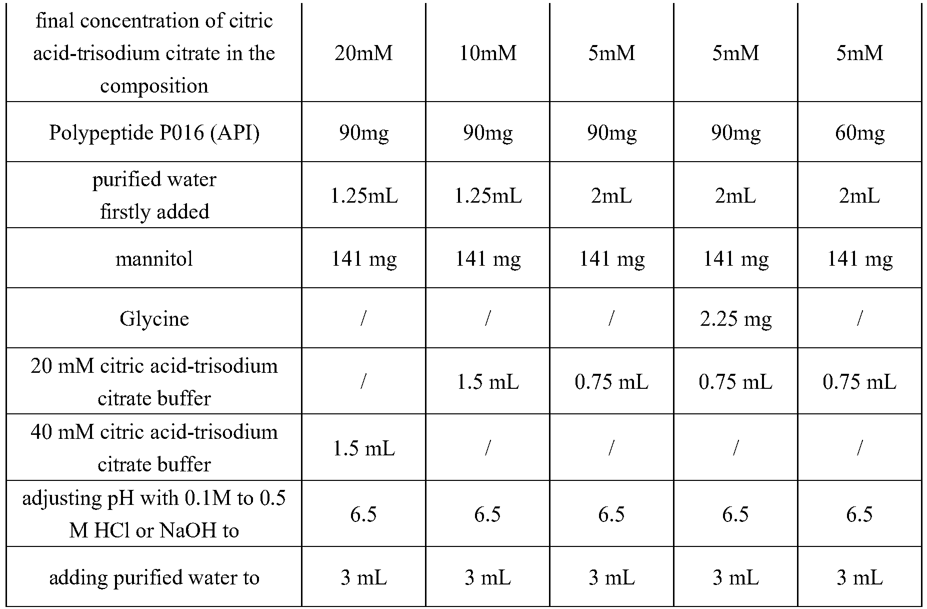

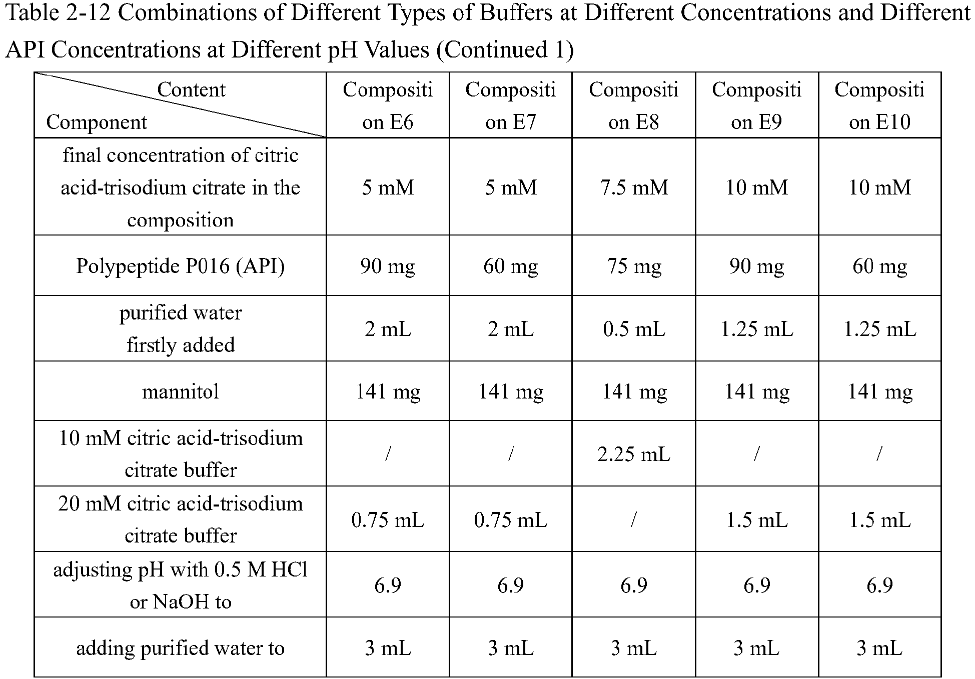

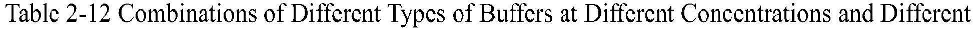

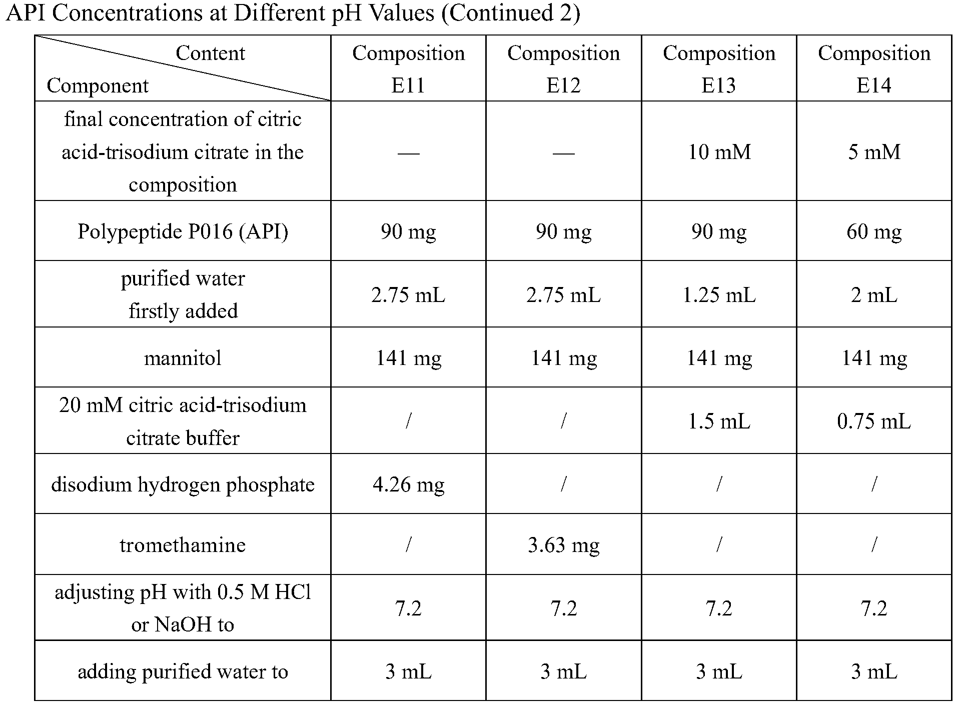

**Table 2-13 Effects of Types and Concentrations of Buffer, API concentration, and pH value on Clarity and Color**

| Composition No. | Concentrations of Buffer, API concentration, and pH | clarity/color | | | | |
|---|---|---|---|---|---|---|
| | | 0 days | 60°C 5 days | 60°C 10 days | 40°C 5 days | 40°C 10 days |
| E1 | 20 mM citric acid-trisodium citrate buffer 30 mg/mL API, pH6.5 | clear colorless | clear colorless | clear colorless | clear colorless | clear colorless |
| E2 | 10 mM citric acid-trisodium citrate buffer 30 mg/mL API, pH6.5 | clear colorless | clear colorless | clear colorless | Clear colorless | clearcolorl ess |
| E3 | 5 mM citric acid-trisodium citrate buffer 30 mg/mL API, pH6.5 | clear colorless | clear colorless | clear colorless | clear colorless | clear colorless |
| E4 | 5 mM citric acid-trisodium citrate buffer 30 mg/mL API, pH6.5 glycine | clear colorless | clear colorless | clear colorless | clear colorless | clear colorless |
| E5 | 5 mM citric acid-trisodium citrate buffer 20 mg/mL API, pH6.5 | clear colorless | clear colorless | clear colorless | clear colorless | clear colorless |
| E6 | 5 mM citric acid-trisodium citrate buffer 30 mg/mL API, pH6.9 | clear colorless | clear colorless | clear colorless | clear colorless | clear colorless |
| E7 | 5 mM citric acid-trisodium citrate buffer 20 mg/mL API, pH6.9 | clear colorless | clear colorless | clear colorless | clear colorless | clear colorless |
| E8 | 7.5 mM citric acid-trisodium citrate buffer 25 mg/mL API, pH6.9 | clear colorless | clear colorless | clear colorless | clear colorless | clear colorless |
| E9 | 10 mM citric acid-trisodium citrate buffer 30 mg/mL API, pH6.9 | clear colorless | clear colorless | clear colorless | clear colorless | clear colorless |
| E10 | 10 mM citric acid-trisodium citrate buffer 20 mg/mL API, pH6.9 | clear colorless | Clear colorless | Clear colorless | Clear colorless | Clear colorless |
| E11 | 10 mM disodium hydrogen phosphate buffer 30 mg/mL API, pH7.2 | clear colorless | clear Yellow 1 | almost clear Yellow 2 | clear colorless | clear colorless |
| E12 | 10 mM tromethamine buffer 30 mg/mL API, pH7.2 | clear colorless | clear colorless | clear colorless | clear colorless | clear colorless |
| E13 | 10 mM citric acid-trisodium citrate buffer 30 mg/mL API, pH7.2 | clear colorless | clear colorless | clear colorless | clear colorless | clear colorless |
| E14 | 5 mM citric acid-trisodium citrate buffer 20 mg/mL API, pH7.2 | clear colorless | clear colorless | clear colorless | clear colorless | clear colorless |

The results in Tables 2-13 above showed that, Compositions E1-E10 and E13-E14 with a pH of 6.5-7.2 using citric acid-trisodium citrate (molar ratio: 1:9) as a buffer and mannitol as an osmotic pressure regulator and containing 20-30 mg/mL of the polypeptide compound of the present invention, remained clear and colorless when stored at high temperatures (40°C, 60°C) for 5 days and 10 days.

When being stored at 60°C for 5 and 10 days, Composition E11 using disodium hydrogen phosphate as a buffer showed a gradually increased turbidity, and the color became darker, again indicating that citric acid-trisodium citrate was more suitable as a buffer for the polypeptide compound of the present invention than disodium hydrogen phosphate.

In Compositions E3 and E4, the presence or absence of glycine as a stabilizer has no significant effect on clarity and color stability.

**Table 2-14 Effect of Types and Concentrations of Buffer, API Concentration, and pH Valueon Purity**

| Composition No. | Concentrations of Buffer, API Concentration, and pH | API Purity (%) | |
|---|---|---|---|
| | | 60°C 10 days | 40°C 10 days |
| E1 | 20 mM citric acid-trisodium citrate buffer 30 mg/mL API, pH6.5 | 88.60 | 98.06 |
| E2 | 10 mM citric acid-trisodium citrate buffer 30 mg/mL API, pH6.5 | 88.95 | 97.97 |
| E3 | 5 mM citric acid-trisodium citrate buffer 30 mg/mL API, pH6.5 | 88.87 | 98.08 |
| E4 | 5 mM citric acid-trisodium citrate buffer 30 mg/mL API, pH6.5 glycine | 89.07 | 97.94 |
| E5 | 5 mM citric acid-trisodium citrate buffer 20 mg/mL API, pH6.5 | 89.52 | 98.01 |
| E6 | 5 mM citric acid-trisodium citrate buffer 30 mg/mL API, pH6.9 | 89.44 | 98.15 |
| E7 | 5 mM citric acid-trisodium citrate buffer 20 mg/mL API, pH6.9 | 89.58 | 98.08 |
| E8 | 7.5 mM citric acid-trisodium citrate buffer 25 mg/mL API, pH6.9 | 89.31 | 98.11 |
| E9 | 10 mM citric acid-trisodium citrate buffer 30 mg/mL API, pH6.9 | 89.08 | 98.11 |
| E10 | 10 mM citric acid-trisodium citrate buffer 20 mg/mL API, pH6.9 | 89.33 | 98.16 |
| E11 | 10 mM disodium hydrogen phosphate buffer 30 mg/mL API, pH7.2 | 74.54 | 97.88 |
| E12 | 10 mM tromethamine buffer 30 mg/mL API, pH7.2 | 87.12 | 97.90 |
| E13 | 10 mM citric acid-trisodium citrate buffer 30 mg/mL API, pH7.2 | 88.96 | 98.25 |
| E14 | 5 mM citric acid-trisodium citrate buffer 20 mg/mL API, pH7.2 | 89.76 | 98.17 |

The results in Tables 2-14 above showed that, after being placed at high temperatures (40°C, 60°C) for 10 days, Compositions E11, E12 and E13 containing 30 mg/mL of the polypeptide compound of the present invention at pH 7.2 showed gradually decrease of the improvement in purity stability imparted by the buffer in the order of citric acid-trisodium citrate, tromethamine, and disodium hydrogen phosphate. When citric acid-trisodium citrate was used as a buffer, Compositions E1-E10 and E13-E14 exhibited optimal purity stability at high temperatures (40°C, 60°C). Moreover, when the total molar concentration of citric acid and trisodium citrate was in the range of 5-20 mM and the polypeptide compound of the present invention was in the range of 20-30 mg/mL, Compositions E1-E10 and Compositions E13-E14 exhibited comparable purity stability at high temperatures (40°C, 60°C). In Compositions E3 and E4, the presence or absence of glycine as a stabilizer has no significant effect on the purity stability of the compositions at high temperatures (40°C, 60°C).

**Table 2-15 Effect of Types and Concentrations of Buffer, API Concentration, and pH Value on Polymer Content**

| Composition No. | Concentrations of Buffer, API Concentration, and pH | Polymer Content (%) | |
|---|---|---|---|
| | | 60°C 10 days | 40°C 10 days |
| E1 | 20 mM citric acid-trisodium citrate buffer 30 mg/mL API pH6.5 | 0.86 | 0.12 |
| E2 | 10 mM citric acid-trisodium citrate buffer 30 mg/mL API pH6.5 | 0.84 | 0.13 |
| E3 | 5 mM citric acid-trisodium citrate buffer 30 mg/mL API pH6.5 | 0.83 | 0.11 |
| E4 | 5 mM citric acid-trisodium citrate buffer 30 mg/mL API pH6.5 glycine | 0.99 | 0.13 |
| E5 | 5 mM citric acid-trisodium citrate buffer 20 mg/mL API pH6.5 | 0.78 | 0.11 |
| E6 | 5 mM citric acid-trisodium citrate buffer 30 mg/mL API pH6.9 | 0.81 | 0.11 |
| E7 | 5 mM citric acid-trisodium citrate buffer 20 mg/mL API pH6.9 | 0.76 | 0.10 |
| E8 | 7.5 mM citric acid-trisodium citrate buffer 25 mg/mL API pH6.9 | 0.80 | 0.12 |
| E9 | 10 mM citric acid-trisodium citrate buffer 30 mg/mL API pH6.9 | 0.78 | 0.10 |
| E10 | 10 mM citric acid-trisodium citrate buffer 20 mg/mL API pH6.9 | 0.80 | 0.11 |
| E11 | 10 mMdisodium hydrogen phosphate buffer 30 mg/mL API pH7.2 | N/A | 0.28 |
| E12 | 10 mM tromethamine buffer 30 mg/mL API pH7.2 | 1.60 | 0.22 |
| E13 | 10 mM citric acid-trisodium citrate buffer 30 mg/mL API pH7.2 | 0.79 | 0.10 |
| E14 | 5 mM citric acid-trisodium citrate buffer 20 mg/mL API pH7.2 | 0.87 | 0.12 |

The results in Tables 2-15 above showed that, after being placed at high temperatures (40°C, 60°C) for 10 days, in the compositions E11, E12 and E13 at pH 7.2 and containing 30 mg/mL of the polypeptide compound of the present invention, the polymer content increased in the order of citric acid-trisodium citrate, tromethamine, and disodium hydrogen phosphate. When citric acid-trisodium citrate was used as a buffer, the polymer content stability of Compositions E1-E10 and E13-E14 at high temperature (40°C, 60°C) was the best. Moreover, when the total molar concentration of citric acid-trisodium citrate was in the range of 5-20 mM and the polypeptide compound of the present invention is in the range of 20-30 mg/mL, after being placed at 40°C for 10 days, the polymer content in the composition increased slightly and was comparable, and the polymer content was 0.10-0.13%.

In conjunction with Table 2-13, Table 2-14 and Table 2-15, in Compositions E3 and E4, the presence or absence of glycine as a stabilizer had no significant effect on clarity, color, purity, and polymer content of the solution of the composition. Incorporating glycine even resulted in a slight increase in the polymer content, indicating that glycine was not suitable as the stabilizer for the polypeptide compound of the present invention.

Both Composition E2 in Table 2-13 and Table 2-14 and Composition C3 in Table 2-8 and Table 2-9 were compositions at pH 6.5 using 47 mg /mL of mannitol, 10 mM buffer and 30 mg/mL API. Composition E2 using 10 mM citric acid-trisodium citrate as a buffer showed improved clarity stability as compared with Composition C3 using 10 mM trisodium citrate alone as a buffer. The color and purity stability at high temperatures (40°C, 60°C) were comparable.

In summary, compositions E1-E10 and E13-E14 with different total molar concentrations (5-20 mM) of citric acid-trisodium citrate, different API concentrations (20-30 mg/mL), and different pH values (6.5-7.2) had comparable clarity, color, API purity, and polymer content when being stored at 40°C and 60°C for 10 days. The stability of all these indexes was excellent, and citric acid-trisodium citrate buffer was overally superior to phosphate buffer and tromethamine buffer.

### [Composition Example 6] Screening of Citric Acid-Trisodium Citrate Buffer Concentration, P016 (API) Concentration, and pH Value -- Citric Acid:Trisodium Citrate = 1:6.15 (1.05 mM: 6.45 mM, Molar Ratio)

According to Table 2-16 below, appropriate amounts of mannitol, citric acid monohydrate, and disodium citrate dihydrate were weighed into a beaker respectively, into which an appropriate amout of purified water (about 80% of the total puried water). The mixture was stirred until dissolved. an appropriate amout of Polypeptide Compound P016 (API) was slowly added to the beaker with stirring until dissolved. The pH value of the resulting mixture was measured. Then 0.1 M HCl aqueous solution or 0.1 M NaOH aqueous solution was added into the beaker to adjust the pH value to the target pH value. Purified water was added to make up the volume, and the mixture was mixed homogeneously and filtered through a 0.22 µm PES membrane, thereby obtaining Compositions F1 to F12 containing Peptide Compound P016 (API) of the present invention.

A composition sample to be tested was obtained by aliquoting Compositions F1 to F12 into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

After resting for a period of time, the resulting compositions samples were measured for clarity, color, pH value (micro pH meter), purity and polymer content over different time at different temperatures or under strong light (1800 1x, 28 µw/cm²) according to the measurement methods as described above, thereby investigating each index and its stability of the compositions containing 4 mg/mL to 30 mg/mL API in the pH range of 6.5-7.4, and the results were recorded in Tables 2-17 to 2-20 below respectively.

Unless otherwise specified, in Compositions F1 to F12 of Composition Example 6, the total molar concentration of the citric acid-trisodium citrate buffer was 7.5 mM, wherein the molar concentration of citric acid was 1.05 mM; the molar concentration of trisodium citrate was 6.45 mM, and the molar ratio of citric acid : trisodium citrate is =1.05:6.45=1:6.15.

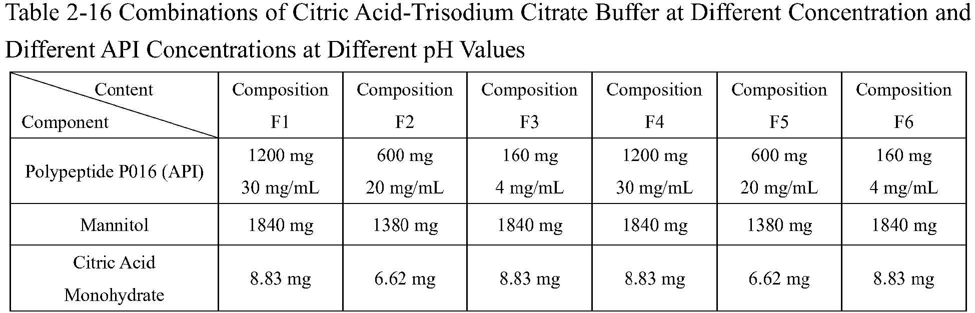

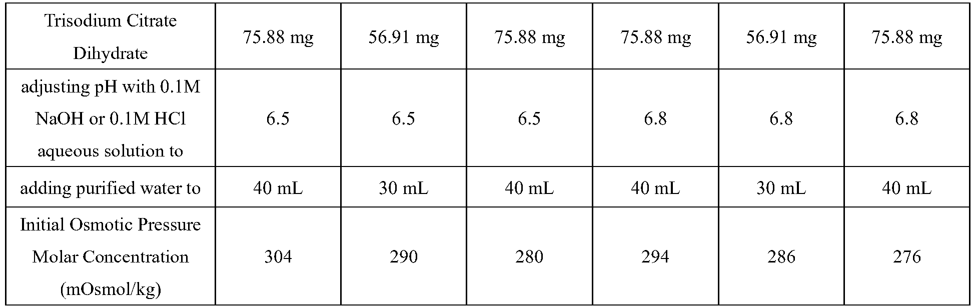

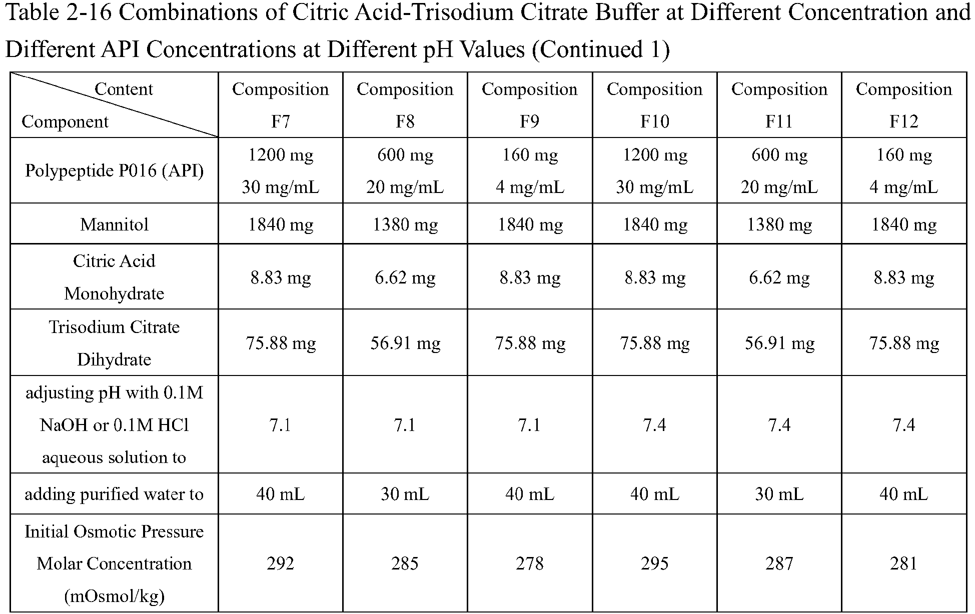

The results in Table 2-16 above showed that, the initial molar osmotic pressures of Compositions F1 to F12 all meet the requirements of near-physiological isotonic values, and were convenient for parenteral administration or injection administration.

**Table 2-17 Effect of Citric Acid-Trisodium Citrate Buffer Concentration, API Concentration, and pH Value on Clarity and Color**

| Composition No. | initial | 60°C 10 days | 40°C 10 days | 40°C 30 days | 25°C 30 days | 2-8°C 30 days | light exposure 10 days |
|---|---|---|---|---|---|---|---|
| Composition F1 | clear colorless | clear colorless | clear colorless | clear colorless | clear colorless | clear colorless | clear colorless |
| Composition F2 | clear colorless | clear colorless | clear colorless | clear colorless | N/A | N/A | clear colorless |
| Composition F3 | clear colorless | clear colorless | clear colorless | clear colorless | clear colorless | clear colorless | clear colorless |
| Composition F4 | clear colorless | clear colorless | clear colorless | clear colorless | clear colorless | clear colorless | clear colorless |
| Composition F5 | clear colorless | clear colorless | clear colorless | clear colorless | N/A | N/A | clear colorless |
| Composition F6 | clear colorless | clear colorless | clear colorless | clear colorless | clear colorless | clear colorless | clear colorless |
| Composition F7 | clear colorless | clear colorless | clear colorless | clear colorless | clear colorless | clear colorless | clear colorless |
| Composition F8 | clear colorless | clear colorless | clear colorless | clear colorless | N/A | N/A | clear colorless |
| Composition F9 | clear colorless | clear colorless | clear colorless | clear colorless | clear colorless | clear colorless | clear colorless |
| Composition F10 | clear colorless | clear colorless | clear colorless | clear colorless | clear colorless | clear colorless | clear colorless |
| Composition F11 | clear colorless | clear colorless | clear colorless | clear colorless | N/A | N/A | clear colorless |
| Composition F12 | clear colorless | clear colorless | clear colorless | clear colorless | clear colorless | clear colorless | clear colorless |

| | | | | | | | |
|---|---|---|---|---|---|---|---|
| N/A: Composition F2 had the same formulation as Compositions F1 and F3, except that the concentrations of P016 were different. Therefore, no data were collected at the detection time point marked by N/A. The same explanation applied to other detection time points marked by N/A. | | | | | | | |

The results of Tables 2-17 above showed that, Compositions F1 to F12 had good solution clarity stability, and remained clear and colorless after being placed at a high temperature of 40°C for 10 days and 30 days, at a high temperature of 60°C for 10 days, and under strong light for 10 days. Compositions F1 and F3, F4 and F6, F7 and F9, F10 and F12 containing 4 mg/mL or 30 mg/mL of P016 remained clear and colorless at a low temperature (2-8°C) for 30 days and at room temperature (25°C) for 30 days.

**Table 2-18 Effect of Citric Acid-Trisodium Citrate Buffer Concentration, API Concentration, and pH Value on pH Stability**

| Composition No. | pH | | | | | | |
|---|---|---|---|---|---|---|---|
| | Initial value | 60°C 10 days | 40°C 10 days | 40°C 30 days | 25°C 30 days | 2-8°C 30 days | light exposure 10 days |
| Composition F1 | 6.53 | 6.52 | 6.52 | 6.57 | 6.57 | 6.56 | 6.54 |
| Composition F2 | 6.54 | 6.51 | 6.51 | 6.57 | N/A | N/A | 6.53 |
| Composition F3 | 6.53 | 6.56 | 6.55 | 6.55 | 6.55 | 6.57 | 6.53 |
| Composition F4 | 6.84 | 6.87 | 6.82 | 6.80 | 6.82 | 6.83 | 6.85 |
| Composition F5 | 6.83 | 6.86 | 6.86 | 6.80 | N/A | N/A | 6.88 |
| Composition F6 | 6.84 | 6.84 | 6.86 | 6.80 | 6.81 | 6.81 | 6.87 |
| Composition F7 | 7.13 | 7.16 | 7.15 | 7.08 | 7.08 | 7.08 | 7.11 |
| Composition F8 | 7.13 | 7.12 | 7.13 | 7.05 | N/A | N/A | 7.12 |
| Composition F9 | 7.13 | 7.15 | 7.15 | 7.10 | 7.09 | 7.06 | 7.13 |
| Composition F10 | 7.43 | 7.43 | 7.42 | 7.31 | 7.30 | 7.31 | 7.42 |
| Composition F11 | 7.41 | 7.42 | 7.43 | 7.29 | N/A | N/A | 7.38 |
| Composition F12 | 7.43 | 7.39 | 7.37 | 7.30 | 7.29 | 7.32 | 7.34 |

The results in Tables 2-18 above showed that, the pH values of compositions F1 to F12 varied by no more than 0.14 when being placed at a low temperature (2-8°C) for 30 days, at room temperature (25°C) for 30 days, at a high temperature (40°C) for 10 days and 30 days, at a high temperature (60°C) for 10 days, or under strong light for 10 days. In particular, in conjunction with the following Table 2-19, the purities of compositions F1 to F12 varied by about 25% after being placed at a high temperature of 60°C or under strong light for 10 days, while the pH of Composition F12 varied by no more than 0.09, the pH of Composition F5 varied by no more than 0.05, and the pH values of the other 10 compositions varied by no more than 0.03, indicating that even after being placed at 60°C or under light for 10 days, a citric acid-trisodium citrate buffer with a total molar concentration of 7.5 mM (the molar concentrations of citric acid and trisodium citrate being 1.05 mM and 6.45 mM, respectively) was sufficient to maintain the pH stability of the composition in the range of pH 6.5-7.4.

**Table 2-19 Effect of Type and Concentration of Citric Acid-Trisodium Citrate Buffer, API Concentration, and pH value on API Purity**

| Composition No. | Purity (%) | | | | | | | |
|---|---|---|---|---|---|---|---|---|
| | Initial Value | 60°C 10 days | 40°C 10 days | 40°C 30 days | 25°C 30 days | 2-8°C 30 days | 2-8°C 4 months | light exposure for 10 days |
| Composition F1 | 98.40 | 73.66 | 96.69 | 92.77 | 97.70 | 98.39 | 98.45 | 96.99 |
| Composition F2 | 98.33 | 72.56 | 96.84 | 92.65 | N/A | N/A | 98.46 | 97.18 |
| Composition F3 | 98.34 | 72.13 | 96.70 | 92.75 | 97.65 | 98.39 | 98.45 | 96.88 |
| Composition F4 | 98.37 | 72.57 | 96.84 | 93.24 | 97.80 | 98.41 | 98.37 | 97.09 |
| Composition F5 | 98.34 | 72.53 | 96.63 | 93.06 | N/A | N/A | 98.45 | 97.43 |
| Composition F6 | 98.34 | 73.15 | 96.74 | 93.21 | 97.80 | 98.39 | 98.41 | 97.27 |
| Composition F7 | 98.36 | 72.87 | 96.79 | 93.51 | 97.88 | 98.42 | 98.39 | 97.12 |
| Composition F8 | 98.41 | 72.94 | 96.77 | 93.67 | N/A | N/A | 98.39 | 97.21 |
| Composition F9 | 98.44 | 72.29 | 96.76 | 93.40 | 97.85 | 98.40 | 98.39 | 97.11 |
| Composition F10 | 98.36 | 73.03 | 96.68 | 93.45 | 98.02 | 98.44 | 98.41 | 97.10 |
| Composition F11 | 98.36 | 72.65 | 96.57 | 93.54 | N/A | N/A | 98.40 | 97.39 |
| Composition F12 | 98.37 | 72.05 | 96.71 | 93.48 | 97.99 | 98.38 | 98.44 | 97.51 |

The results in Tables 2-19 above showed that the purity of Compositions F1 to F12 containing the polypeptide compound of the present invention was about 98.4 %; after being placed at 60°C or under strong light for 10 days, the API purities of the 12 compositions comparatively decreased to 72.05-73.66% and 96.88-97.51%, respectively; after being placed at a high temperature of 40°C for 10 days and 30 days, the API purity of the 12 compositions comparatively decreased to 96.57-96.84% and 92.65-93.67%, respectively; after being placed at room temperature (25°C) for 1 month, the API purities of the compositions containing 4 mg/mL and 30 mg/mL of API decreased to 97.65-98.02% (a decrease of less than 0.7%); after being placed at low temperature (2-8°C) for 30 days and 4 months, no significant change in API purity was observed. In general, the purity stability of the 12 compositions was comparable and highly temperature-dependent. The purity stability of Compositions F1 to F12 was excellent at a low temperature of 2-8°C.

**Table 2-20 Effect of Concentration of Citric Acid-Trisodium Citrate Buffer, API Concentration, and pH value on Polymer Content**

| Composition No. | Polymer Content (%) | | | | | | |
|---|---|---|---|---|---|---|---|
| | 2-8°C 10 days | 60°C 10 days | 40°C 10 days | 40°C 30 days | 25°C 30 days | 2-8°C 30 days | light exposure for 10 days |
| Composition F1 | 0.05 | 0.71 | 0.10 | 0.24 | 0.06 | 0.03 | 0.68 |
| Composition F2 | 0.05 | 0.77 | 0.10 | 0.23 | N/A | N/A | 0.60 |
| Composition F3 | 0.05 | 0.72 | 0.09 | 0.24 | 0.06 | 0.06 | 0.74 |
| Composition F4 | 0.05 | 0.72 | 0.08 | 0.22 | 0.05 | 0.04 | 0.59 |
| Composition F5 | 0.06 | 0.74 | 0.09 | 0.20 | N/A | N/A | 0.47 |
| Composition F6 | 0.05 | 0.66 | 0.10 | 0.18 | 0.05 | 0.06 | 0.56 |
| Composition F7 | 0.05 | 0.72 | 0.08 | 0.18 | 0.05 | 0.03 | 0.62 |
| Composition F8 | 0.04 | 0.73 | 0.08 | 0.18 | N/A | N/A | 0.57 |
| Composition F9 | 0.04 | 0.66 | 0.09 | 0.18 | 0.06 | 0.04 | 0.60 |
| Composition F10 | 0.04 | 0.72 | 0.08 | 0.17 | 0.06 | 0.03 | 0.62 |
| Composition F11 | 0.05 | 0.78 | 0.09 | 0.18 | N/A | N/A | 0.48 |
| Composition F12 | 0.06 | 0.74 | 0.10 | 0.19 | 0.06 | 0.02 | 0.44 |

The results in Table 2-20 above showed that, the initial polymer content of Compositions F1 to F12 was about 0.05%.; after being placed at a high temperature of 60°C for 10 days, the polymer content increased to 0.66-0.78%; after being exposed to strong light for 10 days, it increased to 0.44-0.74%; after being placed at a high temperature of 40°C for 1 month, the polymer content increased to 0.17-0.24%; after being placed at room temperature (25°C ) and low temperature (2-8°C) for 30 days, the polymer content of Compositions F1 to F12 with API concentrations of 4 mg/mL to 30 mg/mL remained at 0.02-0.06%, with almost no change. In general, the polymer content stability of the 12 compositions was comparable and highly temperature-dependent. The polymer content of compositions F1 to F12 was stable at room temperature (25°C) and low temperature (2-8°C).

In summary, the stability of the 12 compositions, namely, Compositions F1 to F12 (7.5 mM citric acid-trisodium citrate buffer, 4, 20, 30 mg/mL API, pH 6.5, 6.8, 7.1, 7.4) was studied at 60°C, 40°C, under strong light, 25°C (room temperature), and 2-8°C (low temperature) for 1 to 4 months. The results showed that the stability of various indexes (clarity, color, pH value, API purity, polymer content) in the 12 compositions, namely, Compositions F1 to F12 at pH 6.5-7.4 with an API concentration of 4-30 mg/mL were comparable, and all 12 compositions had excellent clarity, color and pH stability at low temperature, room temperature, high temperature and under light. The API purity stability and polymer content stability were highly temperature-dependent, and the stability of API purity and polymer content at low temperature (2-8°C) were the best.

In conjuction with Tables 2-18 to 2-20, it can be seen that, in strong light, the clarity and color of Compositions F1 to F12 had no change, while the API purity and polymer content showed light-sensitive characteristics. Therefore, for the composition containing the polypeptide compound of the present invention, light-proof packaging and storage measures should be adopted to avoid the risk of photodegradation.

The compositions at pH 6.5-7.4 containing 46 mg/mL of mannitol as an osmotic pressure regulator, 7.5 mM citric acid-trisodium citrate buffer as a buffer, and 4-30 mg/mL of the polypeptide compound of the present invention had excellent stability of various indexes (clarity, color, pH value, API purity, polymer content) at low temperature, room temperature, and high temperature, and the stability was best at low temperature (2-8°C).

### [Composition Example 7]

### [Preparation of Compositions G1, G1-a and G1-q]

According to Table 2-21-1 or Table 2-21-2 below, 46.00 g of mannitol, 1.90 g of trisodium citrate dihydrate, and 0.22 g of citric acid monohydrate were weighed into a beaker respectively, into which 800 mL of injection water was added and the mixture was stirred homogeneously. 0.5-1 g or 1-30.00 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8 to 7.4. Injection water was added to adjust the volume to 1000 mL, and the mixture was mixed homogeneously, thereby obtaining Composition G1 or Compositions G1-a to G1-q having the following composition: 0.5-1 mg/mL or 1-30 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 1.05 mM: 6.45 mM, that is 1:6.15) as a buffer. Their pH values were in the range of 6.8 to 7.4.

The prepared medicinal liquid can be divided and sealed into 1) neutral borosilicate glass tube injection bottles or 2) pre-filled syringes.
1) A composition sample G1 was obtained by: filtering the composition through a 0.22 µm PES membrane; and aliquoting into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.
2) Composition samples G1-a to G1-q was obtained by filtered with a 0.22µm PES membrane, aliquoting into pre-filled syringes, sealing with a stopper, and marking the volume as 0.5 mL. Similarly, according to Table 2-21-3 to Table 2-21-6 below, composition samples G1-a to G1-q were prepared by the same method as above, except that each raw material was reduced in equal proportions.

**Table 2-21-1 Composition of Composition G1**

| Component | Content | Function |
|---|---|---|
| Polypeptide Compound P016 (API) | 0.5-1g or 1-30.00g (0.5-1 mg/mL or 1-30 mg/mL) | Active Pharmaceutical Ingredient |
| Mannitol | 46.00 g (46 mg/mL) | Osmotic Pressure Regulator |
| Trisodium Citrate Dihydrate | 1.90 g (6.45 mM) | Buffer |
| Citric Acid Monohydrate | 0.22 g (1.05 mM) | Buffer |
| adjusting pH with 0.1M NaOH or 0.1M HCl aqueous solution to | 6.8-7.4 | pH adjusting agent |
| Adding injection water to | 1000 mL | Solvent |

**Table 2-21-2 Components of Compositions G1-a to G1-q (Pre-filled syringes, batch size can be scaled up or down proportionally)**

| Composition | Specification | Polypeptide P016 (API) | Mannitol | Citric Acid Monohydrate | Trisodium Citrate Dihydrate | adjusting pH with 0.1M NaOH or 0.1M HCl aqueous solution to | Adding injection water to |
|---|---|---|---|---|---|---|---|
| G1-a | 0.5 mg/mL | 0.50g | 46.00g | 0.22g | 1.90g | | 1000 mL |
| G1-b | 1 mg/mL | 1.00g | 46.00g | 0.22g | 1.90g | | 1000 mL |
| G1-c | 1.5 mg/mL | 1.50 g | 46.00g | 0.22g | 1.90g | | 1000 mL |
| G1-d | 2 mg/mL | 2.00g | 46.00g | 0.22g | 1.90g | | 1000 mL |
| G1-e | 2.5 mg/mL | 2.50g | 46.00g | 0.22g | 1.90g | 6.8-7.4 | 1000 mL |
| G1-f | 3 mg/mL | 3.00g | 46.00g | 0.22g | 1.90g | | 1000 mL |
| G1-g | 3.5 mg/mL | 3.50g | 46.00g | 0.22g | 1.90g | | 1000 mL |
| G1-h | 4 mg/mL | 4.00g | 46.00g | 0.22g | 1.90g | | 1000 mL |
| G1-i | 4.5 mg/mL | 4.50g | 46.00g | 0.22g | 1.90g | | 1000 mL |
| G1-j | 5 mg/mL | 5.00g | 46.00g | 0.22g | 1.90g | | 1000 mL |
| G1-k | 5.5 mg/mL | 5.50g | 46.00g | 0.22g | 1.90g | | 1000 mL |
| G1-l | 6 mg/mL | 6.00g | 46.00g | 0.22g | 1.90g | | 1000 mL |
| G1-m | 7 mg/mL | 7.00g | 46.00g | 0.22g | 1.90g | | 1000 mL |
| G1-n | 8 mg/mL | 8.00g | 46.00g | 0.22g | 1.90g | | 1000 mL |
| G1-o | 20 mg/mL | 20.00g | 46.00g | 0.22g | 1.90g | | 1000 mL |
| G1-p | 24 mg/mL | 24.00g | 46.00g | 0.22g | 1.90g | | 1000 mL |
| G1-q | 30 mg/mL | 30.00g | 46.00g | 0.22g | 1.90g | | 1000 mL |

**Table 2-21-3 Stability results of composition G1-b (0.5mL:0.5mg, 1mg/mL)**

| Test Item | | 25°C+2°C/RH60%+5%, time | | | 5°C+3°C, time | |
|---|---|---|---|---|---|---|
| | 0 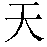 | 1 Month | 2 Months | 3 Months | 1 Month | 3 Months |
| Appearance | colorless clear liquid | colorless clear liquid | colorless clear liquid | colorless clear liquid | colorless clear liquid | colorless clear liquid |
| pH Value | 7.2 | 7.3 | 7.3 | 7.4 | 7.2 | 7.4 |
| Clarity and Color of Solution | clear and colorless solution | clear and colorless solution | clear and colorless solution | clear and colorless solution | clear and colorless solution | clear and colorless solution |
| Polymer | 0.03% | 0.07% | 0.05% | 0.10% | 0.05% | 0.03% |
| Related substances (Total Impurity) | 0.90% | 1.7% | 2.54% | 2.64% | 0.98% | 1.11% |
| Osmolarity | 283mOsmo l/kg | N/A | N/A | N/A | N/A | N/A |
| Visible Foreign Matter | No | No | No | No | No | No |
| Insoluble Particles | 32 particles | 95 particles | 7 particles | 61 particles | 101 particles | 33 particles |
| | 1 particle | 11 particles | 0 particle | 10 particles | 76 particles | 5 particles |
| API Content | 99.5% | 98.7% | 98.2% | 96.8% | 99.7% | 99.3% |

| | | | | | | |
|---|---|---|---|---|---|---|
| Note: In Table 2-21-3, the volume of the composition G1-b used for testing is 0.5 mL, the content of the polypeptide compound P016 is 0.5 mg, and the concentration of the polypeptide compound P016 in the composition is 1 mg/mL. | | | | | | |

**Table 2-21-4 Stability Result of compostions G1-d (0.5mL:1mg, 2mg/mL), G1-h (0.5mL:2mg, 4mg/mL), G1-l (0.5mL:3mg, 6mg/mL), G1-n (0.5mL:4mg, 8mg/mL)**

| Test Item | G1-d 2mg/mL | | | G1-h 4mg/mL | | | G1-l 6mg/mL | | | G1-n 8mg/mL | | |
|---|---|---|---|---|---|---|---|---|---|---|---|---|
| | Day 0 | 25°C±2 °C/RH60 %±5% 1 month | 5°C±3°C 1 month | Day 0 | 25°C±2 °C/RH60 %±5% 1 month | 5°C±3°C 1 month | Day 0 | 25°C±2 °C/RH60 %±5% 1 month | 5°C±3°C 1 month | Day 0 | 25°C±2 °C/RH60 %±5% 1 month | 5°C±3°C 1 month |
| Appearance | colorless and clear liquid | colorless and clear liquid | colorless and clear liquid | colorless and clear liquid | colorless and clear liquid | colorless and clear liquid | colorless and clear liquid | colorless and clear liquid | colorless and clear liquid | colorless and clear liquid | colorless and clear liquid | colorless and clear liquid |
| pH Value | 7.3 | 7.4 | 7.4 | 7.3 | 7.4 | 7.3 | 7.3 | 7.3 | 7.4 | 7.3 | 7.3 | 7.3 |
| Clarity and Color of Solution | clear colorless | clear colorless | clear colorless | clear colorless | clear colorless | clear colorless | clear colorless | clear colorless | clear colorless | clear colorless | clear colorless | clear colorless |
| Polymer | 0.03% | 0.05% | 0.03% | 0.03% | 0.04% | 0.02% | 0.03% | 0.04% | 0.03% | 0.02% | 0.04% | 0.02% |
| Related substances (Total Impurity) | 0.85% | 1.5% | 0.73% | 0.80% | 1.4% | 0.73% | 0.79% | 1.5% | 0.73% | 0.81% | 1.4% | 0.70% |
| Osmolarity | 284mOs mol/kg | N/A | N/A | 287mOs mol/kg | N/A | N/A | 288mOs mol/kg | N/A | N/A | 291mOs mol/kg | N/A | N/A |
| Visible Foreign Matter | No | No | No | No | No | No | No | No | No | No | No | No |
| Insoluble Particles | 74 particles | 136 particles | 72 particles | 108 particles | 147 particles | 63 particles | 137 particles | 145 particles | 86 particles | 157 particles | 175 particles | 74 particles |
| | 1 particle | 15 particles | 2 particles | 3 particles | 16 particles | 3 particles | 7 particles | 8 particles | 5 particles | 3 particles | 12 particles | 4 particles |
| API Content | 101.1% | 101.1% | 101.0% | 101.5% | 101.0% | 101.6% | 102.3% | 102.4% | 101.0% | 101.3% | 102.0% | 101.6% |

| | | | | | | | | | | | | |
|---|---|---|---|---|---|---|---|---|---|---|---|---|
| Note: There is a measurement error of no more than 2.5% for the content of active pharmaceutical ingredients (API). Note: In Table 2-21-3, the volume of the composition G1-d used for testing is 0.5 mL, the content of the polypeptide compound P016 is 1 mg, and the concentration of the polypeptide compound P016 in the composition is 2 mg/mL. The volume of the composition G1-h used for testing was 0.5 mL, the content of the polypeptide compound P016 was 2 mg, and the concentration of the polypeptide compound P016 in the composition was 4 mg/mL. The volume of the composition G1-1 used for testing was 0.5 mL, the content of the polypeptide compound P016 was 3 mg, and the concentration of the polypeptide compound P016 in the composition was 6 mg/mL. The volume of the composition G1-n used for testing is 0.5 mL, the content of the polypeptide compound P016 is 4 mg, and the concentration of the polypeptide compound P016 in the composition is 8 mg/mL. | | | | | | | | | | | | |

**Table 2-21-5 Stability result of composition G1-p (0.5mL:12mg, 24mg/mL)**

| Test Item | | 25°C±2°C/RH60%±5%, time | | | 5°C±3°C, time | |
|---|---|---|---|---|---|---|
| | Day 0 | 1 month | 2 months | 3 months | 1 month | 3 months |
| Appearance | Colorless and clear liquid | Colorless and clear liquid | Colorless and clear liquid | Colorless and clear liquid | Colorless and clear liquid | Colorless and clear liquid |
| pH Value | 7.2 | 7.3 | 7.1 | 7.4 | 7.2 | 7.4 |
| Clarity and Color of Solution | Colorless and clear solution | Colorless and clear solution | Colorless and clear solution | Colorless and clear solution | Colorless and clear solution | Colorless and clear solution |
| Polymer | 0.04% | 0.07% | 0.05% | 0.11% | 0.05% | 0.03% |
| Related substances (Total Impurity) | 0.88% | 1.6% | 2.64% | 2.69% | 1.0% | 1.07% |
| Osmolarity | 298mOsmol/ kg | N/A | N/A | N/A | N/A | N/A |
| Visible Foreign Matter | No | No | No | No | No | No |
| Insoluble Particles | 125 particles | 85 particles | 244 particles | 28 particles | 110 particles | 57 particles |
| | 35 particles | 3 particles | 2 particles | 1 particle | 72 particles | 4 particles |
| API Content | 101.1% | 100.8% | 99.8% | 98.6% | 101.3% | 100.6% |

| | | | | | | |
|---|---|---|---|---|---|---|
| Note: There is a measurement error of no more than 2.5% in the content of active pharmaceutical ingredients. Note: In Table 2-21-5, the volume of the composition G1-p used for testing is 0.5 mL, the content of the polypeptide compound P016 is 12 mg, and the concentration of the polypeptide compound P016 in the composition is 24 mg/mL. | | | | | | |

**Table 2-21-6 Stability Result of Composition G1-q (0.5mL:15mg, 30 mg/mL)**

| Test Item | Test Result | | | | |
|---|---|---|---|---|---|
| | Day 0 | 40°C 10 days | 40°C 30 days | 25±2°C, RH60%±5% 30 days | In light, 10 days |
| Appearance | Colorless and clear liquid | Colorless and clear liquid | Colorless and clear liquid | Colorless and clear liquid | Colorless and clear liquid |
| pH Value | 7.3 | 7.3 | 7.2 | 7.2 | 7.3 |
| Clarity and Color of Solution | Colorless and clear solution | Colorless and clear solution | Colorless and clear solution | Colorless and clear solution | Colorless and clear solution |
| Insoluble Particles | 245 particles | 339 particles | 195 particles | 214 particles | 130 particles |
| | 21 particles | 20 particles | 31 particles | 117 particles | 23 particles |
| Polymer | 0.05% | 0.12% (12 days) | 0.29% | 0.11% | 0.26% |
| Related Substances | 0.83% | 2.54% (12 days) | 4.86% | 1.36% | 0.89 |

| | | | | | |
|---|---|---|---|---|---|
| Note: In Table 2-21-6, the volume of the composition G1-q used for testing is 0.5 mL, the content of the polypeptide compound P016 is 15 mg, and the concentration of the polypeptide compound P016 in the composition is 30 mg/mL. | | | | | |

The results in Table 2-21-3 to Table 2-21-6 above showed that representative compositions with different drug concentrations, for example, drug concentrations ranging from 1 mg/mL to 30 mg/mL, all showed temperature-dependent stability. Moreover, at 25°C, under the index of related substances (total impurities), each composition showed a considerable stability trend. At 2-8°C, indexes such as Appearance, pH, clarity and color of solution, polymer, content, related substances, insoluble particles, visible foreign matter, etc. are stable, indicating that the compositions have excellent stability at 2-8°C.

### [Preparation of Composition G2]

According to Table 2-22 below, 12.65 g of mannitol, 0.52 g of trisodium citrate dihydrate, and 0.06 g of citric acid monohydrate were weighed into a beaker respectively, into which 220 mL of injection water was added and the mixture was stirred homogeneously. 1.1 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 7.2 (allowing pH being within 6.8-7.4). Injection water was added to adjust the volume to 275 mL, and the mixture was mixed homogeneously, filtered through a 0.22 µm PES membrane, thereby obtaining Composition G2 having the following composition: 4 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 1.05 mM: 6.45 mM, that is 1:6.15) as a buffer. Its pH is 7.2 (allowing pH being within 6.8-7.4).

A Composition G2 sample was obtained by: filtering the composition through a 0.22 µm PES membrane; and aliquoting it into a 2 mL neutral borosilicate glass tubing injection vial (sterilized) at a volume of 1.1 mL per vial (250 vials in total); fully inserting into each vial an injection butyl rubber stopper locally coated with a PTFE film (sterilized), and then capping with a cap made of aluminium-plastics composite for antibiotics bottles.

**Table 2-22 Composition of Composition G2**

| Component | Content | Function |
|---|---|---|
| Polypeptide Compound P016 (API) | 1.1 g (4 mg/mL) | Active Pharmaceutical Ingredient |
| Mannitol | 12.65 g (46 mg/mL) | Osmotic Pressure Regulator |
| Trisodium Citrate Dihydrate | 0.52 g (6.45 mM) | Buffer |
| Citric Acid Monohydrate | 0.06 g (1.05 mM) | Buffer |
| adjusting pH with 0.1M NaOH or 0.1M HCl aqueous solution to | 7.2 (allowing pH within 6.8-7.4) | pH adjusting agent |
| adding injection water to | 275 mL | Solvent |

### [Preparation of Composition G3]

According to Table 2-23 below, 12.65 g of mannitol, 0.52 g of trisodium citrate dihydrate, and 0.06 g of citric acid monohydrate were weighed into a beaker respectively, into which 220 mL of injection water was added and the mixture was stirred homogeneously. 8.25 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 7.2 (allowing pH being within 6.8-7.4). Injection water was added to adjust the volume to 275 mL, and the mixture was mixed homogeneously, thereby obtaining Composition G3 having the following composition: 30 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 1.05 mM: 6.45 mM, that is 1:6.15) as a buffer. Its pH is 7.2 (allowing pH being within 6.8-7.4).

A Composition G3 sample was obtained by: filtering the composition through a 0.22 µm PES membrane; and aliquoting it into a 2 mL neutral borosilicate glass tubing injection vial (sterilized) at a volume of 1.1 mL per vial (250 vials in total); fully inserting into each vial an injection butyl rubber stopper locally coated with a PTFE film (sterilized), and then capping with a cap made of aluminium-plastics composite for antibiotics bottles.

**Table 2-23 Composition of Composition G3**

| Component | Content | Function |
|---|---|---|
| Polypeptide Compound P016 (API) | 8.25 g (30 mg/mL) | Active Pharmaceutical Ingredient |
| Mannitol | 12.65 g (46 mg/mL) | Osmotic Pressure Regulator |
| Trisodium Citrate Dihydrate | 0.52 g (6.45 mM) | Buffer |
| Citric Acid Monohydrate | 0.06 g (1.05 mM) | Buffer |
| adjusting pH with 0.1M NaOH or 0.1M HCl aqueous solution to | 7.2 (allowing pH within 6.8-7.4) | pH adjusting agent |
| adding injection water to | 275 mL | Solvent |

### [Preparation of Composition G4]

According to Table 2-24 below, 46.00g of mannitol, 1.985 g of trisodium citrate dihydrate, and 0.158 g of citric acid monohydrate were weighed into a beaker respectively, into which 800 mL of injection water was added and the mixture was stirred homogeneously. 0.5-1 g or 1-30.00 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, and filtered through a 0.22 µm PES membrane, thereby obtaining Composition G4 having the following composition: 0.5-1 mg/mL or 1-30 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 0.75 mM: 6.75 mM, that is 1:9) as a buffer. Its pH is 6.8-7.4.

A Composition G4 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-24 Composition of Composition G4**

| Component | Content | Function |
|---|---|---|
| Polypeptide Compound P016 (API) | 0.5-1 g or 1-30.00 g (0.5-1 mg/mL or 1-30 mg/mL) | Active Pharmaceutical Ingredient |
| Mannitol | 46.00 g (46 mg/mL) | Osmotic Pressure Regulator |
| Trisodium Citrate Dihydrate | 1.985 g (6.75 mM) | Buffer |
| Citric Acid Monohydrate | 0.158 g (0.75 mM) | Buffer |
| adjusting pH with 0.1M NaOH or 0.1M HCl aqueous solution to | 6.8-7.4 | pH adjusting agent |
| adding injection water to | 1000 mL | Solvent |

### [Preparation of Composition G5]

According to Table 2-25 below, 46.00g of mannitol, 1.324 g of trisodium citrate dihydrate, and 0.105 g of citric acid monohydrate were weighed into a beaker respectively, into which 800 mL of injection water was added and the mixture was stirred homogeneously. 0.5-1 g or 1-30.00 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, and filtered through a 0.22 µm PES membrane, thereby obtaining Composition G5 having the following composition: 0.5-1 mg/mL or 1-30 mg/mL of Polypeptide Compound P016 of the present invention as an active pharmaceutical ingredient (API), 46 mg/mL of mannitol as an osmotic pressure regulator, and 5.0 mM citric acid-trisodium citrate buffer (the molar ratio of citric acid to trisodium citrate is 0.50 mM: 4.50 mM, that is 1:9) as a buffer. Its pH is 6.8-7.4.

A Composition G5 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-25 Composition of Composition G5**

| Component | Content | Function |
|---|---|---|
| Polypeptide Compound P016 (API) | 0.5-1 g or 1-30.00 g (0.5-1 mg/mL or 1-30 mg/mL) | Active Pharmaceutical Ingredient |
| Mannitol | 46.00 g (46 mg/mL) | Osmotic Pressure Regulator |
| Trisodium Citrate Dihydrate | 1.324 g (4.50 mM) | Buffer |
| Citric Acid Monohydrate | 0.105 g (0.50 mM) | Buffer |
| adjusting pH with 0.1M NaOH or 0.1M HCl aqueous solution to | 6.8-7.4 | pH adjusting agent |
| adding injection water to | 1000 mL | Solvent |

### [Preparation of Composition G6]

According to Table 2-26 below, 46.00g of mannitol, 1.90 g of trisodium citrate dihydrate, and 0.22 g of citric acid monohydrate were weighed into a beaker respectively, into which 800 mL of injection water was added and the mixture was stirred homogeneously. 1.00 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, thereby obtaining Composition G6 having the following composition: 1.00 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 1.05 mM: 6.45 mM, that is 1:6.15) as a buffer. Its pH is 6.8-7.4.

A Composition G6 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-26 Composition of Composition G6**

| Component | Content | Function |
|---|---|---|
| Polypeptide Compound P016 (API) | 1.00 g (1.00 mg/mL) | Active Pharmaceutical Ingredient |
| Mannitol | 46.00 g (46 mg/mL) | Osmotic Pressure Regulator |
| Trisodium Citrate Dihydrate | 1.90 g (6.45 mM) | Buffer |
| Citric Acid Monohydrate | 0.22 g (1.05 mM) | Buffer |
| adjusting pH with 0.1M NaOH or 0.1M HCl aqueous solution to | 6.8-7.4 | pH adjusting agent |
| adding injection water to | 1000 mL | Solvent |

### [Preparation of Composition G7]

According to Table 2-27 below, 46.00g of mannitol, 1.985 g of trisodium citrate dihydrate, and 0.158 g of citric acid monohydrate were weighed into a beaker respectively, into which 800 mL of injection water was added and the mixture was stirred homogeneously. 1.00 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, thereby obtaining Composition G7 having the following composition: 1.00 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 0.75 mM: 6.75 mM, that is 1:9) as a buffer. Its pH is 6.8-7.4.

A Composition G7 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-27 Composition of Composition G7**

| Component | Content | Function |
|---|---|---|
| Polypeptide Compound P016 (API) | 1.0 g (1.00 mg/mL) | Active Pharmaceutical Ingredient |
| Mannitol | 46.00 g (46 mg/mL) | Osmotic Pressure Regulator |
| Trisodium Citrate Dihydrate | 1.985 g (6.75 mM) | Buffer |
| Citric Acid Monohydrate | 0.158 g (0.75 mM) | Buffer |
| adjusting pH with 0.1M NaOH or 0.1M HCl aqueous solution to | 6.8-7.4 | pH adjusting agent |
| adding injection water to | 1000 mL | Solvent |

### [Preparation of Composition G8-A and Composition G8-B]

According to Table 2-28 below, 46.00g of mannitol, 1.90 g of trisodium citrate dihydrate, and 0.22 g of citric acid monohydrate were weighed into a beaker respectively, into which 800 mL of injection water was added and the mixture was stirred homogeneously. 30.00 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, thereby obtaining Composition G8-B having the following composition: 30.00 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 1.05 mM: 6.45 mM, that is 1:6.15) as a buffer. Its pH is 6.8-7.4.

Similar to the above method, Composition G8-A was prepared, except that the amount of polypeptide compound P016 was 4.00 g.

A G8-A or G8-B Composition sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-28-1 Composition of Composition G8-A and Composition G8-B (batch size being scaled up or down proportionally)**

| Component | Content of G8-A | Content of G8-B | Function |
|---|---|---|---|
| Polypeptide Compound P016 (API) | 4.0g (4.00 mg/mL) | 30.0g (30.00 mg/mL) | Active Pharmaceutical Ingredient |
| Mannitol | 46.00 g (46mg/mL) | 46.00 g (46mg/mL) | Osmotic Pressure Regulator |
| Trisodium Citrate Dihydrate | 1.90 g (6.45 mM) | 1.90 g (6.45 mM) | Buffer |
| Citric Acid Monohydrate | 0.22 g (1.05 mM) | 0.22 g (1.05 mM) | Buffer |
| adjusting pH with 0.1M NaOH or 0.1M HCl aqueous solution to | 6.8-7.4 | 6.8-7.4 | pH adjusting agent |
| adding injection water to | 1000 mL | 1000 mL | Solvent |

**Table 2-28-2 Stability Result of G8-A (4 mg/mL)**

| Test Item | | 25°C±2°C/RH60%±5%, time | | | 5°C±3°C, time | | |
|---|---|---|---|---|---|---|---|
| | Day 0 | 1 month | 3 months | 6 months | 3 months | 6 months | 12 months |
| Appearance | colorless and clear liquid | colorless and clear liquid | colorless and clear liquid | colorless and clear liquid | colorless and clear liquid | colorless and clear liquid | colorless and clear liquid |
| pH Value | 7.3 | 7.2 | 7.3 | 7.2 | 7.3 | 7.3 | 7.4 |
| Clarity and Color of Solution | clear and colorless | clear and colorless | clear and colorless | clear and colorless | clear and colorless | clear and colorless | clear and colorless |
| Polymer | 0.04% | 0.08% | 0.13% | 0.20% | 0.06% | 0.07% | 0.08% |
| Related Substance (Total Impurity) | 0.87% | 1.6% | 3.2% | 5.0% | 1.1% | 1.2% | 1.2% |
| Osmolarity | 292 mOsmol/kg | 294 mOsmol/kg | 288 mOsmol/kg | 287 mOsmol/kg | 287 mOsmol/kg | 286 mOsmol/kg | 282 mOsmol/kg |
| Visible Foreign Matter | No | No | No | No | No | No | No |
| Insoluble Particles | 15 particles | 4 particles | 38 particles | 8 particles | 7 particles | 11 particles | 19 particles |
| | 1 particle | 0 particles | 2 particles | 4 particles | 0 particles | 1 particle | 7 particles |
| API Content | 99.2% | 99.3% | 96.3% | 95.2% | 99.6% | 99.2% | 98.1% |

**2-28-3 Stability Result of G8-B (30 mg/mL)**

| Test Item | | 25°C±2°C/RH60%±5%, time | | | 5°C±3°C, time | | |
|---|---|---|---|---|---|---|---|
| | Day 0 | 1 month | 3 months | 6 months | 3 months | 6 months | 12 months |
| Appearance | colorless and clear liquid | colorless and clear liquid | colorless and clear liquid | colorless and clear liquid | colorless and clear liquid | colorless and clear liquid | colorless and clear liquid |
| pH Value | 7.3 | 7.3 | 7.3 | 7.4 | 7.4 | 7.4 | 7.5 |
| Clarity and Color of Solution | clear and colorless | clear and colorless | clear and colorless | clear and colorless | clear and colorless | clear and colorless | clear and colorless |
| Polymer | 0.05% | 0.09% | 0.13% | 0.19% | 0.07% | 0.07% | 0.08% |
| Related Substance (Total Impurity) | 0.87% | 1.6% | 3.4% | 5.1% | 1.1% | 1.2% | 1.3% |
| Osmolarity | 303 mOsmol/kg | 316 mOsmol/kg | 307 mOsmol/kg | 306 mOsmol/kg | 308 mOsmol/kg | 305 mOsmol/kg | 301 mOsmol/kg |
| Visible Foreign Matter | No | No | No | No | No | No | No |
| Insoluble Particles | 58 particles | 21 particles | 10 particles | 18 particles | 4 particles | 65 particles | 46 particles |
| | 7 particles | 0 particle | 0 particle | 1 particle | 0 particle | 3 particles | 6 particles |
| API Content | 100.6% | 101.1% | 96.7% | 94.4% | 99.4% | 98.1% | 100.4% |

According to Table 2-28-2 and Table 2-28-3, the composition stability data showed that Composition G8-A and Composition G8-B have temperature-dependent stability. Moreover, after long-term storage at 2-8°C for 12 months, the composition indexes were still stable, indicating that the compositions have excellent stability, and all indexes meet the requirements for subcutaneous injection.

### [Preparation of Composition G9]

According to Table 2-29 below, 46.00g of mannitol, 1.985 g of trisodium citrate dihydrate, and 0.158 g of citric acid monohydrate were weighed into a beaker respectively, into which 800 mL of injection water was added and the mixture was stirred homogeneously. 30.0 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, thereby obtaining Composition G9 having the following composition: 30.00 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 0.75 mM: 6.75 mM, that is 1:9) as a buffer. Its pH is 6.8-7.4.

A Composition G9 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-29 Composition of Composition G9**

| Component | Content | Function |
|---|---|---|
| Polypeptide Compound P016 (API) | 30.0 g (30.00 mg/mL) | Active Pharmaceutical Ingredient |
| Mannitol | 46.00 g (46 mg/mL) | Osmotic Pressure Regulator |
| Trisodium Citrate Dihydrate | 1.985 g (6.75 mM) | Buffer |
| Citric Acid Monohydrate | 0.158 g (0.75 mM) | Buffer |
| adjusting pH with 0.1M NaOH or 0.1M HCl aqueous solution to | 6.8-7.4 | pH adjusting agent |
| adding injection water to | 1000 mL | Solvent |

### [Preparation of Composition G10]

According to Table 2-30 below, 46.00g of mannitol, 1.324 g of trisodium citrate dihydrate, and 0.105 g of citric acid monohydrate were weighed into a beaker respectively, into which 800 mL of injection water was added and the mixture was stirred homogeneously. 1.00 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, thereby obtaining Composition G10 having the following composition: 1.00 mg/mL of Polypeptide Compound P016 of the present invention as an active pharmaceutical ingredient (API), 46 mg/mL of mannitol as an osmotic pressure regulator, and 5.0 mM citric acid-trisodium citrate buffer (the molar ratio of citric acid to trisodium citrate is 0.50 mM: 4.50 mM, that is 1:9) as a buffer. Its pH is 6.8-7.4.

A Composition G10 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-30 Composition of Composition G10**

| Component | Content | Function |
|---|---|---|
| Polypeptide Compound P016 (API) | 1.0g (1.00 mg/mL) | Active Pharmaceutical Ingredient |
| Mannitol | 46.00 g (46mg/mL) | Osmotic Pressure Regulator |
| Trisodium Citrate Dihydrate | 1.324 g (4.50 mM) | Buffer |
| Citric Acid Monohydrate | 0.105 g (0.50 mM) | Buffer |
| adjusting pH with 0.1M NaOH or 0.1M HCl aqueous solution to | 6.8-7.4 | pH adjusting agent |
| adding injection water to | 1000 mL | Solvent |

### [Preparation of Composition G11]

According to Table 2-31 below, 46.00g of mannitol, 1.324 g of trisodium citrate dihydrate, and 0.105 g of citric acid monohydrate were weighed into a beaker respectively, into which 800 mL of injection water was added and the mixture was stirred homogeneously. 30.0 g of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 6.8-7.4. Injection water was added to adjust the volume to 1000 mL, and the mixture was mixed homogeneously, thereby obtaining Composition G11 having the following composition: 30.00 mg/mL of Polypeptide Compound P016 of the present invention as an active pharmaceutical ingredient (API), 46 mg/mL of mannitol as an osmotic pressure regulator, and 5.0 mM citric acid-trisodium citrate buffer (the molar ratio of citric acid to trisodium citrate is 0.50 mM: 4.50 mM, that is 1:9) as a buffer. Its pH is 6.8-7.4.

A Composition G11 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-31 Composition of Composition G11**

| Component | Content | Function |
|---|---|---|
| Polypeptide Compound P016 (API) | 30.0g (30.00 mg/mL) | Active Pharmaceutical Ingredient |
| Mannitol | 46.00 g (46mg/mL) | Osmotic Pressure Regulator |
| Trisodium Citrate Dihydrate | 1.324 g (4.50 mM) | Buffer |
| Citric Acid Monohydrate | 0.105 g (0.50 mM) | Buffer |
| adjusting pH with 0.1M NaOH or 0.1M HCl aqueous solution to | 6.8-7.4 | pH adjusting agent |
| adding injection water to | 1000 mL | Solvent |

The Compositions G1-G11 described above in the present invention all exhibited superior stability of solution clarity, color, purity, polymer content, and pH value over time at a high temperature.

### [Composition Example 8 - Preservative Formulation]

### [Preparation of Composition H1]

According to Table 2-32 below, 920 mg of mannitol, 37.94 mg of trisodium citrate dihydrate, and 4.41 mg of citric acid monohydrate were weighed into a beaker respectively, into which 16 mL of injection water was added and the mixture was stirred homogeneously. 600 mg of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 7.2. Injection water was added to adjust the volume to 20 mL, and the mixture was mixed homogeneously, thereby obtaining Composition H1 having the following composition: 30.00 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 1.05 mM:6.45 mM) as a buffer. Its pH is 7.2.

A Composition H1 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-32 Composiiton of Compositions H1 to H3 and Comparative Compositions H-C1 to H-C3**

| Components | H1 | H2 | H3 | H-C1 | H-C2 | H-C3 |
|---|---|---|---|---|---|---|
| Polypeptide compound P016 (API) | 600mg (30 mg/mL) | 600mg (30 mg/mL) | 600mg (30 mg/mL) | 600mg (30 mg/mL) | 600mg (30 mg/mL) | 600mg (30 mg/mL) |
| Mannitol | 920mg (46mg/mL) | 920mg (46mg/mL) | 800mg (40 mg/mL) | 920mg (46mg/mL) | / | / |
| citric acid monohydrate | 4.41mg (1.05 mM) | 4.41mg (1.05 mM) | 4.41mg (1.05 mM) | / | 4.41mg (1.05 mM) | 4.41mg (1.05 mM) |
| trisodium citrate dihydrate | 37.94mg (6.45 mM) | 37.94mg (6.45 mM) | 37.94mg (6.45 mM) | / | 37.94mg (6.45 mM) | 37.94mg (6.45 mM) |
| Sodium dihydrogen phosphate | / | / | / | 17.94mg (7.5 mM) | / | / |
| Propylene glycol | / | / | / | / | / | 560mg (28 mg/mL) |
| Sodium chloride | / | / | / | / | 160mg (8 mg/mL) | / |
| m-cresol | / | 60mg (3 mg/mL) | / | / | / | / |
| phenol | | / | 110mg (5.5 mg/mL) | | | |
| adjusting pH with 0.1M NaOH or 0.1M HCl aqueous solution to | 7.2 | 7.2 | 7.2 | 7.2 | 7.2 | 7.2 |
| adding injection water to | 20 mL | 20 mL | 20 mL | 20 mL | 20 mL | 20 mL |

| | | | | | | |
|---|---|---|---|---|---|---|
| Note: The amount of auxiliary materials allows an error of no more than 5%. For example, for 4.41 mg of citric acid monohydrate, it can vary within the range of 4.378-4.422 mg. | | | | | | |

### [Comparative Composition H-C1, H-C2, H-C3]

As shown in Table 2-32 above, comparative compositions H-C1, H-C2 and H-C3 were prepared by using the same method as preparing H1. The only difference between H-C1 and H1 is that 37.94 mg of trisodium citrate dihydrate and 4.41 mg of citric acid monohydrate in H1 were replaced with 17.94 mg of sodium dihydrogen phosphate (7.5 mM); the only difference between H-C2 and H1 is that 920 mg of mannitol in H1 is replaced with 160 mg sodium chloride; the only difference between H-C3 and H1 is that 920 mg mannitol in H1 is replaced with 560 mg propylene glycol.

### [Preparation of Composition H2 and Composition H3]

According to Table 2-32 above, 920 mg of mannitol, 37.94 mg of trisodium citrate dihydrate, and 4.41 mg of citric acid monohydrate were weighed into a beaker respectively, into which 16 mL of injection water was added and the mixture was stirred homogeneously. 600 mg of Polypeptide Compound P016 (API) was slowly added to the beaker with stirring. The pH value of the resulting mixture was measured. Then a 0.1 M NaOH or 0.1 M HCl aqueous solution was added to the beaker to adjust the pH value to 7.2. 60 mg m-cresol or 110 mg phenol was added with stirring until homogeneously dissolved, then the pH was finely tuned to 7.2. Injection water was added to adjust the volume to 20 mL, and the mixture was mixed homogeneously, thereby obtaining Composition H2 or H3 having the following composition: 30.00 mg/mL of Polypeptide Compound P016 of the present invention as an active pharmaceutical ingredient (API), 46 mg/mL of mannitol as an osmotic pressure regulator, and 7.5 mM citric acid-trisodium citrate buffer (the molar ratio of citric acid to trisodium citrate is 1.05 mM:6.45 mM) as a buffer. Its pH is 7.2.

A Composition H2 or H3 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-33 Clarity and Color of Different Compositions**

| Time Point | Temperature | composition H1 | composition H2 | composition H3 | comparative composition H-C1 | comparative composition H-C2 | comparative composition H-C3 |
|---|---|---|---|---|---|---|---|
| 10 days | -20°C | clear colorless | clear colorless | clear colorless | clear colorless | clear colorless | clear colorless |
| | 40°C | clear colorless | clear colorless | clear colorless | colorless, tubidity 2~3 | clear colorless | clear colorless |
| | 60°C | clear colorless | clear colorless | clear colorless | clear colorless | clear colorless | clear colorless |
| | 25°C | clear colorless | clear colorless | clear colorless | colorless, tubidity 2~3 | colorless, tubidity1 | clear colorless |
| | 2~8°C | clear colorless | clear colorless | clear colorless | colorless, tubidity 3~4 | clear colorless | clear colorless |
| 1 month | 40°C | clear colorless | clear colorless | clear colorless | colorless, tubidity 4 | clear colorless | clear colorless |
| | 25°C | clear colorless | clear colorless | clear colorless | colorless, tubidity 4 | colorless, tubidity 4 | colorless, tubidity 2~3 |
| | 2~8°C | clear colorless | clear colorless | clear colorless | colorless, tubidity 4 | clear colorless | clear colorless |
| 3 months | 25°C | clear colorless | clear colorless | clear colorless | colorless, tubidity 4 | colorless, tubidity 4 | colorless, tubidity 2~3 |
| | 2~8°C | clear colorless | clear colorless | clear colorless | colorless, tubidity 4 | colorless, tubidity1~2 | clear colorless |
| 6 months | 40°C | clear, yellow 2 | clear, yellow 0.5~1 | clear, yellow 1 | clear, yellow 2 | clear, yellow 0.5~1 | clear, yellow 1 |
| | 60°C | clear, yellow 7 | clear, yellow 7~8 | clear, yellow 7~8 | clear, yellow 10 | clear, yellow 7~8 | clear, yellow 7~8 |
| | 25°C | clear colorless | clear colorless | clear colorless | colorless, tubidity 4 | colorless, tubidity 4 | colorless, tubidity 4 |
| | 2~8°C | clear colorless | clear colorless | clear colorless | colorless, tubidity 4 | colorless, tubidity 1~2 | colorless, tubidity 1 |

The results in Table 2-33 show:
1) Compared with the comparable composition H-C1 (phosphate), H-C2 (sodium chloride), H-C3 (propylene glycol), composition H1 (containing citrate and mannitol) still showed better solution clarity Spend.
2) Unexpectedly, compositions H2 and H3 containing phenol or m-cresol as preservative have smaller color darkness changes at 40°C than composition H1 without preservatives. The addition of preservatives improved the stability of color property of the composition.

**Table 2-34 HPLC-UV determination of chromatographic purity of different compositions**

| Time Point | Temperature | composition H1 | composition H2 | composition H3 | comparative composition H-C1 | comparative composition H-C2 | comparative composition H-C3 |
|---|---|---|---|---|---|---|---|
| 10 days | -20°C | 96.52% | 96.40% | 96.43% | 96.51% | 96.52% | 96.53% |
| | 40°C | 94.74% | 94.72% | 94.41% | 94.35% | 94.75% | 94.73% |
| | 60°C | 81.53% | 81.26% | 80.38% | 77.89% | 80.89% | 82.30% |
| 1 month | 40°C | 91.46% | 90.99% | 90.94% | 88.28% | 91.13% | 91.41% |
| 3 months | 2~8°C | 96.03% | 96.45% | 96.42% | 84.52% | 95.92% | 96.41% |
| | 25°C | 93.69% | 94.21% | 93.86% | 78.32% | 41.49% | 87.48% |
| 6 months | 2~8°C | 95.83% | 95.82% | 95.79% | / | / | / |
| | 25°C | 90.71% | 91.91% | 91.63% | / | / | / |

**Table 2-35 SEC-HPLC determination of polymers in different compositions**

| Time point | temperature | composition H1 | composition H2 | composition H3 | comparative composition H-C1 | comparative composition H-C2 | comparative composition H-C3 |
|---|---|---|---|---|---|---|---|
| 10 days | -20°C | 0.07% | 0.09% | 0.09% | 0.08% | 0.12% | 0.10% |
| | 40°C | 0.15% | 0.17% | 0.18% | 0.20% | 0.18% | 0.16% |
| | 60°C | 0.59% | 0.63% | 0.60% | 2.92% | 0.68% | 0.55% |
| 1 month | 40°C | 0.25% | 0.26% | 0.25% | 0.22% | 0.30% | 0.25% |
| 3 months | 2~8°C | 0.13% | 0.13% | 0.13% | 0.11% | 0.13% | 0.12% |
| | 25°C | 0.12% | 0.21% | 0.22% | 0.29% | 0.39% | 1.06% |
| 6 months | 2~8°C | 0.07% | 0.08% | 0.08% | / | / | / |
| | 25°C | 0.24% | 0.18% | 0.17% | / | / | / |

In summary, at high concentrations of active P016 drug, compared to the preservative-free formulation of H1, the preservative-containing H2 and H3 formulations also showed comparable profiles of polymer, related substances, clarity and stability, and improved the thermal stability of the color property. Comparison between compositions H1 to H3 of the present invention and comparative compositions H-C1 to H-C3 once again showed that the auxiliary material composition of citrate buffer system and mannitol osmotic pressure regulator had excellent thermal stability of physical and chemical characteristics.

### [Composition Example 9 - Preservative Formulation]

15 mM citrate buffer: 1) 15 mM citric acid: Weighing 0.3143 g of citric acid monohydrate, diluting with water and dissolvingto 100 mL. 2) 15 mM trisodium citrate: Weighing 0.4414 g trisodium citrate dihydrate, diluting with water and dissolving to 100 mL. 3) Mixing 10.5mL of 15 mM citric acid and 64.5mL of 15 mM trisodium citrate to prepare a 15 mM citrate buffer.

55 mg/3 mL phenol stock solution: Taking the correct amount of phenol, diluting it with water and dissolving it to a constant volume.

30 mg/3 mL m-cresol stock solution: Taking an accurate amount of m-cresol, diluting it with water and dissolving it to a constant volume.

Preparation of compositions I1 to I12: According to the following table 2-36, adding an appropriate amount of mannitol into each container, adding an appropriate amount of injection water, citrate buffer, glycerol or propylene glycol, and sodium chloride as needed, stirting and mixing; weighing an appropriate amount of P016 polypeptide and adding it slowly, shaking well and dissolving to obtain a clear solution, then usig 0.1M HCl or 0.1M NaOH to adjust the pH value, adding 3 mL of phenol stock solution or m-cresol stock solution dropwise as needed, adjusting the pH to the target value as needed, and adjusting the volume to a final volume of 10 mL with injection water, and mixing well. A Composition I1 or I2 sample was obtained by: filtering the composition through a 0.22 µm PES membrane; and aliquoting it into a 2 mL neutral borosilicate glass tubing injection vial (sterilized) at a volume of 1.0 mL per vial; fully inserting into each vial an injection butyl rubber stopper locally coated with a PTFE film (sterilized), and then capping with a cap made of aluminium-plastics composite for antibiotics bottles.

**Table 2-36 Composition of Compositions I1 to I12**

| No. | Component | I1 | I2 | I3 | I4 | I5 | I6 | I7 | I8 | I9 | I10 | I11 | I12 |
|---|---|---|---|---|---|---|---|---|---|---|---|---|---|
| 1 | Polypeptide compound P016 (API) | 300mg | 300mg | 300mg | 300mg | 300mg | 300mg | 300mg | 300mg | 300mg | 300mg | 300mg | 300mg |
| 2 | 15 mM citrate buffer | SmL (7.5 mM) | SmL (7.5 mM) | SmL (7.5 mM) | SmL (7.5 mM) | SmL (7.5 mM) | SmL (7.5 mM) | SmL (7.5 mM) | SmL (7.5 mM) | SmL (7.5 mM) | SmL (7.5 mM) | SmL (7.5 mM) | SmL (7.5 mM) |
| 3 | Mannitol | 460mg (46mg/mL ) | 460mg (46mg/mL ) | / | / | 220mg (22mg/mL ) | 220mg (22mg/mL ) | / | / | 180mg (18mg/mL) | 200mg (20mg/mL) | / | / |
| 4 | Glycerol | / | / | 220mg (22mg/mL ) | / | 120mg (12mg/mL ) | / | / | 120mg (12mg/mL ) | 120mg (12mg/mL) | / | / | 120mg (12mg/mL) |
| 5 | Propylene glycol | / | / | / | 160mg (16mg/mL) | / | 100mg (10mg/mL ) | 110mg (11mg/mL ) | / | / | 100mg (10mg/mL) | 100mg (10mg/mL) | / |
| 6 | Sodium chloride | / | / | / | / | / | / | 35mg | 35mg | / | / | 30mg | 30mg |
| 7 | 55 mg/3 mL phenol | / | / | / | 3mL (5.5mg/mL ) | / | / | / | / | 3mL (5.5mg/mL ) | 3mL (5.5mg/mL ) | 3mL (5.5mg/mL ) | 3mL (5.5mg/mL ) |
| 8 | 30 mg/3 mL m-cresol | / | / | 3mL (3 mg/mL) | / | 3mL (3 mg/mL) | 3mL (3 mg/mL) | 3mL (3 mg/mL) | 3mL (3 mg/mL) | / | / | / | / |
| 9 | Injection water | 3mL | 3mL | 0 | 0 | 0 | 0 | 0 | 0 | 0 | 0 | 0 | 0 |
| 10 | adjusting pH with 0.1M NaOH or 0.1M HCl aqueous solution to | 7.2 | 8.0 | 7.2 | 7.2 | 7.2 | 7.2 | 7.2 | 7.2 | 7.2 | 7.2 | 7.2 | 7.2 |
| 11 | adding injection water to | 10 mL | 10 mL | 10 mL | 10 mL | 10 mL | 10 mL | 10 mL | 10 mL | 10 mL | 10 mL | 10 mL | 10 mL |

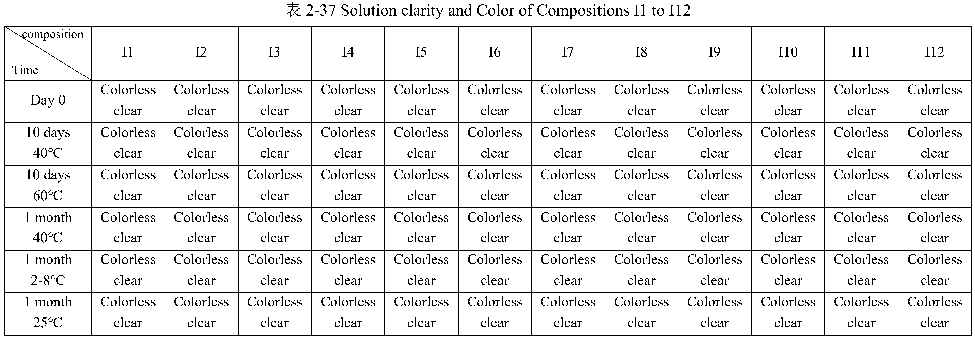

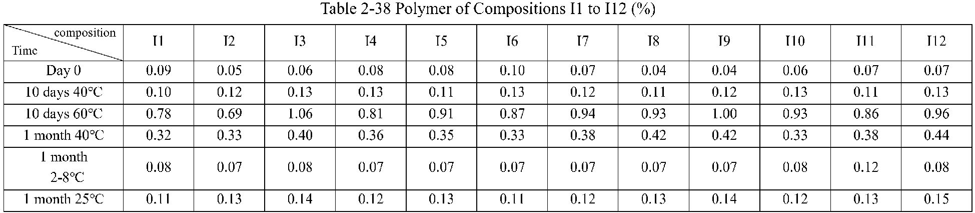

**Table 2-39 Purity of Compositions I1 to I12 (%)**

| composition | I1 | I2 | I3 | I4 | I5 | I6 | I7 | I8 | I9 | I10 | I11 | I12 |
|---|---|---|---|---|---|---|---|---|---|---|---|---|
| Day 0 | 99.15 | 99.08 | 99.13 | 99.13 | 99.07 | 99.15 | 99.19 | 99.13 | 99.21 | 99.12 | 99.15 | 99.11 |
| 10 days, 40°C | 97.06 | 97.03 | 96.21 | 96.97 | 97.21 | 97.22 | 97.35 | 97.41 | 97.07 | 97.21 | 97.40 | 97.20 |
| 10 days, 60°C | 73.72 | 73.12 | 70.72 | 73.38 | 72.89 | 73.01 | 72.77 | 74.10 | 71.18 | 70.89 | 74.74 | 74.48 |
| 1 month, 40°C | 90.82 | 90.03 | 89.34 | 90.72 | 91.40 | 91.36 | 91.62 | 91.59 | 90.87 | 91.51 | 91.62 | 91.27 |
| 1 month, 2-8°C | 98.95 | 98.95 | 98.92 | 98.97 | 98.96 | 98.94 | 98.97 | 98.95 | 98.97 | 98.97 | 98.94 | 98.91 |
| 1 month, 25°C | 98.52 | 98.01 | 97.69 | 98.08 | 98.17 | 98.17 | 98.25 | 98.24 | 98.11 | 98.23 | 97.26 | 97.24 |

The results in Table 2-37 to Table 2-39 showed:
Solution clarity: Compositions I3 to I12 contained phenol or m-cresol as preservative. Although they contained propylene glycol, glycerin, sodium chloride or their combination as osmotic regulator and stored at 25°C and 40°C for 1 month, and at 60°C for 10 days, no increase in turbidity occurred. This was different from the preservative-free formulations. When the preservative-free compositions used propylene glycol or sodium chloride as osmotic pressure regulators, they were prone to increased turbidity. Unexpectedly, it was discovered that the use of phenol or m-cresol as preservative allowed the composition to use more types of osmotic pressure regulators (such as mannitol, propylene glycol, glycerin, sodium chloride and their combinations) without destroying their physical stability of solution property/clarity.

Polymer (%): Compared with compositions I1 and I2 without preservatives, compositions I3 to I12 containing preservatives showed considerable stability trends at 40°C or 25°C or 2-8°C. The introduction of phenol or m-cresol preservatives, as well as individual or combined osmotic pressure regulators, did not lead to a deterioration of the polymer index in compositions I3 to I12. Purity (%): Compositions I1 to I12 all exhibited temperature-dependent stability. Compositions I3 to I12 used different combinations of preservatives (m-cresol or phenol) and osmotic pressure regulators (mannitol, propylene glycol, glycerol, sodium chloride), and all showed similar temperature-dependent purity decreases.

### Amino Acid Sequence:

| Numbering in sequence listing | Name | Sequence |
|---|---|---|
| (SEQ ID NO:1) | P001 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; and the C-terminal amino acid is amidated as a C-terminal primary amide (SEQ ID NO:1) |
| (SEQ ID NO:2) | P007 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:2). |
| (SEQ ID NO:3) | P008 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:3). |
| (SEQ ID NO:4) | P013 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:4). |
| (SEQ ID NO:5) | P014 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:5). |
| (SEQ ID NO:6) | P015 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:6). |
| (SEQ ID NO:7) | P016 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:7). |
| (SEQ ID NO:8) | P017 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:8). |
| (SEQ ID NO:9) | P018 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:9). |
| (SEQ ID NO:10) | P019 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (SEQ ID NO:10). |
| (SEQ ID NO:11) | P020 | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:11). |
| (SEQ ID NO:12) | Formula (I) | Y-X1-E-G-T-X2-T-S-D-Y-X3-I-X4-L-D-K-Q-A-Q-A-E-F-V-K-W-L-L-K-G-G-P- S-S-G-A-P-P-P-S-K; wherein X₁ is Aib; X₂ is αMePhe; X₃ is Aib; X₄ is Aib; |
| | | 1 K, which is selected from the group consisting of K at position 16, K at position 24, K at position 28 and K at position 40 of Formula I, is chemically modified through conjugation to the ε-amino of the K side-chain with ([2-(2-amino-ethoxy)-ethoxy]-acetyl)ₐ-(γ-Glu)_{b}-CO-(CH₂)_{c}-Z, wherein each a is independently selected from an integer of 1, 2 and 3, each b is independently selected from an integer of 1, 2 and 3, and each c is independently selected from an integer of 16, 18 and 20, wherein Z is independently selected from the group consisting of -CH₃, carboxylic acids or carboxylic acid bioisosteres, phosphonates or sulfonates, preferably - COOH; and the C-terminal amino acid is amidated as a C-terminal primary amide (SEQ ID NO:12). |

## Claims

1. A pharmaceutical composition, wherein the pharmaceutical composition has a pH ranging from about 5.0 to about 9.0, and the pharmaceutical composition comprises from about 0.1 to about 50 mg/mL of a polypeptide compound of the following Formula I: 
Y-Aib-E-G-T-αMePhe-T-S-D-Y-Aib-I-Aib-L-D-K-Q-A-Q-A-E-F-V-K²⁴-W-L-L-K²⁸-G-G-P-S-S-
G-A-P-P-P-S-K      Formula I (SEQ ID NO:12);
 wherein
1 K, which is selected from the group consisting of K at position 16, K at position 24, K at position 28 and K at position 40 of Formula I, is chemically modified through conjugation to the ε-amino of the K side-chain with ([2-(2-amino-ethoxy)-ethoxy]-acetyl)ₐ-(γ-Glu)_{b}-CO-(CH₂)_{c}-Z, wherein each a is independently selected from an integer of 1, 2 and 3, each b is independently selected from an integer of 1, 2 and 3, and each c is independently selected from an integer of 16, 18 and 20, wherein Z is independently selected from the group consisting of -CH₃, carboxylic acids or carboxylic acid bioisosteres, phosphonates or sulfonates, preferably -COOH; and the C-terminal amino acid is amidated as a C-terminal primary amide.

2. The pharmaceutical composition according to claim 1, wherein the polypeptide compound of Formula I is
Y-Aib-E-G-T-αMePhe-T-S-D-Y-Aib-I-Aib-L-D-K-Q-A-Q-A-E-F-V-K²⁴-W-L-L-K²⁸-G-G-P-S-S-G-A-P-P-P-S-K Formula I, wherein
K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (P007, SEQ ID NO:2); or
K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (P013, SEQ ID NO:4); or
K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (P014, SEQ ID NO:5); or
K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (P015, SEQ ID NO:6); or
K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (P016, SEQ ID NO:7); or
K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (P008, SEQ ID NO:3); or
K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (P017, SEQ ID NO:8); or
K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (P018, SEQ ID NO:9); or
K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (P019, SEQ ID NO:10); or
K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (P020, SEQ ID NO:11).

3. The pharmaceutical composition according to claim 1 or 2, wherein the pharmaceutical composition further comprises an osmotic pressure regulator, a buffer and water, and optionally additional excipient or carrier, wherein the additional excipient or carrier includes one or more of a pH adjusting agent, a preservative, an antioxidant and a stabilizer.

4. The pharmaceutical composition according to claim 3, wherein the buffer is selected from the group consisting of acetic acid, succinic acid, citric acid, phosphoric acid, gluconic acid, oxalic acid, lactic acid, tartaric acid, fumaric acid, 4-hydroxyethylpiperazineethanesulfonic acid (HEPES), tromethamine (Tris), disodium hydrogen phosphate, trisodium citrate, disodium citrate, trisodium citrate dihydrate, citric acid-trisodium citrate, citric acid monohydrate-trisodium citrate dihydrate, disodium hydrogen phosphate-citric acid, sodium phosphate, dipotassium hydrogen phosphate, potassium dihydrogen phosphate, potassium phosphate, diammonium hydrogen phosphate, ammonium dihydrogen phosphate, ammonium phosphate, disodium hydrogen phosphate-potassium dihydrogen phosphate, and a combination thereof.

5. The pharmaceutical composition according to claim 3 or 4, wherein the molar concentration of the buffer is from about 0.5 to about 50 mM, based on the total volume of the aqueous solution.

6. The pharmaceutical composition according to any one of claims 3 to 5, wherein the osmotic pressure regulator is selected from the group consisting of sodium chloride, phosphates, citrates, borates, tartrates, lactose, trehalose, sucrose, glucose, mannitol, sorbitol, xylitol, L-glycine, L-histidine, arginine, lysine, isoleucine, aspartic acid, tryptophan, threonine, glycerol, 1,2-propylene glycol, 1,3-propylene glycol, 1,3-butylene glycol, polyethylene glycol, or any combination thereof.

7. The pharmaceutical composition according to any one of claims 3 to 6, wherein the content of the osmotic pressure regulator is from about 1 to about 100 mg/mL, based on the total volume of the aqueous solution.

8. The pharmaceutical composition according to any one of claims 3 to 7, wherein the pH adjusting agent is an aqueous sodium hydroxide solution or hydrochloric acid.

9. The pharmaceutical composition according to any one of claims 3 to 8, wherein the preservative is selected from the group consisting of phenol, m-cresol, cresol, phenoxyethanol, chlorobutanol, benzyl alcohol, benzoate derivatives, and any combination thereof.

10. The pharmaceutical composition according to any one of claims 1 to 9, wherein the pharmaceutical composition is an aqueous solution, and based on the total volume of the aqueous solution, the pharmaceutical composition comprises:
about 0.1 to about 50 mg/mL of the compound of Formula I of claim 1, for example, about 1 to about 40 mg/mL of Compound P016, P014 or P020,
citric acid and trisodium citrate at a total molar concentration of from 0.5 to about 50 mM, preferably about 1.0 to 10 mM of citric acid-trisodium citrate, wherein the molar ratio of citric acid to trisodium citrate is about 1:4-50, preferably about 1:4-20,
0 to about 50 mg/mL, preferably about 0.1 to about 50 mg/mL, more preferably about 1 to about 10 mg/mL, further more preferably about 2 to about 6 mg/mL of the preservative,
about 1 to about 100 mg/mL of the osmotic pressure regulator, preferably about 1 to about 100 mg/mL of mannitol, 1,2-propylene glycol, 1,3-propylene glycol, glycerol, sodium chloride, or any combination thereof,
optionally an aqueous sodium hydroxide solution or hydrochloric acid, and
water,
wherein the aqueous solution has a pH ranging from about 5.0 to about 9.0, for example, from about 5.8 to about 9.0, preferably from about 6.0 to about 8.8, and more preferably from about 6.5 to about 7.4.

11. The pharmaceutical composition according to any one of claims 1 to 9, wherein the pharmaceutical composition is an aqueous solution, and based on the total volume of the aqueous solution, the pharmaceutical composition comprises:
about 0.1 to about 50 mg/mL of the compound of Formula I of claim 1, for example, about 1 to about 40 mg/mL of Compound P016, P014 or P020,
citric acid and trisodium citrate at a total molar concentration of from 0.5 to about 50 mM, preferably about 1.0 to about 10 mM of citric acid-trisodium citrate, wherein the molar ratio of citric acid to trisodium citrate is about 1:4-50, preferably about 1:4-20,
about 0.1 to about 100 mg/mL of mannitol,
optionally a preservative, and
water,
wherein the aqueous solution has a pH ranging from about 5.0 to about 9.0, for example, from about 5.8 to about 9.0, preferably from about 6.0 to about 8.8, and more preferably from about 6.5 to about 7.4.

12. The pharmaceutical composition according to any one of claims 1 to 11, wherein the osmotic pressure of the pharmaceutical composition is about 200 to about 500 mOsm/kg, preferably about 250 to about 350 mOsm/kg, and more preferably about 260 to about 320 mOsm/kg, wherein the osmotic pressure is measured by using an osmotic pressure molar concentration meter.

13. A kit comprising a container and the pharmaceutical composition according to any one of claims 1 to 12 contained in the container.

14. Use of the pharmaceutical composition according to any one of claims 1 to 12 for the manufacture of a medicament for treating a disease, such as diabetes mellitus, obesity, a disease associated with diabetes mellitus, and/or a disease associated with obesity.

15. The use according to claim 14, wherein the unit dose of the pharmaceutical composition is about 0.1 mg to about 0.5 mg or about 0.5 mg to about 50 mg.
